# EUROPEAN PATENT SPECIFICATION

(11) **EP 2 734 548 B1**
(45) Date of publication and mention of the grant of the patent: **17.08.2016**
(21) Application number: 12738101.0
(22) Date of filing: 19.07.2012
(51) Int. Cl.: C07K 16/24

(54) **TNF-ALPHA-ANTIGEN-BINDING PROTEINS AGAINST WITH INCREASED FCRN BINDING FOR USE IN THERAPY**
TNF-ALPHA-ANTIGENBINDENDE PROTEINE MIT ERHÖHTER FCRN-BINDUNG ZUR VERWENDUNG IN DER THERAPIE
PROTÉINES DE LIAISON À UN ANTIGÈNE TNF-ALPHA AYANT UNE LIAISON ACCRUE À FCRN DESTINÉE À UNE UTILISATION THÉRAPEUTIQUE

(30) Priority: 19.07.2011 GB 201112429
(43) Date of publication of application: 28.05.2014
(60) Divisional application: 15197750.1
(73) Proprietor: Glaxo Group Limited, Brentford, Middlesex TW8 9GS (GB)
(72) Inventor: ELLIS, Jonathan, Henry, Stevenage Hertfordshire SG1 2NY (GB); MOLLOY, Michael, J, Stevenage Hertfordshire SG1 2NY (GB); SHAH, Tejash, Stevenage Hertfordshire SG1 2NY (GB); TOMLINSON, Ian, M, Stevenage Hertfordshire SG1 2NY (GB); YASIN, Ahmed, Stevenage Hertfordshire SG1 2NY (GB)
(74) Representative: Lee, Alison Lesley
(86) International application number: PCT/EP2012/064129
(87) International publication number: WO 2013/011076

(56) References cited:
- WO-A1-2009/083246
- WO-A1-2010/097385
- WO-A2-2008/057240
- WO-A2-2009/068649
- WO-A2-2009/073805
- WO-A2-2010/102251
- WO-A2-2010/136492
- US-A1- 2009 163 699
- PAUL R HINTON ET AL: "An engineered human IgG1 antibody with longer serum half-life", THE JOURNAL OF IMMUNOLOGY, THE AMERICAN ASSOCIATION OF IMMUNOLOGISTS, US, vol. 176, no. 1, 1 January 2006 (2006-01-01), pages 346-356, XP002484005, ISSN: 0022-1767
- HINTON PAUL R ET AL: "Engineered human IgG antibodies with longer serum half-lives in primates", JOURNAL OF BIOLOGICAL CHEMISTRY, AMERICAN SOCIETY FOR BIOCHEMISTRY AND MOLECULAR BIOLOGY, US, vol. 279, no. 8, 20 February 2004 (2004-02-20), pages 6213-6216, XP002482523, ISSN: 0021-9258, DOI: 10.1074/JBC.C300470200
- A. DATTA-MANNAN ET AL: "Monoclonal Antibody Clearance: IMPACT OF MODULATING THE INTERACTION OF IgG WITH THE NEONATAL Fc RECEPTOR", JOURNAL OF BIOLOGICAL CHEMISTRY, vol. 282, no. 3, 1 January 2007 (2007-01-01), pages 1709-1717, XP55043665, ISSN: 0021-9258, DOI: 10.1074/jbc.M607161200
- DATTA-MANNAN AMITA ET AL: "HUMANIZED IGG1 VARIANTS WITH DIFFERENTIAL BINDING PROPERTIES TO THE NEONATAL FC RECEPTOR: RELATIONSHIP TO PHARMACOKINETICS IN MICE AND PRIMATES", DRUG METABOLISM AND DISPOSITION, WILLIAMS AND WILKINS, BALTIMORE, MD, US, vol. 35, no. 1, 1 January 2007 (2007-01-01), pages 86-94, XP009077715, ISSN: 0090-9556, DOI: 10.1124/DMD.106.011734
- DALL'ACQUA W F ET AL: "Properties of human IgG1s engineered for enhanced binding to the neonatal Fc receptor (FcRn)", JOURNAL OF BIOLOGICAL CHEMISTRY, AMERICAN SOCIETY FOR BIOCHEMISTRY AND MOLECULAR BIOLOGY, US, vol. 281, no. 33, 21 June 2006 (2006-06-21), pages 23514-23524, XP002404904, ISSN: 0021-9258, DOI: 10.1074/JBC.M604292200
- ROBERT L SHIELDS ET AL: "High resolution mapping of the binding site on human IgG1 for Fc gamma RI, Fc gamma RII, Fc gamma RIII and FcRn and design of IgG1 variants with improved binding to the Fc gamma R", JOURNAL OF BIOLOGICAL CHEMISTRY, AMERICAN SOCIETY FOR BIOCHEMISTRY AND MOLECULAR BIOLOGY, US, vol. 276, no. 9, 2 March 2001 (2001-03-02) , pages 6591-6604, XP002638208, ISSN: 0021-9258, DOI: 10.1074/JBC.M009483200 [retrieved on 2000-11-28] cited in the application

## Description

### Field

The invention relates to novel variants of anti-TNF antibodies with increased half-life for use in a method of treating a disease.

### Background

Antibodies are heteromultimeric glycoproteins comprising at least two heavy and two light chains. Aside from IgM, intact antibodies are usually heterotetrameric glycoproteins of approximately 150Kda, composed of two identical light (L) chains and two identical heavy (H) chains. Each heavy chain has at one end a variable domain (VH) followed by a number of constant regions. Each light chain has a variable domain (VL) and a constant region at its other end; the constant region of the light chain is aligned with the first constant region of the heavy chain and the light chain variable domain is aligned with the variable domain of the heavy chain. Depending on the amino acid sequence of the constant region of their heavy chains, human antibodies can be assigned to five different classes, IgA, IgD, IgE, IgG and IgM. IgG and IgA can be further subdivided into subclasses, IgG1, IgG2, IgG3 and IgG4; and IgA1 and IgA2. The variable domain of the antibody confers binding specificity upon the antibody with certain regions displaying particular variability called complementarity determining regions (CDRs). The more conserved portions of the variable region are called Framework regions (FR). The variable domains of intact heavy and light chains each comprise four FR connected by three CDRs. The constant regions are not directly involved in the binding of the antibody to the antigen but exhibit various effector functions such as participation in antibody dependent cell-mediated cytotoxicity (ADCC), phagocytosis via binding to Fcγ receptor, half-life/clearance rate via neonatal Fc receptor (FcRn) and complement dependent cytotoxicity via the C1q component of the complement cascade. The nature of the structure of an IgG antibody is such that there are two antigen-binding sites, both of which are specific for the same epitope. They are therefore, monospecific.

In adult mammals, FcRn, also known as the neonatal Fc receptor, plays a key role in maintaining serum antibody levels by acting as a protective receptor that binds and salvages antibodies of the IgG isotype from degradation. IgG molecules are endocytosed by endothelial cells, and if they bind to FcRn, are recycled out into circulation. In contrast, IgG molecules that do not bind to FcRn enter the cells and are targeted to the lysosomal pathway where they are degraded.

The neonatal FcRn receptor is believed to be involved in both antibody clearance and the transcytosis across tissues (see Junghans R.P (1997) Immunol.Res 16. 29-57 and Ghetie et al (2000) Annu.Rev.lmmunol. 18, 739-766).

WO 9734631 discloses a composition comprising a mutant IgG molecule having increased serum half-life and at least one amino acid substitution in the Fc-hinge region. Amino acid substitution at one or more of the amino acids selected from number 252, 254, 256, 309, 311 or 315 in the CH2 domain or 433 or 434 in the CH3 domain is disclosed.

WO 00/42072 discloses a polypeptide comprising a variant Fc region with altered FcRn binding affinity, which polypeptide comprises an amino acid modification at any one or more of amino acid positions 238, 252, 253, 254, 255, 256, 265, 272, 286, 288, 303, 305, 307, 309, 311, 312, 317, 340, 356, 360, 362, 376, 378, 380, 386,388, 400, 413, 415, 424,433, 434,435, 436, 439, and 447 of the Fc region.

WO 02/060919 discloses a modified IgG comprising an IgG constant domain comprising amino acid modifications at one or more of positions 251, 253, 255, 285-290, 308-314, 385-389, and 428-435. WO 2004035752 discloses a modified antibody of class IgG wherein at least one amino acid residue from the heavy chain constant region selected from the group consisting of amino acid residues 250, 314, and 428 is different from that present in an unmodified class IgG antibody.

Shields et al. (2001, J Biol Chem ; 276:6591-604) used alanine scanning mutagenesis to alter residues in the Fc region of a human IgG1 antibody and then assessed the binding to human FcRn. Positions that effectively abrogated binding to FcRn when changed to alanine include I253, S254, H435, and Y436. Other positions showed a less pronounced reduction in binding as follows: E233-G236, R255, K288, L309, S415, and H433. Several amino acid positions exhibited an improvement in FcRn binding when changed to alanine.

Dall'Acqua et al. (2002, J Immunol.;169:5171-80) described random mutagenesis and screening of human IgG1 hinge-Fc fragment phage display libraries against mouse FcRn. They disclosed random mutagenesis of positions 251, 252, 254-256, 308, 309, 311, 312, 314, 385-387, 389, 428, 433, 434, and 436.

WO2006130834 discloses modified IgG comprising an IgG comprising an IgG constant domain comprising amino acid modifications at one or more positions of 252, 254, 256, 433, 434 and 436. Therefore, modification of Fc domains of IgG antibodies has been discussed as a means of increasing the serum half- life of therapeutic antibodies. However, numerous such modifications have been suggested with varying and sometimes contradictory results in different antibodies. For example, US 2009/0163699 relates to Fc variants that have altered binding to FcRn and/or improved half life. One such modification disclosed relates to the mutations herein referred to as the 'YTE' mutations. In addition an RSV antibody was modified using mutations to enhance half life ( Dall'Acqua et al Protein Structure and Folding: Properties of Human IgG1s Engineered for Enhanced Binding to the Neonatal Fc Receptor (FcRn) June 21, 2006 J. Biol. Chem. 2006, 281:23514-23524.

The administration of antigen binding proteins as therapeutics requires injections with a prescribed frequency relating to the clearance and half-life characteristics of the protein.

Adalimumab is a monoclonal antibody against TNF-alpha which is used for treatment of rheumatoid arthritis, psoriatic arthritis, ankylosing spondylitis, and Crohn's disease. It is produced by recombinant DNA technology using a mammalian cell expression system. It consists of 330 amino acids and has a molecular weight of approximately 148 kilodaltons. See United States Patent 6090382. At doses of 0.5 mg/kg (-40 mg), clearance for adalimumab is said to range from 11 to 15 ml/hour, the distribution volume (Vₛₛ) ranges from 5 to 6 litres and the mean terminal phase half-life was approximately two weeks (Summary of Product Characteristics available from www.medicines.org.uk). These half life and clearance properties mean that currently adalimumab needs to be administered once every two weeks. In some patients depending on disease it may be necessary to administer a loading dose such as for example in psoriasis patients. This dosage may differ from the maintenance dose.

### Summary of invention

In one aspect, the invention relates to an antigen binding protein which specifically binds to TNF-alpha comprising:
(i) CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); and
(ii) a neonatal Fc receptor (FcRn) binding portion of a human IgG1 constant domain comprising amino acid substitutions relative to the human IgG1 constant domain at amino acid residues 252, 254 and 256 numbered according to EU index of Kabat and wherein the substitution at residue 252 is a substitution of met with tyr; the substitution at residue 254 is a substitution of ser with thr and the substitution at residue 256 is a substitution of thr with glu;
wherein the antigen binding protein has an increased FcRn binding affinity at pH 6 and increased half-life as compared to an IgG comprising the light chain sequence of SEQ ID No. 2 and the heavy chain sequence of SEQ ID No.12. for use in a method of treating a disease wherein the antigen binding protein is administered to patients at a single dose between 35 to 45 mg at a four to eight weekly interval.

In a further aspect the antigen binding protein has a human IgG1 constant domain of SEQ ID No. 13 before amino acid substitutions are introduced.

In a further aspect the antigen binding protein is administered to patients subcutaneously as a single 40 mg dose no more than once every four weeks or no more than once every eight weeks.

In a further aspect the half-life of the antigen binding protein is increased 2 fold, 3 fold, 4 fold or 5 fold as compared to the native IgG.

In a further aspect the administration of the antigen binding protein no more than once every four weeks in patients achieves the mean steady-state trough concentration in the patient population of between 4 µg/ml to 7 µg/ml or for example between 5 µg/ml to 6 µg/ml.

In a further aspect the clearance of the antigen binding protein is 2ml/ hr to 4ml/hr.

In a further aspect the antigen binding protein comprises a constant domain as shown in SEQ ID No: 7

In a further aspect the antigen binding protein is an antibody.

In yet a further aspect the antigen binding protein is to be administered with methotrexate, preferably wherein the antigen binding protein is administered for the treatment of rheumatoid arthritis.

In a further aspect the antigen binding protein comprises the heavy chain sequence as shown in SEQ ID No 5 optionally with a light chain sequence as shown in SEQ ID No: 2.

In a further aspect the antigen binding protein is for use in the treatment of rheumatoid arthritis, polyarticular juvenile idiopathic arthritis, psoriatic arthritis, ankylosing spondylitis, Crohn's disease or Psoriasis.

Throughout the specification the term "human IgG1 constant domain" encompasses all allotypes and variants thereof known to a person skilled in the art.

### Brief Description of Figures

**Figure 1** **-** Binding of anti-TNFα antibodies to human TNFα
**Figure 2** **-** Analysis of binding activity of anti-TNFα antibodies to human TNFα following an accelerated stressor study
**Figure 3** **-** Binding of anti-TNFα antibodies to human TNFα following incubation in 25% human serum for 2 weeks
**Figure 4** **-** Binding of anti-TNFα antibodies to human TNFα following freeze-thaw
**Figure 5** **-** Analysis of anti-TNFα antibodies to FcγRIIIa receptors (a) Binding to human FcγRIIIa (valine 158 variant) **(b)** Binding to human FcγRIIIA (phenylalanine 158 variant)
**Figure 6** **-** Average dose normalised plasma concentrations of BPC2604 in female cynomolgus monkeys and pascolizumab in male cynomolgus monkeys following a single intravenous (1 hr infusion)

### Detailed Description of Invention

The invention relates to novel antigen binding proteins binding specifically to TNF-alpha comprising:
(i) CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); and
(ii) a neonatal Fc receptor (FcRn) binding portion of a human IgG1 constant domain comprising amino acid substitutions relative to the human IgG1 constant domain at amino acid residues 252, 254 and 256 numbered according to EU index of Kabat and wherein the substitution at residue 252 is a substitution of met with tyr; the substitution at residue 254 is a substitution of ser with thr and the substitution at residue 256 is a substitution of thr with glu;
wherein the antigen binding protein has an increased FcRn binding affinity at pH 6 and increased half-life as compared to an IgG comprising the light chain sequence of SEQ ID No. 2 and the heavy chain sequence of SEQ ID No.12. for use in a method of treating a disease wherein the antigen binding protein is administered to patients at a single dose between 35 to 45 mg at a four to eight weekly interval.

In particular, novel variants of anti-TNF antibodies such as adalimumab which show increased binding to the FcRn receptor and increased half life as compared to adalimumab. Adalimumab is an IgG monoclonal antibody comprising the light chain sequence of SEQ ID No. 2 and heavy chain sequence of SEQ ID No.12.

Specific modifications to adalimumab as described herein show particular improvements in FcRn binding as shown in the examples below. Affinity matured variants of adalimumab also show improvement in anti-TNF-alpha binding and/or neutralisation activity.

The novel antigen binding proteins have an increased binding to the FcRn receptor and increased half life and/ or increased Mean Residence Time and/ or decreased Clearance. It is considered that binding to FcRn results in longer serum retention *in vivo.* In order to increase the retention of the Fc proteins *in vivo,* the increase in binding affinity is observed around pH 6. In one aspect, there is described an antigen binding protein with optimised binding to FcRn.

The half-life of the antigen binding protein is increased 2 to 6 fold, such as 2 fold, 3 fold, 4 fold, 5 fold or 6 fold as compared to an IgG comprising the light chain sequence of SEQ ID No. 2 and heavy chain sequence of SEQ ID No.12. Preferably, the half-life of the antigen binding protein is increased 3 fold, 4 fold, or more compared to an IgG comprising the light chain sequence of SEQ ID No. 2 and heavy chain sequence of SEQ ID No.12. For example, if the IgG is adalimumab having a half life of 10 days or in the range of 10 to 20 days then in one aspect an antigen binding protein shows a half life of about 40 to 80 days. For example an antigen binding protein comprising a heavy chain sequence of SEQ ID NO:5..

The antigen binding protein of the invention administered no more than once every four weeks in patients, achieves mean steady-state trough concentrations between 2 µg/ml to 7 µg/ml. Preferably, the mean steady-state trough concentrations are between 4 µg/ml to 7 µg/ml and more preferably between 5 µg/ml to 6 µg/ml.

The antigen binding protein administered no more than once every 28 days in patients, achieves mean steady-state trough concentrations between 2 µg/ml to 7 µg/ml. Preferably, the mean steady-state trough concentrations are between 4 µg/ml to 7 µg/ml and more preferably between 5 µg/ml to 6 µg/ml.

The antigen binding protein can be administered once every 4, 5, 6, 7 or 8 weeks to achieve comparable mean steady-state trough concentrations as those achieved by adalimumab, when administered once every two weeks at the same dose, for example the antigen binding protein can be administered once every 7 or 8 weeks.

The antigen binding protein can be administered once every 25-80 days for example once every 40-60 days, or for example once every 28, 35, 42, 49 or 56 days to achieve comparable mean steady-state trough concentrations as those achieved by adalimumab, when administered once every 14 days at the same dose for example every 49 to 60 days, for example every 56 days.

The antigen binding protein has a 2 fold, or 4 fold, or 6 fold, or 8 fold or greater affinity for human FcRn at pH 6 as assessed by ProteOn XPR36 protein interaction array system at 25°C wherein the antibodies are immobilised on the chip. Preferably, the antigen binding protein has an affinity for human FcRn between about 100 to about 500 KD(nM), such as between about 130 to about 360 KD(nM) or between about 140 to about 250KD(nM) or between about 140 to about 210KD(nM).

The clearance of the antigen binding protein is 2 to 10 ml/hr, preferably 2 to 5ml/hr or 2 to 4ml/hr or 2 to 3ml/hr, such as 2, 2.5, 3, 4 or 5 ml/hr. The antigen binding protein shows a clearance rate which is 2 fold, 3 fold, 4 fold or 5 fold lower than adalimumab. For example, clearance for an antigen binding protein is in the ranges specified above or 2 fold, 3 fold, 4 fold or 5 fold lower than adalimumab at a human dose of about 40 mg.

As described herein,
antigen binding protein is a variant of adalimumab (IgG comprising the light chain sequence of SEQ ID No. 2 and the heavy chain sequence of SEQ ID No.12), the variant comprising one or more substitutions in the FcRn binding portion of the IgG constant domain to increase the half-life of the variant compared with adalimumab, wherein when the variant is administered to patients at a single dose of 40 mg at a four to eight weekly interval, preferably eight weekly interval, the mean steady-state trough antibody concentration in the patient population does not fall below 5 µg/ml for example, the mean steady-state trough antibody concentration in the patient population does not fall below 6 µg /ml, between dosing intervals.

Described herein the antigen binding protein comprises amino acid modifications in the Fc region of said antigen binding protein, wherein said modification is at positions 252, 254 and 256 of the Fc region as compared to same position in the adalimumab sequence, wherein the numbering of the amino acids in the Fc region is that of the EU index in Kabat.

The wild type human IgG1 has amino acid residues Val-Leu-His-Gln-Asp-Trp-Leu at positions 308-314, amino acid residues Leu-Met- Ile-Ser-Arg-Thr at positions 251-256, amino acid residues Met-His-Glu-Ala-Leu-His-Asn-HisTyr at positions 428-436, and amino acid residues Gly-Gln-Pro-Glu-Asn at positions 385-389. Residue numbering may differ for IgG2-4.

The antigen binding protein comprises one or more amino acid substitution relative to the human IgG1 constant domain comprising the sequence of SEQ ID No. 13.

In one aspect the one or more amino acid substitution in the FcRn binding portion of the human IgG1 heavy chain constant domain is at amino acid residues 252, 254 and 256 numbered according to EU index of Kabat and the aa substitution at residue 252 is a substitution of met with tyr, the aa substitution at residue 254 is a substitution of ser with thr; and the aa substitution at residue 256 is a substitution of glu. Preferably, the IgG1 constant domain is as shown in SEQ ID No: 7.

In one aspect the antigen binding protein is an antibody.

For example, the antigen binding protein comprises the heavy chain sequence as shown in SEQ ID No 5 optionally with a light chain sequence as shown in SEQ ID No: 2.

Also described is an antigen binding protein as disclosed herein for use in the treatment of disease in a human.

As described herein the disease to be treated by the antigen binding protein is rheumatoid arthritis, polyarticular juvenile idiopathic arthritis, psoriatic arthritis, ankylosing spondylitis, Ulcerative colitis, spondyloarthropathy, Crohn's disease or Psoriasis.

In one aspect, the antigen binding protein is to be administered with methotrexate. The methotrexate can be delivered before, after or at the same time, or substantially the same time, as the antigen binding protein. In a preferred aspect the antigen binding protein is to be administered with methotrexate to a patient suffering from rheumatoid arthritis. In one aspect, methotrexate is administered to patients receiving an antigen binding protein as described herein to reduce the immunogenic effect of the antigen binding protein. In one aspect, the antigen binding protein is administered to patients already receiving methotrexate. Methotrexate may be substituted by another acceptable compound which reduced the immune response to the antigen binding protein, for example corticosteroids.

In one aspect, the antigen binding protein is administered to the patient as a single 20, 30, 35, 40, 45, 50, 55, 60, 65, 70, 75 or 80 mg dose no more than once every four weeks, preferably once every 5, 6, 7, or 8 weeks and most preferably once every 8 weeks. Preferably, the dose is 40 to 80 mg, for example 40mg.

Also described is an antigen binding protein with enhanced FcRn binding and having one or more additional substitutions, deletions or insertions that modulate another property of the effector function. Once expressed by the desired method, the antigen binding protein is then examined for in vitro activity by use of an appropriate assay. Presently conventional ELISA and Biacore assay formats are employed to assess qualitative and quantitative binding of the antigen binding construct to its target. Additionally, other in vitro assays may also be used to verify neutralizing efficacy prior to subsequent human clinical studies performed to evaluate the persistence of the antigen binding protein in the body despite the usual clearance mechanisms.

The dose and duration of treatment relates to the relative duration of the molecules of the present invention in the human circulation, and can be adjusted by one of skill in the art depending upon the condition being treated and the general health of the patient based on the information provided herein. It is envisaged that repeated dosing (e.g. once every 4 weeks, 5 weeks, 6 weeks, 7 weeks or 8 weeks) over an extended time period (e.g. four to six months) maybe required to achieve maximal therapeutic efficacy.

The mode of administration of the therapeutic agent as herein described may be any suitable route which delivers the agent to the host. The antigen binding proteins are particularly useful for parenteral administration, i.e., subcutaneously (s.c.), intrathecally, intraperitoneally, intramuscularly (i.m.), intravenously (i.v.), or intranasally. In one aspect the antigen binding proteins are administered via a subcutaneous auto injector pen or a subcutaneous pre-filled syringe.

Antigen binding proteins as herein described may be prepared as pharmaceutical compositions containing an effective amount of the antigen binding protein as an active ingredient in a pharmaceutically acceptable carrier. In the prophylactic agent, an aqueous suspension or solution containing the antigen binding construct, preferably buffered at physiological pH, in a form ready for injection is preferred. The compositions for parenteral administration will commonly comprise a solution of the antigen binding construct or a cocktail thereof dissolved in a pharmaceutically acceptable carrier, preferably an aqueous carrier. A variety of aqueous carriers may be employed, e.g., 0.9% saline, 0.3% glycine, and the like. These solutions may be made sterile and generally free of particulate matter. These solutions may be sterilized by conventional, well known sterilization techniques (e.g., filtration). The compositions may contain pharmaceutically acceptable auxiliary substances as required to approximate physiological conditions such as pH adjusting and buffering agents, etc. The concentration of the antigen binding protein in such pharmaceutical formulation can vary widely, i.e., from less than about 0.5%, usually at or at least about 1% to as much as 15 or 20% by weight and will be selected primarily based on fluid volumes, viscosities, etc., according to the particular mode of administration selected.

The TNF-alpha antigen binding proteins described herein may be formulated in the concentration range of 20 to 300 mg/mL. For example, the antigen binding protein is present in a concentration of 20-200 mg/mL or 50-100 mg/mL; or about 40 mg/mL or about 45 mg/mL or about 50 mg/mL or about 55 mg/mL or about 60 mg/mL or about 70 mg/mL or about 80 mg/mL or about 90 mg/mL, or about 100mg/mL. In one aspect, the TNF-alpha antigen binding protein is at a concentration of about 50 mg/mL.

In one aspect, the antigen binding protein is provided or administered at a dose of about 40 mg. Preferably the antigen binding protein is suitable for subcutaneous delivery and is delivered subcutaneously. Other dosing or administration routes may also be used, as disclosed herein.

In one aspect the antigen binding proteins shows increased Mean Residence Time as compared to an IgG comprising the light chain sequence of SEQ ID No. 2 and heavy chain sequence of SEQ ID No.12.

The binding ability of modified IgGs and molecules comprising an IgG constant domain or FcRn binding portion thereof can be characterized by various *in vitro* assays. PCT publication WO 97/34631 by Ward discloses various methods in detail. For example, in order to compare the ability of the modified IgG or fragments thereof to bind to FcRn with that of the wild type IgG, the modified IgG or fragments thereof and the wild type IgG can be radio-labeled and reacted with FcRn-expressing cells *in vitro.* The radioactivity of the cell-bound fractions can be then counted and compared. The cells expressing FcRn to be used for this assay are may be endothelial cell lines including mouse pulmonary capillary endothelial cells (B10, D2.PCE) derived from lungs of B10.DBA/2 mice and SV40 transformed endothelial cells (SVEC) (Kim et al., J Immunol., 40: 457-465,1994) derived from C3H/HeJ mice. However, other types of cells which express sufficient number of FcRn, including mammalian cells which express recombinant FcRn of a species of choice, can be also used. Alternatively, after counting the radioactivity of the bound fraction of modified IgG or that of unmodified IgG, the bound molecules can be then extracted with the detergent, and the percent release per unit number of cells can be calculated and compared.

Affinity of antigen binding proteins for FcRn can be measured by surface plasmon resonance (SPR) measurement using, for example, a BIAcore 2000 (BIAcore Inc.) as described previously (Popov et al., Mol. Immunol., 33: 493-502,1996; Karlsson et al., J Immunol. Methods, 145: 229-240,1991). In this method, FcRn molecules are coupled to a BIAcore sensor chip (e. g., CM5 chip by Pharmacia) and the binding of modified IgG to the immobilized FcRn is measured at a certain flow rate to obtain sensorgrams using BIA evaluation 2.1 software, based on which on-and off-rates of the modified IgG, constant domains, or fragments thereof, to FcRn can be calculated. Relative affinities of antigen binding proteins and unmodified IgG for FcRn can be also measured by a simple competition binding assay. Furthermore, affinities of modified IgGs or fragments thereof, and the wild type IgG for FcRn can be also measured by a saturation study and the Scatchard analysis.

Transfer of modified IgG or fragments thereof across the cell by FcRn can be measured by in vitro transfer assay using radiolabeled IgG or fragments thereof and FcRn- expressing cells and comparing the radioactivity of the one side of the cell monolayer with that of the other side. Alternatively, such transfer can be measured *in vivo* by feeding 10-to 14-day old suckling mice with radiolabeled, modified IgG and periodically counting the radioactivity in blood samples which indicates the transfer of the IgG through the intestine to the circulation (or any other target tissue, e. g., the lungs). To test the dose-dependent inhibition of the IgG transfer through the gut, a mixture of radiolabeled and unlabeled IgG at certain ratio is given to the mice and the radioactivity of the plasma can be periodically measured (Kim et al., Eur. R Immunol., 24: 2429-2434,1994).

The half-life of antigen binding proteins can be measured by pharmacokinetic studies according to the method described by Kim et al. (Eur. J. of Immuno. 24: 542,1994). According to this method, radiolabeled antigen binding protein is injected intravenously into mice and its plasma concentration is periodically measured as a function of time, for example, at 3 minutes to 72 hours after the injection. The clearance curve thus obtained should be biphasic. For the determination of the in vivo half-life of the modified IgGs or fragments thereof, the clearance rate in β-phase is calculated and compared with that of the unmodified IgG.

Antigen binding proteins may be assayed for the ability to immunospecifically bind to an antigen. Such an assay may be performed in solution (e. g., Houghten, BiolTechniques, 13: 412-421,1992), on beads (Lam, Nature, 354: 82-84,1991, on chips (Fodor, Nature, 364: 555-556,1993), on bacteria (U. S. Patent No. 5,223,409), on spores (U. S. Patent Nos. 5,571,698; 5,403,484; and 5,223,409), on plasmids (Cull et al., Proc. Natl. Acad. Sci. USA, 89: 1865-1869,1992) or on phage (Scott and Smith, Science, 249: 386-390,1990; Devlin, Science, 249: 404-406,1990; CuVirla et al., Proc. Natl. Acad. Sci. USA, 87: 6378-6382, 1990; and Felici, J : Mol. Biol., 222: 301-310, 1991). Antibodies that have been identified to immunospecifically bind to an antigen or a fragment thereof can then be assayed for their specificity affinity for the antigen.

The antigen binding proteins may be assayed for immunospecific binding to an antigen and cross-reactivity with other antigens by any method known in the art. Immunoassays which can be used to analyze immunospecific binding and cross-reactivity include, but are not limited to, competitive and non-competitive assay systems using techniques such as western blots, radioimmunoassays, ELISA (enzyme linked immunosorbent assay),"sandwich" immunoassays, immunoprecipitation assays, precipitin reactions, gel diffusion precipitin reactions, immunodiffusion assays, agglutination assays, complement-fixation assays, immunoradiometric assays, fluorescent immunoassays, protein A immunoassays, to name but a few. Such assays are routine and well known in the art (see, e. g., Ausubel et al, eds, 1994, Current Protocols in Molecular Biology, Vol. 1, John Wiley & Sons, Inc., New York,).

In one aspect, BIAcore kinetic analysis is used to determine the binding on and off rates of antibodies to an antigen. BIAcore kinetic analysis comprises analyzing the binding and dissociation of an antigen from chips with immobilized antibodies on their surface.

Antigen binding protein: The term "antigen binding protein" as used herein includes reference to antibodies, antibody fragments and other protein constructs, which are capable of binding to TNF-alpha.

Antibody: The term "antibody" is used herein in the broadest sense and includes reference to molecules with an immunoglobulin-like domain and includes monoclonal, recombinant, polyclonal, chimeric, humanised, bispecific and heteroconjugate antibodies.

Human IgG1 heavy chain constant domain: refers to human amino acid sequence for the IgG1 heavy chain constant domain that is found in nature, including allelic variations.

"Half-life (t1/2)" refers to the time required for the concentration of the antigen binding polypeptide to reach half of its original value. The serum half-life of proteins can be measured by pharmacokinetic studies according to the method described by Kim et al. (Eur. J. of Immuno. 24: 542, 1994). According to this method, radiolabeled protein is injected intravenously into mice and its plasma concentration is periodically measured as a function of time, for example, at about 3 minutes to about 72 hours after the injection. Other methods for pharmacokinetic analysis and determination of the half-life of a molecule will be familiar to those skilled in the art. Details may be found in Kenneth, A et al: Chemical Stability of Pharmaceuticals: A Handbook for Pharmacists and in Peters et al, Pharmacokinetic analysis: A Practical Approach (1996). Reference is also made to "Pharmacokinetics", M Gibaldi & D Perron, published by Marcel Dekker, 2nd Rev. ex edition (1982), which describes pharmacokinetic parameters such as t alpha and t beta half lives and area under the curve (AUC), and "Clinical Pharmacokinetics: Concepts and Applications", Rowland and Tozer, Third Edition (1995).

"Clearance (CL)" refers to the volume of plasma irreversibly cleared of a protein per unit time. Clearance is calculated as the Dose/AUC (AUC : is the Area Under Curve or Area under the plasma drug concentration time curve). Clearance can also be calculated by the rate of drug elimination divided by the plasma concentration of the drug (rate of elimination = CL*concentration)

"Mean Residence Time (MRT)" is the average time that the antigen binding polypeptides reside in the body before being irreversibly eliminated. Calculated as MRT= AUMC/AUC.

"Steady state concentration" (Css) is the concentration reached when the drug elimination rate becomes equal to drug administration rate as a result of continued drug administration. Css fluctuates between peak and trough levels and is measured in microgram/ml. "Mean steady-state trough concentration" refers to the mean of the trough level across the patient population at a given time.

"Comparable mean steady-state trough concentration" refers to mean steady-state trough concentration which is the same or within about 10% to 30% of the stated value. Comparable mean steady-state trough concentration for the antigen binding polypeptides may be considered to be those mean steady-state trough concentrations that are 0.8 to 1.25 times the mean steady-state trough concentration achieved with an IgG comprising the light chain sequence of SEQ ID No. 2 and the heavy chain sequence of SEQ ID No. 12.

Half lives and AUC can be determined from a curve of serum concentration of drug (for example the antigen binding polypeptide) against time. Half life may be determined through compartmental or non-compartmental analysis. The WINNONLIN™ analysis package (available from Pharsight Corp., Mountain View, CA94040, USA) can be used, for example, to model the curve. In one embodiment, "half life" refers to the terminal half life.

Specifically binds: The term "specifically binds" as used throughout the present specification in relation to antigen binding proteins means that the antigen binding protein binds to TNF-alpha with no or insignificant binding to other unrelated proteins. The term however does not exclude the fact that the antigen binding proteins may also be cross-reactive with closely related molecules. The antigen binding proteins described herein may bind to TNF-alpha with at least 2, at least 5, at least 10, at least 50, at least 100, or at least 1000 fold greater affinity than they bind to closely related molecules. CDRs:
"CDRs" are defined as the complementarity determining region amino acid sequences of an antigen binding protein. These are the hypervariable regions of immunoglobulin heavy and light chains. There are three heavy chain and three light chain CDRs (or CDR regions) in the variable portion of an immunoglobulin. Thus, "CDRs" as used herein refers to all three heavy chain CDRs, all three light chain CDRs, all heavy and light chain CDRs, or at least two CDRs.

Throughout this specification, amino acid residues in variable domain sequences and full length antibody sequences are numbered according to the Kabat numbering convention. Similarly, the terms "CDR", "CDRL1", "CDRL2", "CDRL3", "CDRH1", "CDRH2", "CDRH3" used in the Examples follow the Kabat numbering convention. For further information, see Kabat et al., Sequences of Proteins of Immunological Interest, 4th Ed., U.S. Department of Health and Human Services, National Institutes of Health (1987).

% identity of variants : The term "identical" or "sequence identity" indicates the degree of identity between two nucleic acid or two amino acid sequences when optimally aligned and compared with appropriate insertions or deletions. The variants described herein may have 90, 91, 92, 93, 94, 95, 96, 97, 98, or 99% identity to the native CDR or variable domain sequences at the amino acid level.

The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. In one aspect such open ended terms also comprise within their scope a restricted or closed definition, for example such as "consisting essentially of", or "consisting of".

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

All of the compositions and/or methods disclosed and claimed herein can be made and executed without undue experimentation in light of the present disclosure. While the compositions and methods of this invention have been described in terms of preferred embodiments, it will be apparent to those of skill in the art that variations may be applied to the compositions and/or methods and in the steps or in the sequence of steps of the method described herein without departing from the concept, spirit and scope of the invention. All such similar substitutes and modifications apparent to those skilled in the art are deemed to be within the spirit, scope and concept of the invention as defined by the appended claims.

The present invention is further described by reference to the following examples, not limiting upon the present invention.

### Examples

### Example 1: Cloning of antibody expression vectors

The DNA expression constructs encoding the variable heavy (VH) and variable light (VL) domains of an anti-TNFα antibody were previously prepared de novo and included restriction sites for cloning into mammalian expression vectors. Both heavy and light chain variable domain sequences were sequence optimised for expression in mammalian cells (for methodology see WO2009024567 and Kotsopoulou et al, J Biotechnol (2010) 146: 186-193). Information describing the heavy and light chain variable region sequences can be found in US patent US6090382. To generate the constructs used in this study, the variable heavy domain (VH) sequences were amplified using PCR. The PCR primers contained *HindIII* and *SpeI* restriction sites to frame the VH domain containing the signal sequence for cloning into a pTT mammalian expression vectors containing the human γ1 constant region. Similarly the VL domain sequence was amplified by PCR using primers containing *HindIII* and *BsiWI* restriction sites to facilitate cloning into a pTT mammalian expression vector containing the human kappa constant region. The heavy chain expression plasmid was given the code SJC322 and the light chain expression plasmid was given the plasmid code SJC321.

DNA expression constructs encoding alternative variable heavy and light chain regions of anti-TNFα antibodies with modifications in the CDR regions (as described in Rajpal et al. PNAS (2005) 102(24): pg 8466-8471) were prepared de novo by build up of overlapping oligonucleotides and similar molecular biology techniques to those described above. The resulting plasmids encoding the heavy and light chains of variants cb1-3, cb2-6 and cb2-44 are described in Table 1.

### Example 2: Engineering of the Fc region

Forward and reverse priming primers were used to introduce modifications (M252Y/S254T/T256E and T250Q/M428L) into the human γ1 constant region of the plasmid encoding the heavy chain of pascolizumab (anti-IL-4 antibody) using the Quikchange protocol (Promega).

As described in Example 1 above, a PCR fragment encoding the VH domain of an anti-TNFα antibody was generated using a previously constructed, codon optimised vector as a template. The resulting fragment was cloned using *HindIII* and *SpeI* into a pTT expression vector containing the modified human γ1 constant region described in the preceding paragraph. The plasmid encoding the heavy chain of the anti-TNFα antibody with the M252Y/S254T/T256E modification was designated SJC324. The plasmid encoding the heavy chain with the T250Q/M428L modification was designated SJC323.

Forward and reverse priming primers were used to introduce modifications into the human γ1 constant region of anti-TNFα heavy chain expression plasmid SJC322 using the Quikchange protocol (Promega). Plasmid SJC326 encodes the anti-TNFα heavy chain containing the M428L/N434S modification in the human γ1 constant region. Plasmid SJC328 encodes the anti-TNFα heavy chain containing the V308F modification in the human γ1 constant region.

### Example 3: Expression of antibodies in HEK2936E cells using pTT5 episomal vectors

Expression plasmids encoding the heavy and light chains described above were transiently co-transfected into HEK 293 6E cells. Expressed antibody was purified from the supernatant by affinity chromatography using a 1ml HiTrap Protein A column (GE Healthcare). Table 1 below shows the list of antibodies produced.

Some antibodies were also expressed in CHO cells using a different set of expression vectors. See Examples 13, 14 and 15 for a description of the molecular biology, expression and purification.

**Table 1: List of expressed antibodies**

| **BPC code** | **CDR variant** | **Fc modifications** | **Heavy chain expression vector** | **SEQ ID of heavy chain** | **Light chain expression vector** | **SEQ ID of light chain** |
|---|---|---|---|---|---|---|
| BPC1492 | None | Wild-type | SJC322 | 12 | SJC321 | 2 |
| BPC1494 | None | M252Y/S254T/T256 E | SJC324 | 5 | SJC321 | 2 |
| BPC1496 | None | M428L/N434S | SJC326 | 9 | SJC321 | 2 |
| BPC1493 | None | T250Q/M428L | SJC323 | 15 | SJC321 | 2 |
| BPC1498 | None | V308F | SJC328 | 18 | SJC321 | 2 |
| BPC1499 | cb1-3 | Wild-type | SJC336 | 150 | SJC339 | 147 |
| BPC1500 | cb2-44 | Wild-type | SJC337 | 151 | SJC340 | 148 |
| BPC1501 | cb2-6 | Wild-type | SJC336 | 150 | SJC338 | 149 |

### Example 4: Binding of antibodies to tumour necrosis factor alpha in a direct binding ELISA

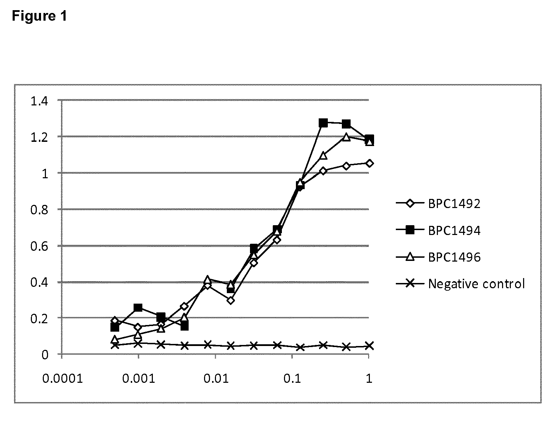

A binding ELISA was carried out to test the binding of the expressed antibodies purified using protein A to recombinant tumour necrosis factor alpha (TNFα). ELISA plates were coated with recombinant human TNFα at 0.1µg/ml and blocked with blocking solution (4% BSA. Various dilutions of the purified antibody were added (diluted in 4% BSA in T Tris-buffered saline at pH8.0 containing 0.05% Tween 20) and the plate was incubated for 1 hour at room temperature before washing in deionised water. Binding was detected by the addition of a peroxidase labelled anti human kappa light chain antibody (Sigma A7164) in blocking solution. The plate was incubated for 1 hour at room temperature before washing in deionised water. The plate was developed by addition of OPD substrate (Sigma P9187) and colour development stopped by addition of 2M HCl. Absorbance was measured at 490nm with a plate reader and the mean absorbance plotted against concentration. The results are shown in Figure 1 and confirm that all the antibodies have a similar profile.

### Example 5: Analysis of antibodies in an L929 in vitro neutralisation assay

This assay was used to test the neutralising ability of the antibodies to neutralise TNF-α and inhibit cell death. Briefly, L929 cells were seeded in a 96-well flat-bottomed plate at 10,000/well in 100µl RPMI 1640 (w/o phenol red) and incubated overnight at 37°C, 5% CO₂. Cells were sensitised with 1.25µg/ml actinomycin D for 1 hour. For the neutralising study, 0.001-60µg/ml (0.0067- 400 nM) anti-TNF-α mAb was pre-incubated with approx. 2ng/ml (approximately 0.05nM) TNF-α in a 1:1 ratio for 1 hour at room temperature. For control group, RPMI was used in place of the antibody. Following the 1 h pre-incubation with actinomycin D, 20 µl of antibody-antigen complex was added per well. 10ul media alone was added to wells as a negative control. Plates were incubated at 18 hour at 37°C, 5% CO₂. Following this treatment period, cell viability was determined by a cell titer-Glo Luminescent assay kit according to manufacturer's instructions (Promega, Madison USA). For L929 assay, the percentage cell viability of the unknowns was expressed as a percentage of the untreated group (taken as a 100%) and IC50 values were determined by Graphpad prism. Differences in IC50 values of antibodies was assessed by one-way ANOVA (Newman-Keuls post hoc test) and considered significant at P-values of less than 0.05. Data is represented as mean ± SEM, of n=4 experiments measured in duplicate. IC50 values for each antibody were determined and are listed in Table 2 below. The results show that the potency of all the antibodies tested are comparable.

**Table 2: IC₅₀ values for various anti-TNFα antibodies in an L929 neutralisation assay**

| **Antibody** | **IC₅₀ value (µg/ml)** |
|---|---|
| BPC1492 | 1.19 ± 0.10 |
| BPC1494 | 1.20 ± 0.13 |
| BPC1496 | 1.18 ± 0.10 |
| Adalimumab | 1.09 ± 0.07 |

Table 3 shows the IC50 values derived from the experiment. The results indicate that the improved anti-TNFα antibodies (BPC1499, BPC1500, BPC1501) show increased potency in this assay compared to BPC1492 and adalimumab.

**Table 3: IC₅₀ values for improved anti-TNFα antibodies in an L929 neutralisation assay**

| **Antibody** | **IC₅₀ value (µg/ml)** |
|---|---|
| BPC1492 | 1.19 ± 0.1 |
| BPC1499 | 0.21 ± 0.04 |
| BPC1500 | 0.13 ± 0.02 |
| BPC1501 | 0.21 ± 0.03 |
| Adalimumab | 1.09 ± 0.07 |

### Example 6: Effect of antibodies on in vitro IL-6 release

The neutralising ability of antibodies was determined by measuring their effect on inhibiting TNF-α mediated IL-6 release from whole blood cells. Briefly, 130 µL of whole blood was added to each well and plates were incubated at 37°C in a humidified 5% CO₂ incubator for 1 hour. For the neutralising study, 0.001-30µg/ml (0.0067- 200 nM) TNF-α mAb was pre-incubated with 10ng/ml (approx. 0.4 nM) TNF-alpha in a 1:1 ratio for 1 hour at 4°C. For control group, RPMI was used in place of the antibody. Following this pre-treatment, 20 µl of antigen-antibody complex or RPMI (negative control) was added per well and plates were incubated for 24 hour at 37°C, 5% CO₂. 100 µL PBS (w/o MgCl₂ or CaCl₂) added to each well and placed on plate shaker for 10 mins at 500 rpm. Plates were then spun at 2000 rpm for 5 mins. 120 µL supernatant was carefully removed and transferred to fresh 96-well round bottomed plate and IL-6 release was determined using an MSD based assay kit (Meso Scale Diagnostics, Maryland USA). For the whole blood assay, the MSD signal for each sample was read using a MSD SECTOR® Imager 2400 and IL-6 release from the cells was quantified using a standard data analysis package in PRISM 4.00 software (GraphPad. San Diego, USA). The percentage of IL-6 inhibition by each antibody was expressed as a percentage of the TNF-α alone treated group. Hence, dose response curves were obtained for each antibody and IC50 values were determined. Using the log of the IC50 values, the difference in potency of the antibodies was determined by one-way ANOVA (Newman-Keuls post hoc test) and considered significant at P-values of less than 0.05 for each donor (n=3). Data is represented as mean ± SEM of three donors, measured in duplicate. Table 4 below shows the IC50 values derived from these data. These results suggest that there is no significant difference in potency between the antibodies tested.

**Table 4: IC₅₀ values for various anti-TNF antibodies in a TNFα-induced IL-6 release assay**

| **Antibody** | **IC₅₀ value (nM)** |
|---|---|
| BPC1492 | 0.72 ± 0.32 |
| BPC1494 | 0.62 ± 0.11 |
| BPC1496 | 0.64 ± 0.13 |
| Adalimumab | 0.47 ± 0.09 |

The IC50 values are shown in Table 5. The results indicate that the improved anti-TNFα antibodies (BPC1499, BPC1500, BPC1501) show increased potency in this assay.

**Table 5: IC₅₀ values for various improved anti-TNF antibodies in a TNFα-induced IL-6 release assay**

| **Antibody** | **IC₅₀ value (nM)** |
|---|---|
| BPC1492 | 0.72 ± 0.32 |
| BPC1494 | 0.62 ± 0.12 |
| BPC1499 | 0.14 ± 0.02 |
| BPC1500 | 0.11 ± 0.05 |
| BPC1501 | 0.15 ± 0.03 |
| Adalimumab | 0.47 ± 0.09 |

### Example 7: Accelerated stressor studies

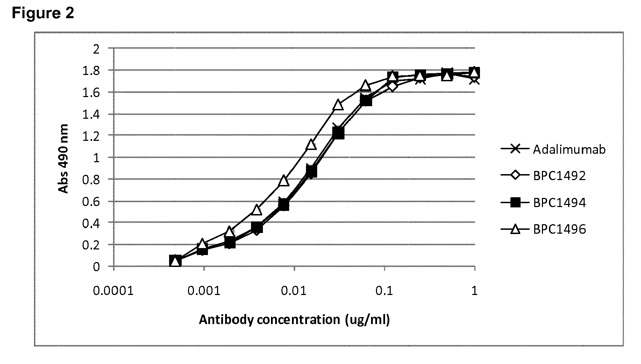

Prior to the study, antibodies to be tested were quantified on a spectrophotometer at OD280nm and diluted to 1.1mg ml in PBS (pH7.4). An aliquot was removed and 10%v/v of 500mM sodium acetate was added to give a final concentration of1mg/ml at pH5.5 and the sample inspected for precipitation. The remaining sample in PBS had 10% PBS v/v added to a final concentration of 1mg/ml at pH7.4 and an aliquot of this sample was removed to provide a baseline aggregation level (as monitored by size exclusion chromatography). The samples were then incubated at 37°C for two weeks in an incubator, after which the samples were re-quantified on a spectrophotometer at OD280nm and assessed (by size exclusion chromatography) for aggregation. The samples were tested for human TNFα binding in a direct binding ELISA. The results are shown in Figure 2 and confirm that the binding activity of all antibodies tested is comparable following the accelerated stressor study.

### Example 8: Stability study in 25% human serum

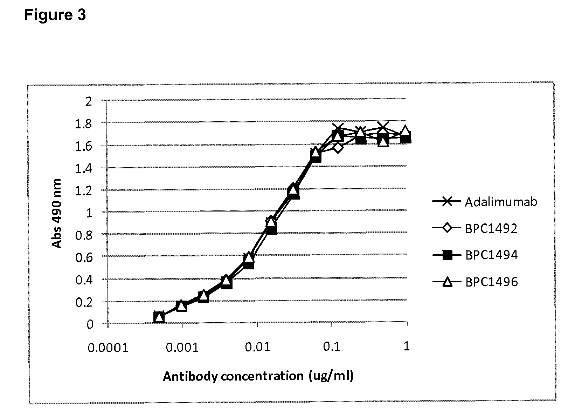

Prior to the study, antibodies to be tested were quantified on a spectrophotometer at OD280nm and diluted to 1.25 mg/ml in PBS (pH7.4). An aliquot was removed and 25%v/v of human serum was added to give a final concentration of 1mg/ml. The remaining sample in PBS had 25% PBS v/v added to a final concentration of 1mg/ml and an aliquot of this sample was removed to provide a baseline level. The samples were then incubated at 37°C for two weeks in an incubator, after which the samples were tested for human TNFα binding in a direct binding ELISA. The results are shown in Figure 3 and confirm that the binding activity of all antibodies tested is comparable following incubation in 25% human serum for two weeks.

### Example 9: Analysis of binding to human TNFα following freeze-thaw

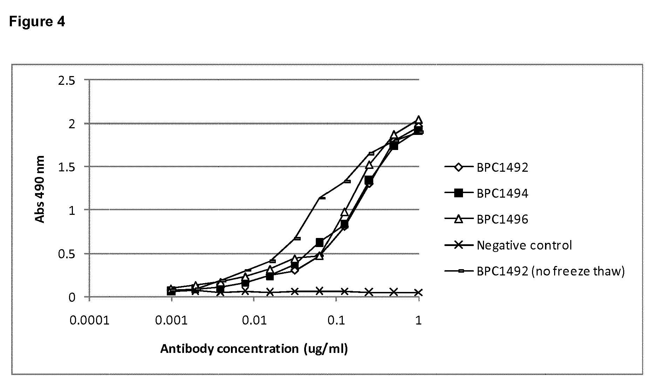

Antibody samples were diluted to 1mg/ml in a buffer containing 50mM Acetate and 150mM NaCl (pH6.0), snap-frozen in dry ice and then thawed at 4°C overnight. Binding of the antibodies to human TNFα was tested in comparison to an antibody which had not been snap-frozen.To assess the binding activity following freeze-thaw, ELISA plates were coated with recombinant human TNFα at 1µg/ml and blocked with blocking solution (4% BSA in Tris buffered saline). Various concentrations were added to the coated plates and incubated for 1 hour at room temperature before washing in deionised water. Binding was detected by the addition of a peroxidase labelled anti human kappa light chain antibody (Sigma A7164) in blocking solution. The plate was incubated for 1 hour at room temperature before washing in deionised water. The plate was developed by addition of OPD substrate (Sigma P9187) and colour development stopped by addition of 2M HCL. Absorbance was measured at 490nm with a plate reader and the mean absorbance plotted against concentration. The results are shown in Figure 4 and confirm that the binding activity of all antibodies tested is comparable following freeze-thaw.

### Example 10: Analysis of binding of anti-TNFα antibodies to FcγRIIIa

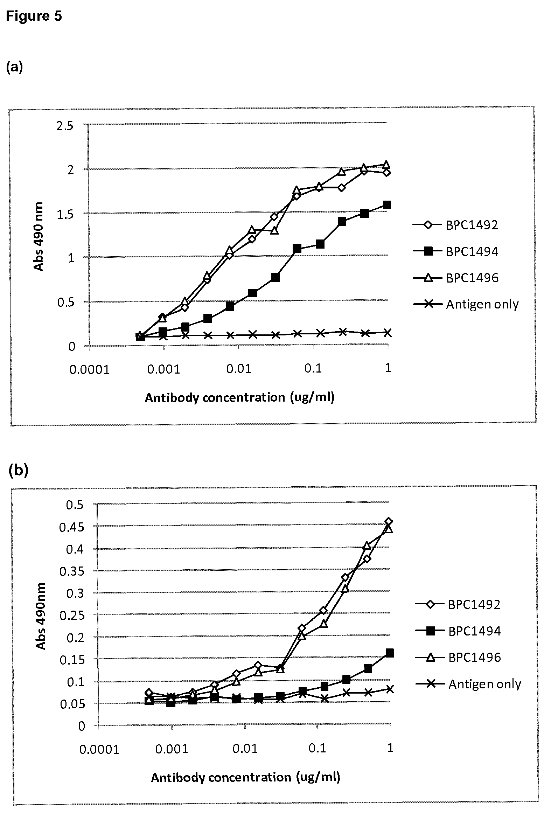

ELISA plates were coated with recombinant human FcγRIIIa (V158 and F158 variants) at 1µg/ml and blocked with blocking solution (4% BSA in Tris buffered saline). Various concentrations were added to the coated plates and incubated for 1 hour at room temperature before washing in deionised water. Binding was detected by the addition of a peroxidase labelled anti human kappa light chain antibody (Sigma A7164) in blocking solution. The plate was incubated for 1 hour at room temperature before washing in deionised water. The plate was developed by addition of OPD substrate (Sigma P9187) and colour development stopped by addition of 2M HCl. Absorbance was measured at 490nm with a plate reader and the mean absorbance plotted against concentration. The results are shown in Figure 5a and 5b and confirms that BPC1494 has reduced capacity to bind FcγRIIIa (V158 and F158 variants) compared to BPC1492 and BPC1496.

### Example 11: ProteOn Analysis: FcRn Binding

Antibodies for testing were immobilised to similar levels on a GLC biosensor chip (BioRad 176-5011) by primary amine coupling. Recombinant human and cynomolgus FcRn were used as analytes at 2048nM, 512nM, 128nM, 32nM, and 8nM, an injection of buffer alone (i.e. 0nM) was used to double reference the binding curves. Regeneration of the antibody surface following FcRn injection used HBS-N at pH9.0, the assay was run on the ProteOn XPR36 Protein Interaction Array System at 25°C and run in HBS-N pH7.4 and HBS-N pH6.0 with the FcRn diluted in appropriate buffer. Affinities were calculated using Equilibrium model, inherent to the ProteOn analysis software, using a "Global R-max" for binding at pH6.0 and the R-max from binding at pH6.0 for affinity calculation at pH7.4. Since the binding curves did not reach saturation at pH7.4, the values obtained are unlikely to be true affinities however they can be used to rank constructs. The results are shown in Table 6 and confirm that BPC1494 and BPC1496 have an improved affinity for human and cyno FcRn at pH6.0 when compared to BPC1492.

**Table 6 Affinities of Anti-TNF alpha constructs binding to Human and Cyno FcRn**

| **BPC Number** | **Human pH6.0 KD(nM)** | **Human pH7.4 KD(nM)** | **Cyno pH6.0 KD(nM)** | **Cyno pH7.4 KD(nW)** |
|---|---|---|---|---|
| BPC1492 | 554 | 21200 | 579 | 29700 |
| BPC1494 | 204 | 2320 | 239 | 2640 |
| BPC1496 | 144 | 1910 | 154 | 2100 |
| BPC1497 | 428 | 15500 | 464 | 20800 |
| BPC1498 | 357 | 5910 | 402 | 6280 |
| BPC1493 | 264 | 4390 | 295 | 4690 |

### Example 12: PK studies in human FcRn transgenic mice.

In a single dose pharmacokinetic study BPC1494 and BPC1492, were administered intravenously (IV) at 1 mg/kg to two different strains of FcRn humanised mice and one strain deficient in FcRn (Petkova et al. Int. Immunol (2010) 18(12): 1759-1769). Plasma samples were analyzed for BPC1494 or BPC1492, as appropriate, using a validated Gyrolab fluorescent immunoassay.

The methods used biotinylated human TNF alpha as the capture antigen and an Alexa labelled anti-human IgG (Fc specific) antibody as the detection antibody. Using an aliquot of mouse plasma diluted 1:10 with assay buffer, the lower limit of quantification (LLQ) was 100 ng/mL and the higher limit of quantification (HLQ) was100,000 ng/mL. Plasma concentrations below the lowest standards were considered to be not quantifiable. QC samples prepared at three different concentrations and stored with the study samples, were analysed with each batch of samples against separately prepared calibration standards. For the analyses to be acceptable, at least one QC at each concentration must not deviate from nominal concentration by more than 20%. The QC results from this study met these acceptance criteria.

PK analysis was performed by non-compartmental pharmacokinetic analysis using WinNonLin, version 6.1. All computations utilised the nominal blood sampling times. The systemic exposure to BPC1494 and BPC1492 was determined by calculating the area under the plasma concentration time curve (AUC) from the start of dosing until the last quantifiable time point (AUC₀₋ₜ) using the linear log trapezoidal calculation method. Further PK parameters could not be derived from the data due discrepancies in sample labelling.

**Table 7 - Summary pharmacokinetic parameters for BPC1494 and BPC1492 following a single intravenous administration (bolus) at a target dose of 1 mg/kg to transgenic mice**

| Compound | Strain | Cmax (ug/mL) | AUC (hr*ug/mL) |
|---|---|---|---|
| BPC1494 | 1 | 13.8 | 2240 |
| BPC1492 | | 14.8 | 1730 |
| BPC1494 | 2 | 12.0 | 1320 |
| BPC1492 | | 13.2 | 1060 |
| BPC1494 | 3 | 13.6 | 214 |
| BPC1492 | | 12.2 | 250 |

| | | | |
|---|---|---|---|
| Strain 1 = mFcRn-/- hFcRn (32) Tg/Tg Strain 2 = mFcRn-/- hFcRn (276) Tg/Tg Rag1-/- Strain 3 = mFcRn -/-/Rag1-/- Similar Cₘₐₓ concentrations were obtained for all groups. In both human FcRn knock-in mouse strains BPC1494 had a higher exposure (AUC₀₋ₜ) than BPC1492, although this difference was not notable (1.3 fold). In the absence of both human and mouse FcRn BPC1492 had a higher exposure than BPC1494. | | | |

### Example 13: Cloning of antibody expression vectors into pEF vectors

In some cases, the DNA encoding the expression cassettes for the heavy and light chains were excised from the vectors described in Example 3 using *HindIII* and *EcoRI* and cloned into pEF vectors, where expression occurs from the hEF1a promoter, using standard molecular biology techniques (for description of vectors see Kotsopoulou et al J. Biotechnol (2010) 146: 186-193).

**Table 8**

| BPC code | Fc modification | Heavy chain expression vectors | Light chain expression vector | Heavy chain SEQ ID No. | Light chain SED ID No. |
|---|---|---|---|---|---|
| BPC1492 | None | SJC330 | SJC329 | 12 | 2 |
| BPC1494 | M252Y/S254T/T256E | SJC331 | SJC329 | 5 | 2 |
| BPC1496 | M428L/N434S | SJC332 | SJC329 | 9 | 2 |

### Example 14: Expression of antibodies in CHO cells using pEF expression vectors

Expression plasmids encoding heavy and light chains were co-transfected into CHO DG44 cells and expressed at scale to produce antibody. For the generation of BPC1492 plasmids SJC329 and SJC330 were used. For the expression of BPC1494 plasmids SJC329 and SJC331 were used. For BPC1496 plasmids SJC329 and SJC332 were used.

Briefly, 30µg DNA (15µg heavy chain and 15µg light chain) was linearised overnight with *Not1* restriction enzyme. The resultant restricted DNA was then ethanol precipitated and re-dissolved in TE buffer. From culture, 6X10⁶ CHO DG44 cells were obtained and washed in 10ml of PBS. The cell pellet was then re-suspended in 300µl of Amaxa solution V. 100µl of the aforementioned cell suspension was then added into to each of three Amaxa cuvettes, which also contained 3µg of the linearised DNA. The cuvettes were inserted into an Amaxa nucleofector II device and electroporated with pre-set programme U-023. The contents of the three cuvettes (300µl) of electroporated cells were added to 10ml of warmed MR14 medium (including nucleosides and BSA) and incubated in a T75 flask for 48 hours. Following this period, the medium was changed to nucleoside-free-MR14 (MR14 containing only BSA)). Every 3-4 days, conditioned medium was removed and replaced with fresh selection medium. Once cells had undergone recovery, the medium was substituted to 2X MR14 and IgG expression was confirmed by nephlometry. 2L shake-flasks were seeded with 1L of the IgG-expressing cells at 0.6X10⁶/ml and grown for 7 days. Cells were separated from supernatant by centrifugation and the supernatant was used for protein purification. 1 litre cell culture supernatants were purified using a 2-step automated process on an AKTA Xpress system. The antibody was captured on a 5ml MabSelectSure column and then washed prior to elution. The eluted antibody was then loaded onto a 440ml Superdex 200 gel filtration column and 2ml fractions collected in a 96-well block. Fractions of purified antibody were pooled and 0.2µm filtered and then concentrated to ~5mg/ml using Amicon spin concentrators. The final material was again 0.2µm filtered and then dispensed into sterile tubes for delivery. The final material was subject to analytical SEC to determine aggregation, an endotoxin assay, LC-MS for accurate mass determination (included PNGaseF and untreated material to determine glycosylation), SDS PAGE electrophoresis, PMF for sequence confirmation and A280 for concentration determination.

### Example 15: Alternative method for expression of antibodies in CHO cells using pEF expression vectors

DHFR-null CHO DG44 cells were obtained from Dr. Chasin of Columbia University. These cells were subsequently adapted to a chemically defined medium. These adapted host cells were designated DG44-c and are cultured in proprietary chemically defined medium supplemented with Glutamax and HT-supplement.

Generation of the polyclonal pool: For more details on protocols see WO2009024567 and Kotsopoulou et al, J. Biotechnol (2010) 164(4): 186-193. Briefly, DG44-c cells were transfected with plasmids encoding the heavy and light chains and DHFR and neoR respectively by electroporation (using the Amaxa nucleofector system). At 48 hours post transfection, selection was initiated by addition of G418 (at a final concentration of 400µg/ml) and removal of HT. When viability and cell counts increased sufficiently (in this case 2 months post transfection) methotrexate (MTX) was added at a final concentration of 5nM. Cells were scaled up and production curves were initiated 9-16 days after addition of MTX. For these production curves cells were seeded at 0.6-0.8x10⁶ cells/ml in chemically defined media and were fed on days 6, 9 or 10, 12 or 13 and/or 16. Supernatant was collected when viability dropped to approximately 50% and the cells were removed by centrifugation at 4000g for 30 mins followed by filtration through a sartobran capsule.

Antibodies were purified at room temperature using a two step chromatographic procedure: Initial capture was performed using a 50ml MabSelect SuRe column (GE Healthcare) followed by Size Exclusion Chromatography (SEC) with a 1.5L Superdex 200 pg SEC (GE Healthcare). The conditioned media was loaded onto a pre-equilibrated MabSelect SuRe column at a flow rate of 9cm/h. Following washing to base line with equilibration buffer (50mm Tris pH 8.0, 2M NaCl) the column was washed with a low salt buffer buffer (50mM NaCl Tris pH 8.0, 150mM NaCl) until conductivity was stable. The column was then eluted with elution buffer (25mM Citrate pH 2.5). Fractions corresponding to peak protein elution were immediately neutralized with 1/10 vol. 1.0M Tris pH 8.0 which were then pooled and filtered through a 0.2µm bottletop filter. The recovered sample was loaded at 21cm/h onto the SEC column pre-equilibrated with SEC buffer (50mM Na Acetate, 150mM NaCl). The fractions containing the main (monomeric) protein peak were pooled and filter sterilized.

Antibodies prepared by this method were used for analytical comparability studies summarised in the following example.

### Examples 16: Analytical comparability on stressed and control samples

Size exclusion chromatography was carried out to determine the aggregation levels of the protein. The optimised method involved injection of the sample onto a TOSOH TSK G3000SWXL column which had been equilibrated in 100 mM sodium phosphate, 400 mM NaCl, pH 6.8. Absorbance was measured at both 280nm and 214nm. Reverse-phase HPLC separates proteins and their isoforms based on hydrophobicity. Protein was injected onto a PLRP-S 1000 °A 8µm column and eluted using a gradient produced by 50%Formic acid, and 95% Acetonitrile. Absorbance was measured at 280nm. The purity of the molecule is reported as a percentage of the main peak area relative to the total peak area. Different isoforms of the mAb were separated on the basis of their pl values using capillary isoelectric focussing (clEF). IEF separation was performed on a 10cm, UV280 transparent cartridge capillary. The optimised method involved a solution containing 5% pH 3-10 ampholytes, 10mM NaOH, protein of interest and internal pl markers (7.05 and 9.5) which was loaded into the capillary by pressure injection.

The specific activity of antibodies (adalimumab, BPC1494, BPC1496) was determined using MSD. In brief, 96-well plates were coated with 50µL per well TNFα diluted to 1 µg/mL in PBS. The plate was incubated on the bench top at ambient temperature without shaking for 2 hours. The coating solution was removed and the plate was blocked with 50µL per well of 1% BSA in PBS, with 0.05% Polysorbate 20. The plate was incubated for 1 hour at 24°C with shaking at 400 rpm and then washed 4 times with wash buffer. The antibodies were diluted in 0.1 % BSA in PBS with 0.05% Polysorbate 20 and 30µl of each sample was added to the plate. The plate was incubated for 1 hour at 24°C with shaking at 400 rpm. The plate was then washed 4 times with wash buffer. Anti-human IgG sulfotag was diluted 1 in 5000 in assay buffer. 30µL was added to each well of the plate and then incubated for 1.5 hour at 24°C, with shaking at 400 rpm. The plate was then washed 4 times with wash buffer. The 4x MSD Read Buffer concentrate was diluted to 1x using deionised water. 100µL was then added per well of the plate. The plate was then read using the MSD Sector Imager instrument. From the signals obtained from the assay, specific activities of the molecules were calculated.

### Deamidation analysis

Deamidation is a common post-translational modification that can occur to asparagine and glutamine residues, but is most commonly observed with asparagine residues, particularly when adjacent to a glycine residue. In order to examine how susceptible these residues are and to determine the effects of deamidation on potency, adalimumab, BPC1494 and BPC1496 were exposed to a stress study. The stress was carried out by incubation in 1% ammonium bicarbonate at pH 9.0, for 48 hrs, conditions which have previously been shown to cause deamidation. The stressed samples were incubated alongside a control (in PBS) and were compared to this as well as an unstressed reference and analysed using c-IEF, SEC and Binding ELISA. Forced deamidation was also done on all samples in the presence and absence of EDTA. It has been shown previously that forced deamidation conditions cause fragmentation in addition to deamidation. EDTA prevents and or minimizes the fragmentation.

### Oxidation analysis

Oxidation of various residues can occur throughout the processing and storage of proteins; however the most commonly oxidised residue is methionine, which was the focus of this screen. Oxidation susceptibility of these residues was examined through exposure to stress conditions by incubation in 5mM and 50 mM H₂O₂ for 30minutes and evaluated using RP-HPLC, SEC and ELISA.

### Summary of results

Both BPC1494 and BPC1496 behave very favourably compared to adalimumab as shown by analytical comparability on both stressed and control samples. For all antibodies tested, no significant degradation was observed under forced oxidation conditions as shown by all analytical techniques employed. Significant deamidation as measured by c-IEF was observed at pH 9.0 as expected for all antibodies tested. In addition we saw significant fragmentation for all antibodies tested as shown by SEC at pH 9.0 in samples without EDTA, this is also as expected. There is a reduction in the pl value, (approximately 0.2) of BPC1494 when compared to adalimumab. This is attributed to the presence of an additional glutamic acid residue in the heavy chain sequence of the BPC1494 thus making it more acidic. Forced deamidation and oxidation had minimal impact on binding and this was observed for BPC1494, BPC1496 and adalimumab.

### Example 17: Analysis of binding of improved antibodies by ELISA

Antibodies BPC1499, 1500 and 1501 were assessed for binding activity by ELISA as described in Example 4. Using two different antigen coating concentrations (0.1 and 1.0 µg/ml), the antibodies did not show any difference in their binding profile when compared with BPC1492. Under the conditions tested, it appears that the ELISA does not discriminate between antibodies with different reported binding activities. The same antibodies were assessed using methodologies described in Examples 18, 5 and 6 which are considered more sensitive assays. In these assays, antibodies BPC1499, 1500 and 1501 show improved binding affinity and improved potency when compared with BPC1492.

### Example 18: Biacore Analysis of TNF alpha binding using a Capture surface

Protein A and anti-human IgG (GE Healthcare BR-1008-39) were coupled on separate flow cells on a CM3 biosensor chip. These surfaces were used to capture the antibodies for binding analysis. Recombinant human and cynomolgus TNF alpha were used as analytes at 64nM, 21.33nM, 7.11nM, 2.37nM, 0.79nM, an injection of buffer alone (i.e. 0nM) used to double reference the binding curves. Regeneration of the capture surface was carried out using 100mM phosphoric acid and 3M MgCl₂. The run was carried out on the Biacore T100 machine at 37°C using HBS-EP as running buffer. The constructs BPC1494 and BPC1496 showed reduced binding to Protein A and the anti-human IgG surface making these surfaces unsuitable for generating kinetics for those molecules.

**Table 9 Kinetic Analysis of Human and Cyno TNF alpha Binding to Captured Anti-TNF alpha Antibodies.**

| **Construct** | **Analyte** | **Capiture Surface** | **ka(1/Ms)** | **kd(1/s)** | **KD(nM)** |
|---|---|---|---|---|---|
| BPC1492 | human TNFα | Protein A | 2.12E+06 | 1.10E-04 | 0.05196 |
| BPC1494 | human TNFα | Protein A | Data | not | Analysable |
| BPC1496 | human TNFα | Protein A | Data | not | Analysable |
| BPC1500 | human TNFα | Protein A | 2.68E+06 | 4.19E-05 | 0.01561 |
| BPC1492 | human TNFα | anti-human IgG | 6.78E+06 | 1.73E-04 | 0.02554 |
| BPC1494 | human TNFα | anti-human IgG | Data | not | Analysable |
| BPC1496 | human TNFα | anti-human IgG | Data | not | Analysable |
| BPC1500 | human TNFα | anti-human IgG | 4.51E+06 | 7.07E-05 | 0.01568 |
| BPC1492 | Cyno TNFα | Protein A | 1.10E+06 | 1.11E-04 | 0.101 |
| BPC1494 | Cyno TNFα | Protein A | Data | not | Analysable |
| BPC1496 | Cyno TNFα | Protein A | Data | not | Analysable |
| BPC1500 | Cyno TNFα | Protein A | 2.34E+06 | 3.51E-05 | 0.01503 |
| BPC1492 | Cyno TNFα | anti-human IgG | 1.96E+06 | 3.75E-04 | 0.1911 |
| BPC1494 | Cyno TNFα | anti-human IgG | Data | not | Analysable |
| BPC1496 | Cyno TNFα | anti-human IgG | Data | not | analysable |
| BPC1500 | Cyno TNFα | anti-human IgG | 4.48E+06 | 2.09E-04 | 0.04667 |

### Example 19: ProteOn Reverse Assay Binding Analysis

Biotinylated TNF alpha was mixed with biotinylated BSA at a 1:49 ratio, at a final total protein concentration of 20µg/ml (i.e. 0.4µg biotinylated TNF alpha and 19.6µg biotinylated BSA). This mixture was captured on a NLC biosensor chip (a single flowcell) (Biorad 176-5021). The chip surface was conditioned with 10mM glycine pH3.0 till a stable signal was achieved. The antibodies to be tested were used as analytes at 256nM, 64nM, 16nM, 4nM and 1nM and 0nM. The binding curves were referenced against a flowcell coated with biotinylated BSA alone. Regeneration was achieved using 10mM glycine pH3.0. Data was fitted to the 1:1 model inherent to the ProteOn analysis software.

**Table 10 Apparent Kinetics of Anti-TNF alpha antibodies binding to Neutravidin Captured TNF alpha**

| **BPC Number** | **ka (1/Ms)** | **kd (1/s)** | **KD (nM)** |
|---|---|---|---|
| BPC1499 | 2.27E+06 | 1.72E-05 | 0.008 |
| BPC1500 | 2.06E+06 | 3.00E-05 | 0.015 |
| BPC1501 | 1.17E+06 | 6.97E-05 | 0.06 |
| BPC1496 | 6.33E+05 | 4.04E-04 | 0.639 |
| BPC1494 | 7.23E+05 | 3.50E-04 | 0.484 |
| BPC1492 | 7.89E+05 | 3.21 E-04 | 0.407 |

This data is one set of two experiments which were carried out (second set not shown). The KD ranking of the data is representative of both data sets.

### Example 20: Construction of alternative antibodies which bind to human TNFα

The DNA expression constructs encoding additional variable heavy regions with modifications in the CDR regions (as described in Rajpal et al. PNAS (2005) 102(24): pg 8466-8471) were prepared de novo by build up of overlapping oligonucleotides and similar molecular biology techniques to those described in Example 1. Examples of DNA sequences encoding the variable heavy domains of these variant antibodies are given in SED IQ NO: 81, 83, 85, 87, 89, 91, 93 and 95. The DNA expression constructs encoding additional variable light domain regions with modifications in the CDR regions (as described in Rajpal et al. PNAS (2005) 102(24): pg 8466-8471) were prepared de novo by build up of overlapping oligonucleotides and similar molecular biology techniques to those described in Example 1. Examples of DNA sequences encoding the variable light domains of these variant antibodies are given in SED IQ NO: 97, 99, 101, 103, 105, 107, 109, 111, 113, 115, 117, 119, 121, 123, 125, 127, 129, 131, 133, 135 and 137. Once constructed, the expression plasmids encoding the heavy and light chains were transiently co-transfected into HEK 293 6E cells. Expressed antibody were purified from the supernatant and assessed for activity using the methods similar to those described in Example 6..

### Example 21: Construction of expression vectors for BPC2604 (Pascolizumab-YTE)

The pTT-based DNA expression constructs encoding the heavy chain of pascolizumab was engineered to include the following changes M252Y/S254T/T256E (EU index numbering) using the Quikchange protocol (Promega).

### Example 22: Expression/purification of Pasco and Pasco-YTE vectors

Expression plasmids encoding the heavy and light chains of BPC2604 were transiently co-transfected into HEK 293 6E cells. Expressed antibody was purified from the bulk supernatant using a two step purification carried out by affinity chromatography and SEC using a 5ml MabSelectSure column and Superdex 200 column on an AKTA Xpress.

### Example 23: BIAcore analysis of Pasco vs. Pasco YTE for FcRn binding

Antibodies were immobilised on a GLM chip (20µg/ml in acetate pH4.5) by primary amine coupling. Human, cynomolgus, rat and mouse FcRn receptors used at 2048, 512, 128, 32 and 8nM. 0nM used for double referencing. Assay were carried out in HBS-EP pH7.4 and HBS-EP pH6.0 (FcRn receptor diluted in appropriate running buffer for each pH. The surface was regenerated for FcRn binding with 200mM Tris pH9.0. Data was fitted to an equilibrium model, with R-max set to highest R-max obtained of any construct. The results are shown in Table 11 below and confirm that the YTE-modified pascolizumab (BPC2604) shows improved binding to FcRn at pH6.0 compared to pascolizumab.

**Table 11: Affinities of anti-IL-4 antibody constructs for Human and Cyno FcRn (n.a.b. is no analysable binding)**

| | | **KD (nM) at pH6.0: R-max = 1020** | | | | **KD (nM) at pH7.4: R-max = 1020** | | | |
|---|---|---|---|---|---|---|---|---|---|
| Antibody | Fc modification | Human FcRn | Cyno FcRn | Mouse FcRn | Rat FcRn | Human FcRn | Cyno FcRn | Mouse FcRn | Rat FcRn |
| BPC2604 | M252Y/S254T/T256E | 98 | 92.1 | 53.4 | 66.0 | 11600 | 11100 | 2160 | 4330 |
| Pascolizumab | None | 541 | 505 | 205 | 228 | n.a.b | n.a.b | n.a.b | n.a.b |

### Example 24: PK studies with Pasco vs. Pasco-YTE

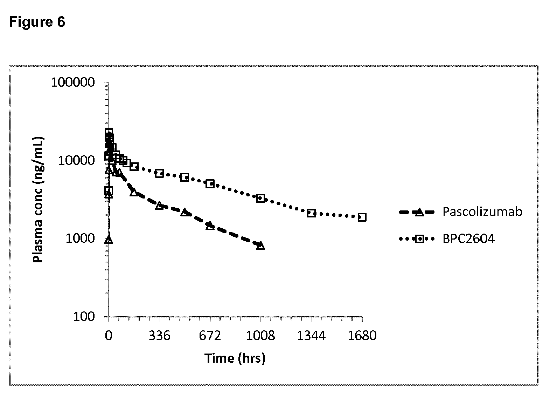

Figure 6 shows the average dose normalised plasma concentrations of pascolizumab-YTE (BPC2604)*)* in female cynomolgus monkeys and pascolizumab in male cynomolgus monkeys following a single intravenous (1 hr infusion) administration at a target dose of 1 mg/kg. The data for BPC2604 and pascolizumab were generated in separate studies. Plasma antibody concentrations for pascolizumab and BPC2604 were assessed by chemi-luminescence ELISA using IL-4 as the capture reagent and anti-human IgG (Fc specific)-HRP conjugate as the detection reagent. The validated range for the assay was 50-5000 ng/mL. The results are shown in Figure 6. Both compounds had similar Cmax but BPC2604 had a 3-fold lower plasma clearance resulting in 3-fold increase in AUC and 2-fold increase in half-life (T½).

### Example 25: Formulation studies at 5mg/ml

The stability of adalimumab and the TNF-alpha variant BPC1494 in two formulations was compared. Formulation 'A' (citrate-phosphate buffer) is the marketed adalimumab formulation made up of 6.16 mg/ml Sodium chloride + 0.30 mg/ml Sodium citrate monobasic + 1.30 mg/mL Citric acid monohydrate + 12 mg/ml Mannitol + 0.86mg/mL Monobasic sodium phosphate dihydrate + 1.53mg/mL Dibasic sodium phosphate dihydrate + 1.0 mg/ml PS80 at pH 5.2.

Formulation 'B' (acetate buffer) is composed of 6.81 mg/mL (50mM) Sodium Acetate trihydrate + 10mg/mL (1% w/v) Arginine + 0.0186mg/mL (0.05mM) EDTA + 2.98mg/mL (51mM) Sodium Chloride + 0.2mg/mL (0.02% w/v) Polysorbate 80, adjusted to pH 5.5 using HCIlor NaOH.

The TNF-alpha variant BPC1494 material used in this study was made in a Chinese Hamster Ovary (CHO DG44) cell line and purified using a two step process involving mAb Select Sure followed by Superdex column 200pg. Adalimumab (Product code NDC 0074-3799-02, Lot number 91073LX40) manufactured by Abbott Laboratories was used.

Adalimumab was re-formulated into Formulation 'B' by overnight dialysis at 5°C using a 10KDa Slide - A - Lyzer cassette (Product Number 66830, Lot Number LJ150514); produced by Thermo Scientific (Rockford, IL ;USA). This experiment was carried out at a different time point to the other three formulations.Both Adalimumab in Formulations 'A' and 'B' were diluted to 5mg/mL using their respective formulation buffers. The TNF-alpha variant BPC1494 molecule was also formulated in Formulations A and B at ~5mg/mL. A total of 4 samples were filtered through a MillexGV 0.22um filter under a clean laminar flow condition before being transferred into labelled pre-sterilized glass vials and incubated at 5°C, 25°C and 40°C for up to 14 weeks. Samples were taken at selected time points and analysed using SEC-HPLC (Table 12), clEF (Table 13). Other assays as described below were also carried out to assess the stability of the antibodies.

### Appearance by visual observation

Samples were inspected for clarity under daylight conditions. Both antibodies in each formulation remained unchanged (clear colourless solution) after 14 weeks storage at 5°C, 25°C and 40°C.

### Protein concentration (A280nm) Measurement

Protein concentration was measured using a nanodrop spectrometer, which is indicative of protein stability. The extinction coefficient for adalimumab is 1.46 and for TNF-alpha variant BPC1494 is 1.48. There was no significant difference in the results after 14 weeks storage at 5°C, 25°C and 40°C.

### pH

pH was measured for all samples stored under different storage conditions to determine whether any significant pH drifts had occurred. All results remained within assay variability after 14 weeks storage at 5°C, 25°C and 40°C.

### Size exclusion chromatography (SEC)

This method separates soluble protein molecules in the solution based on size and not molecular weight. In theory, small molecules will penetrate every small pore of the stationary phase and hence will elute later. The chromatogram obtained enables the determination of percentage area of aggregates, monomer and low molecular weight (MW) fragments. The presence of aggregates and/or low molecular weight species is indicative of protein degradation. Increased stability corresponds to a high percentage of monomeric species (Mono) together with a low percentage of Total Aggregates (TA) and Total Low Molecular weight Fragments (TLMWF).

SEC-HPLC data (Table 12) shows that the TNF-alpha variant BPC1494 was relatively more stable in formulation 'B' compared to formulation A after storage at 25°C and 40°C for 14 weeks. Furthermore, TNF-alpha variant BPC1494 was relatively more stable, or at least as stable as adalimumab in formulation A. The results for adalimumab in formulation B are all within 5% TA and/or TLMWF. Therefore, formulation B has advantages over formulation A for both TNF-alpha variant BPC1494 and adalimumab.

For example, Table 12 shows that after storage at 25°C for 8 weeks, TNF-alpha variant BPC1494 in formulation A has 2.3% TLMWF while formulation B produced only 1.5%. Furthermore, TNF-alpha variant BPC1494 in formulation B was relatively more stable than adalimumab in formulation A (1.8% TLMWF). Similarly, at the 14 week time point at 25°C, 3.15% TLMWF was observed for TNF-alpha variant BPC1494 in formulation 'A' compared to 2.3% TLMWF in formulation 'B'. Furthermore, TNF-alpha variant BPC1494 in 'B' was relatively more stable than adalimumab in formulation A (3.4% TLMWF). A similar trend for TLMWF was observed for both molecules on incubation at 40°C for 4 weeks (adalimumab in 'A': 3.6%; TNF-alpha variant BPC1494 in 'A': 4.1%; TNF-alpha variant BPC1494 in 'B': 2.6%).

Also, results for Total Aggregate (TA) show that at 14 weeks at 25°C, the TNF-alpha variant BPC1494 was relatively more stable in 'B' (0.3%) than in 'A' (0.5%); and relatively more stable than adalimumab in formulation A (0.4%).

### Capillary Iso-Electric Focusing (c-IEF)

This technique is used for determining the charge profile of molecules. A broad pl range reflects greater charge heterogeneity of the Product and in addition a broad pl range may be indicative of degradation. Typically the number of peaks will increase with increased degradation. The C-IEF data of Table 12 supports the SEC findings in Table 13.

The % area of main isoform (%AMI) was comparable between adalimumab in formulation A and TNF-alpha variant BPC1494 in formulation B at Weeks 8 and 14 at 25°C (56.0-57.7 and 53.2 respectively). At these time points and temperature, formulation B shows a slight advantage over 'A' for TNF-alpha variant BPC1494.

Similarly, adalimumab is relatively more stable in formulation 'B' than in formulation 'A' (see Week 4 data). For example, increased changes in charge heterogeneity (i.e. increase in number of peaks) were observed for adalimumab incubated for up to 4 weeks at 40°C in formulation 'A' compared to formulation 'B' (8 peaks and 6 peaks respectively). TNF-alpha variant BPC1494 showed a more consistent charge heterogeneity of 5 peaks at all timepoints and temperatures.

### Functional binding assay

The binding activity of adalimumab and TNF-alpha variant BPC1494 in the two formulations was assessed by Biacore. Over a 14 week period of storage at 5°C, 25°C and 40°C, the samples showed similar %binding within assay variability.

Hence, it can be concluded that formulation 'B' can serve as an alternative to formulation 'A' in a clinical setting without compromising the stability of the protein and potentially eliminating the pain associated with the marketed adalimumab formulation (A).

Importantly, this data shows that not only does the acetate formulation (B) improve the stability of the TNF-alpha variant BPC1494 compared to the citrate-phosphate formulation (A); but the acetate formulation is comparable or slightly better than the citrate-phosphate formulation when stabilising adalimumab.

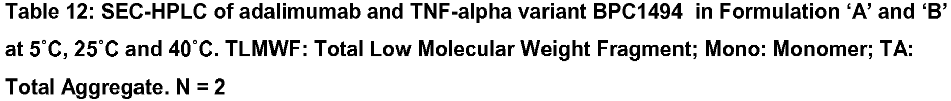

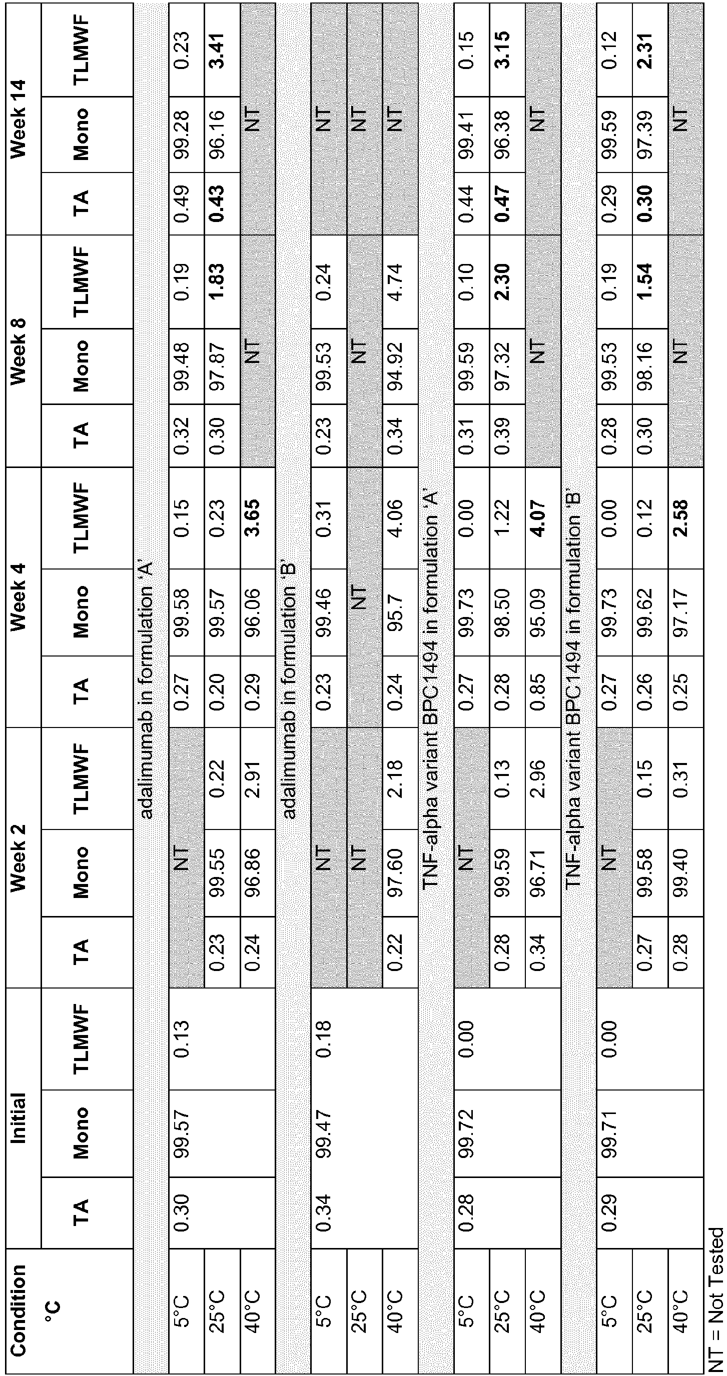

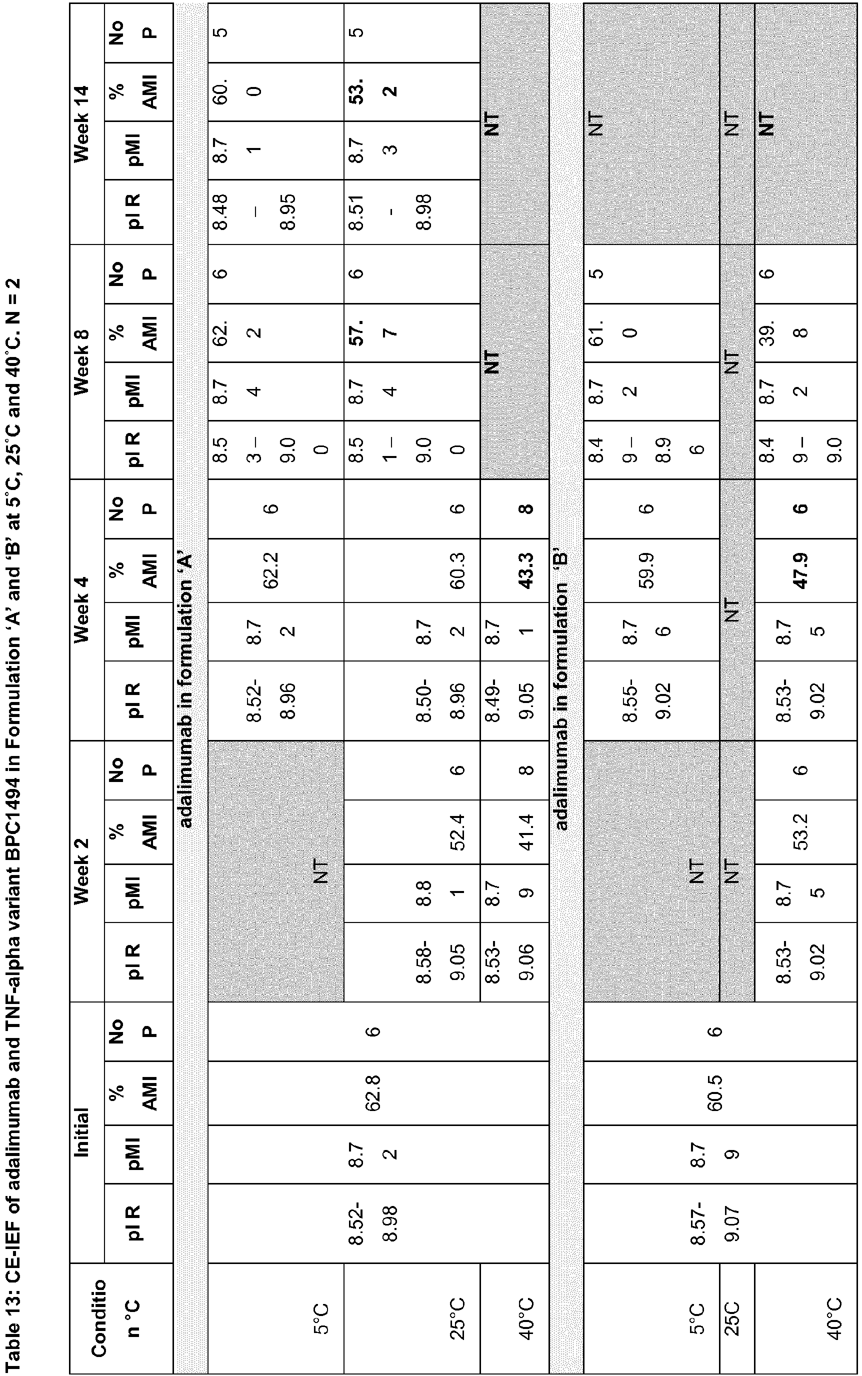

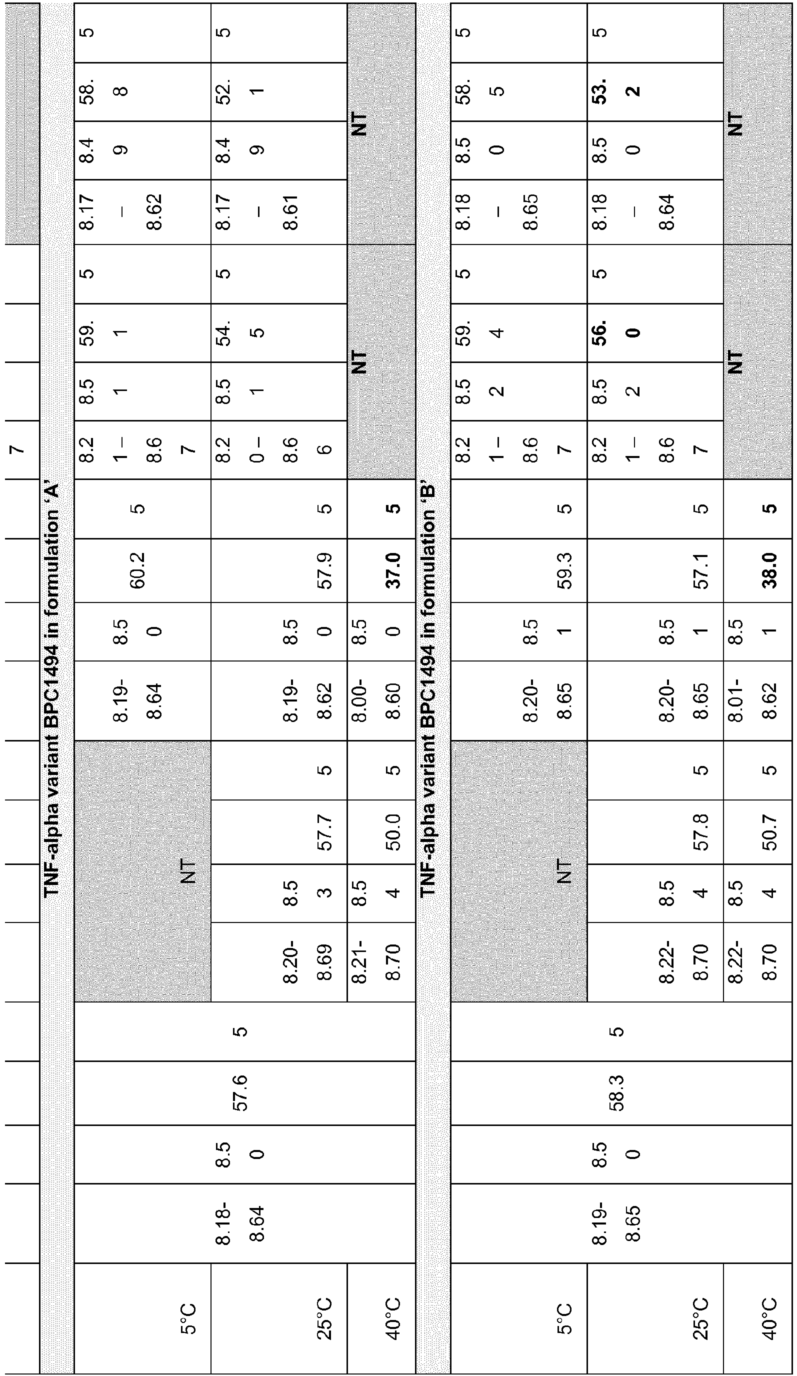

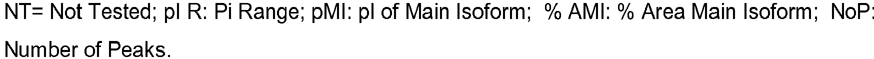

### Example 26: Formulation studies at 50mg/ml

As shown in the previous example 25, adalimumab and TNF-alpha variant BPC1494 at 5mg/mL in formulation 'B' can serve as an alternative to formulation 'A'. This example is focused on comparing the stability of adalimumab in its marketed formulation 'A' compared to formulation 'B' and other TNF-alpha variants at 50mg/ml.

Two samples of TNF-alpha variant BPC1494 were analysed, one expressed in CHO DG44 cells and one expressed in CHOK1 cells. A second TNF-alpha variant BPC1496 was made in a CHO-DG44 cell line. All three samples were expressed and purified using mAb Select Sure. In contrast to Example 25, no Superdex column step was carried out. Adalimumab (Product code N 00515-01, Lot number 02136XH12) manufactured by Abbott Laboratories, as in Example 25. Adalimumab was formulated in formulations 'A' (as purchased) and 'B' (by buffer exchange) as described above in Example 25, and the TNF-alpha variants (BPC1494 and 1496) were formulated in 'B', all at ~50mg/mL (total of 5 samples). The samples were filtered with MillexGV 0.22um filter under clean laminar flow conditions before being transferred into labelled pre-sterilized glass vials and incubated at 5°C and 40°C for up to 9 weeks. At selected time-points, samples were taken and analysed using SEC-HPLC (Table 14), clEF (Table 15). Other assays as described below were also carried out.

### Appearance by visual observation.

Samples were observed for clarity under daylight conditions. Both antibodies in both formulations remained unchanged (clear colourless solution) after 9 weeks storage at 5°C and 40°C.

### Protein concentration (A280nm) Measurement

Protein concentration was measured using a nanodrop spectrometer, which is indicative of protein stability. There was no significant difference in the results after 9 weeks storage at 5°C and 40°C.

### Size exclusion chromatography (SEC)

SEC-HPLC data (Table 13) showed that adalimumab at 50mg/ml was relatively more stable in formulation 'B' compared to formulation 'A' after storage at 40°C for 9 weeks. Also, the TNF-alpha variants (BPC1494 and 1496) were relatively as stable or more stable in 'B' as adalimumab in 'B'. No comparison between the variants in 'A' and 'B' was carried out.

Note that the Initial TA levels for the TNF-alpha variants were relatively higher than for adalimumab. Therefore, the results include a % change column at the right hand side to compare the changes from Initial to Week 9 at 40°C. For example, table 13 shows that after 9 week storage, the percentage change in total low molecular weight fragment (TLMWF) in formulation 'B' was between 3.82-4.96% compared to 6.08% in formulation 'A'. Similarly, the monomer percentage change in formulation 'A' was greater for adalimumab than for 'B' (7.54 and 4.52% respectively). The TNF-alpha variants in 'B' were all relatively at least as stable or more stable as adalimumab in formulation 'A' (% change at Week 9). The results at week 4 for all samples are within the 5% TA and/or TLMWF allowance for a commercial product. Therefore, 'B' has advantages over 'A' for both TNF-alpha variants and adalimumab at 50 mg/ml.

In particular, the TNF-alpha variant BCP1496 showed a low TLMWF value of 3.86 at Week 9 at 40°C.

### Capillary Iso-Electric Focusing (c-IEF)

C-IEF data (Table 15) supports the findings in Table 14.

Formulation B shows a reduced % change of %AMI at week 9 for adalimumab as compared to Formulation A (23.53 and 27.57 respectively).

The TNF-alpha variants in 'B' are more stable in terms of charge heterogeneity (i.e. increase in number of peaks) than adalimumab (in both 'A' and 'B'). For example, at Week 9 there were 5 and 6 peaks for each of the variants; and 6 and 9 peaks for adalimumab, at 5°C and 40°C respectively.

In particular, the TNF-alpha variant BCP1496 and adalimumab, both in 'B', showed a low % change in %AMI at week 9 of 25.83 and 23.53 respectively. The relatively higher % change in %AMI at week 9 for the TNF-alpha variant BCP1496 (CHO DG44) of 38.13 may be due to the relatively high initial %AMI of 75.03.

### Functional binding assay (ELISA)

The biological activity of adalimumab and the TNF-alpha variants in the two formulations was assessed by Biacore. Over the 9 week period of storage at 5°C and 40°C, the samples showed the same %binding within assay variability.

Hence, it can be concluded that formulation 'B' can serve as an alternative to formulation 'A' in a clinical setting without compromising the stability of the antibody at 50mg/mL dosage strength.

**Table 14: SEC-HPLC of adalimumab and TNF-alpha variants BPC1494 and 1496 in Formulations 'A' and 'B' at 5°C, 25°C and 40°C. TLMWF - Total Low Molecular Weight Fragment; Mono - Monomer; TA- Total Aggregate. N = 2**

| Condition °C | Initial | | | Week 1 | | | Week 2 | | | Week 4 | | | Week 9 | | | % Change at Week 9 | | |
|---|---|---|---|---|---|---|---|---|---|---|---|---|---|---|---|---|---|---|
| | TA | Mono | TLMWF | TA | Mono | TLMWF | TA | Mono | TLMWF | TA | Mono | TLMWF | TA | Mono | TLMWF | TA | Mono | TLMWF |
| adalimumab in formulation 'A' | | | | | | | | | | | | | | | | | | |
| 5°C | 0.30 | 99.62 | 0.08 | 0.30 | 99.54 | 0.16 | 0.32 | 99.53 | 0.15 | 0.29 | 99.51 | 0.21 | 0.34 | 99.49 | 0.17 | | | |
| 40°C | | | | 0.39 | 99.13 | 0.48 | 0.51 | 99.03 | 0.46 | 0.54 | 98.77 | 0.69 | 1.75 | 92.08 | 6.16 | 1.45 | 7.54 | 6.08 |
| adalimumab in formulation 'B' | | | | | | | | | | | | | | | | | | |
| 5°C | 0.42 | 99.45 | 0.13 | 0.34 | 99.49 | 0.17 | 0.38 | 99.45 | 0.16 | 0.35 | 99.42 | 0.23 | 0.38 | 99.44 | 0.18 | | | |
| 40°C | | | | 0.41 | 99.36 | 0.23 | 0.48 | 99.23 | 0.29 | 0.54 | 98.85 | 0.61 | 0.88 | 94.93 | 4.19 | 0.46 | 4.52 | 4.06 |
| TNF-alpha variant BPC1494 (CHO DG44) in formulation 'B' | | | | | | | | | | | | | | | | | | |
| 5°C | 2.76 | 97.13 | 0.11 | 2.65 | 97.25 | 0.09 | 3.29 | 96.45 | 0.25 | 2.66 | 97.18 | 0.16 | 2.83 | 97.05 | 0.12 | | | |
| 40°C | | | | 2.96 | 96.74 | 0.29 | 2.98 | 96.70 | 0.32 | 2.96 | 96.36 | 0.68 | 4.08 | 90.85 | 5.07 | 1.32 | 6.28 | 4.96 |
| TNF-alpha variant BPC1494 (CHOK1) in formulation 'B' | | | | | | | | | | | | | | | | | | |
| 5°C | 2.35 | 97.64 | 0.00 | 2.32 | 97.69 | 0.00 | 2.36 | 97.64 | 0.00 | 2.33 | 97.67 | 0.00 | 2.56 | 97.40 | 0.04 | | | |
| 40°C | | | | 2.67 | 97.09 | 0.24 | 2.79 | 96.99 | 0.22 | 3.17 | 96.20 | 0.63 | 4.91 | 90.22 | 4.88 | 2.56 | 7.42 | 4.88 |
| TNF-alpha variant BPC1496 in formulation 'B' | | | | | | | | | | | | | | | | | | |
| 5°C | 1.19 | 98.78 | 0.04 | 1.47 | 98.49 | 0.04 | 1.62 | 98.34 | 0.03 | 1.71 | 98.15 | 0.14 | 1.92 | 97.95 | 0.13 | | | |
| 40°C | | | | 1.75 | 97.99 | 0.26 | 1.46 | 98.27 | 0.27 | 1.40 | 98.05 | 0.54 | 2.64 | 93.50 | 3.86 | 1.45 | 5.28 | 3.82 |

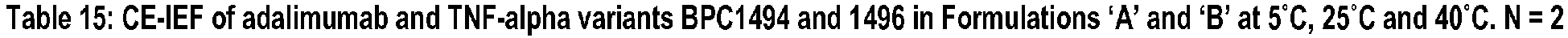

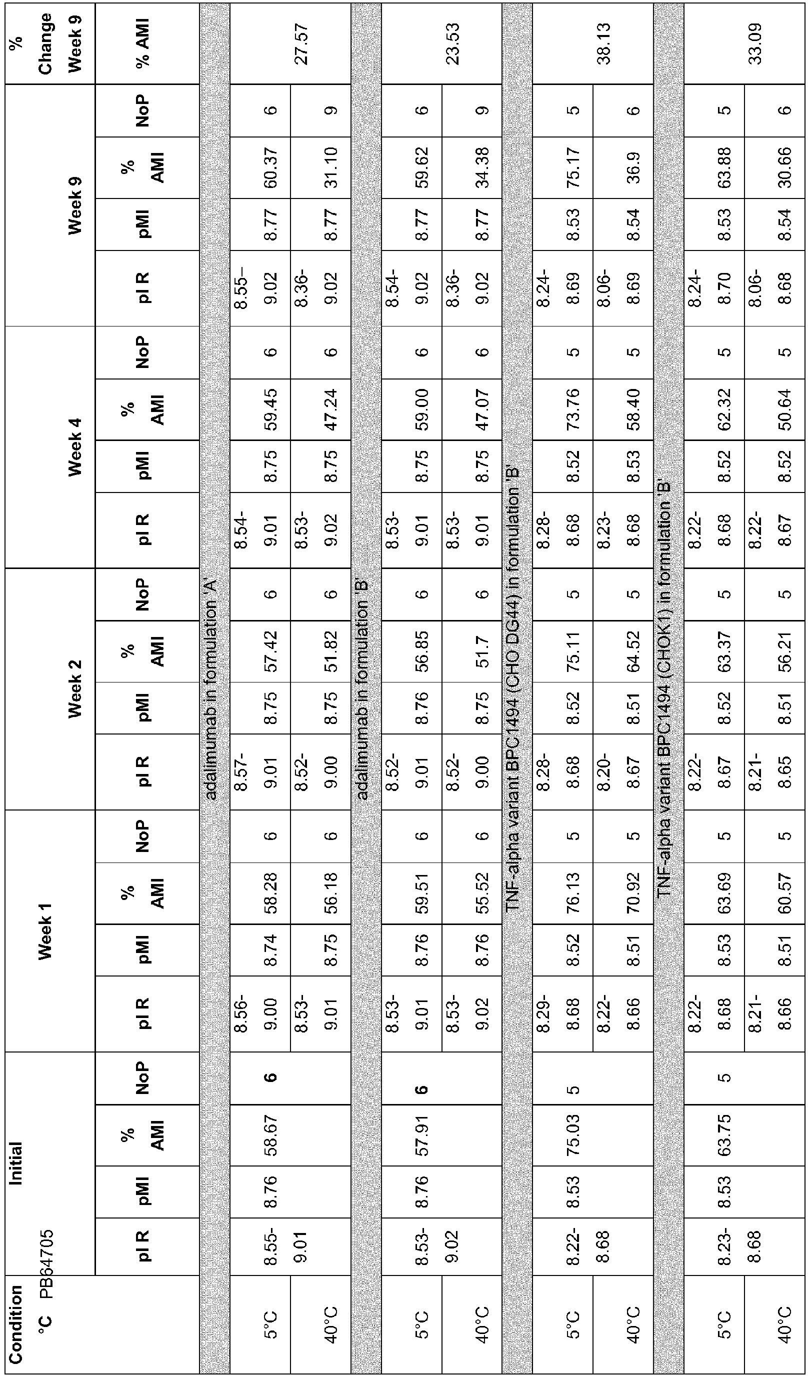

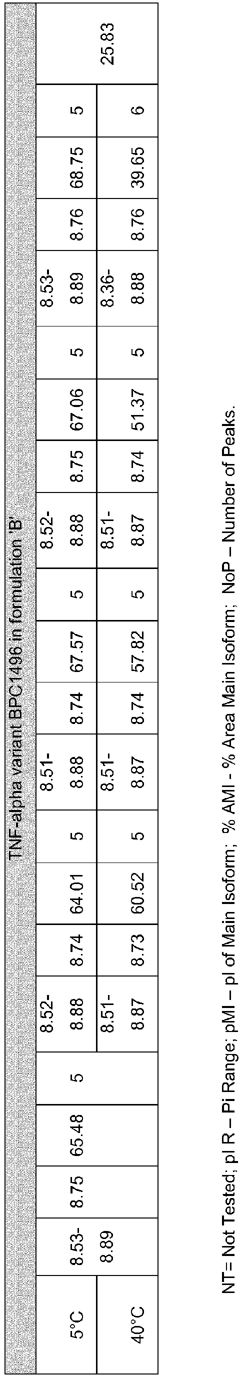

### Example 27: Plasma concentrations of BPC1494 following subcutaneous administration in the male cynomolgus monkey

In a repeat dose pharmacokinetic study BPC1494 was administered sub-cutaneously weekly or biweekly for 4 weeks at 30 or 100 mg/kg to male cynomolgus monkeys. For group 2 (n=3), the animals were administered 2x 30mg/kg doses on day 1 (approximately 1 hour apart) followed by a single 30 mg/kg dose on days 8, 15 and 22. For group 3 (n=3), the animals were administered with 2x 30mg/kg doses on day 1 (approximately 1 hour apart) followed by a single 30 mg/kg dose on day 15. For group 4 (n=3), the animals were administered with 2x 100mg/kg doses on day 1 (approximately 1 hour apart) followed by a single 100 mg/kg dose on day 15. Plasma samples were taken at intervals throughout the dosing and recovery phases of the study.

Plasma samples were analyzed for BPC1494 using a qualified analytical method based on sample dilution followed by immunoassay analysis Plasma samples were analyzed for BPC1494 or BPC1492. The method used 10 µg/ml biotinylated recombinant human TNF-alpha as the capture antigen and a 1:100 dilution of AlexaFluor 647-labelled anti-human IgG (Fc specific) antibody as the detection antibody (G18-145). The lower limit of quantification (LLQ) for BPC1494 was 1 µg/mL using a 50 µL aliquot of 100-fold diluted monkey plasma with a higher limit of quantification (HLQ) of 100 µg/mL. The computer systems that were used on this study to acquire and quantify data included Gyrolab Workstation Version 5.2.0, Gyrolab Companion version 1.0 and SMS2000 version 2.3. PK analysis was performed by non-compartmental pharmacokinetic analysis using WinNonlin Enterprise Pheonix version 6.1.

Pharmacokinetic data is presented in Table 16 with parameters determined from last dose received on Week 4 to the time point (t) 840 hours post dosing for 30 mg/kg/week dose group (2) and last dose received on Week 3 to the time point (t)1008 hours post dosing for 30 & 100 mg/kg/biweekly dose groups (3 and 4).

**Table 16: Individual and Mean Pharmacokinetic Parameters for BPC1494 in the Male Cynomolgus Monkey Following Subcutaneous Dosing of BPC1494 at 30 mg/kg/week or 30 and 100 mg/kg/biweekly over a 4-Week Investigative Study**

| Dose (mg/kg/biweekly) | Animal Number | Pharmacokinetic Parameters b | | | | | | |
|---|---|---|---|---|---|---|---|---|
| | | AUC0-t (mg.h/mL) | Cmax (mg/mL) | Median Tmax (h) | t½ (h) | MRT (h) | Estimated c CL_F (mL/h/kg) | Estimated c Vz_F (mL/kg) |
| 30a | P12M-272 | 923 | 1.51 | 168 | 616 | 367 | 0.125 | 111 |
| | P12M-273 | 758 | 1.29 | 168 | 604 | 368 | 0.141 | 123 |
| | P12M-274d | 21.3 | 0.135 | 24 | 226 | 142 | 3.01 | 978 |
| | Mean | 841 (568) | 1.40 (0.977) | 168 | 610 (482) | 367 (292) | 0.133 (1.09) | 117 (404) |
| | | | | | | | | |
| 30 | P12M-275 | 743 | 1.08 | 24 | 420 | 419 | 0.115 | 69.5 |
| | P12M-276 | 538 | 2.31 | 48 | 197 | 307 | 0.141 | 40.2 |
| | P12M-277d | 239 | 1.09 | 24 | 123 | 189 | 0.217 | 38.6 |
| | Mean | 641 (507) | 1.70 (1.49) | 36 (24) | 309 (247) | 363 (305) | 0.128 (0.158) | 54.9 (49.4) |
| | | | | | | | | |
| 100 | P12M-278 | 2760 | 5.89 | 24 | 398 | 374 | 0.0998 | 57.3 |
| | P12M-279 | 2480 | 5.21 | 72 | 332 | 362 | 0.131 | 62.9 |
| | P12M-280 | 2080 | 4.10 | 72 | 331 | 364 | 0.123 | 58.8 |
| | Mean | 2440 | 5.07 | 72 | 354 | 367 | 0.118 | 59.7 |

| | | | | | | | | |
|---|---|---|---|---|---|---|---|---|
| a) Group 2 animals received 30 mg/kg weekly for 4 weeks b) Pharmacokinetic parameters determined from last dose received on Week 4 to the time point (t) 840 hours post dosing for 30 mg/kg/week and last dose received on Week 3 to the time point (t)1008 hours post dosing for 30 & 100 mg/kg/biweekly c) Cl_F and Vz_F are estimates due to elimination phase following multiple doses and steady state not yet achieved. Parameter estimates have been calculated from i) using AUC0-168 or 336, ii) extrapolation of data from week 1 based on half-life and iii) using total dose over the defined sampling with AUC0-inf d) Animal 274 and 277 excluded from mean pharmacokinetic calculations based on scientific judgment that these animals are likely to be exhibiting an anti-drug antibody response. Mean data shown in parentheses are inclusive of these animals. | | | | | | | | |

### Example 28: SPR binding analysis of FcRn to Protein L captured anti-TNFα mAbs

The study was carried out using the ProteOnTM XPR36 (BioRadTM) biosensor machine, a surface plasmon based machine designed for label free kinetic/affinity measurements. Protein L was immobilised on a GLM chip (BioRad, Cat No: 176-5012) by primary amine coupling. This surface was then used to capture the humanised antibodies, human and cyno FcRn (both in-house materials) was then used as analytes at 2048nM, 512nM, 128nM, 32nM, and 8nM, an injection of buffer alone (i.e. 0nM) used to double reference the binding curves. Regeneration of the protein L surface was carried out using Glycine-HCl pH1.5. The assay was run at 25°C and run in HBS-EP pH7.4 and HBS-EP pH6.0 with human or cynomolgus FcRn diluted in appropriate buffer. Affinities were calculated using the Equilibrium model, inherent to the ProteOn analysis software, using a "Global R-max" for binding at pH6.0 and the R-max from binding at pH6.0 for affinity calculation at pH7.4. Since the binding curves did not reach saturation at pH7.4, the values obtained are unlikely to be true affinities however were used to rank the binding of the antibodies tested.

The binding affinity of different batches of BPC1492, BPC1494 and BPC1496 for human FcRn was compared using antibodies captures by Protein L. Table 17shows the results from a series of experiments using this format. The data confirms that BPC1494 and BPC1496 have an improved affinity for recombinant human FcRn compared to BPC1492 at both pH6.0 and pH7.4. The fold improvement in binding affinity of BPC1494 for FcRn compared to BPC1492 differs from experiment to experiment due to changes in the Protein L activity on the capture. However, in the experiments shown in Table 17, the fold improvement in binding affinity at pH6.0 ranges between 3.5-fold and 16.3-fold. It was not possible to determine the fold improvement in binding affinity at pH7.4 due to the weak binding activity of human IgG for FcRn at neutral pH.

The binding affinity of different batches of BPC1492, BPC1494 and BPC1496 for cynomolgus FcRn was also compared using antibodies captured with Protein L. Table 18 shows the results from the experiment using this format. The data confirms that BPC1494 has an improved affinity for recombinant cynomolgus FcRn compared to BPC1492 at both pH6.0 and pH7.4. The fold improvement in binding affinity of BPC1494 (range 41.8-46.8nM) for cynomolgus FcRn compared to BPC1492 (range 394-398nM) is approximately 9-fold at pH6. It was not possible to determine the fold improvement in binding affinity at pH7.4 due to the weak binding activity of BPC1492 for FcRn.

**Table 17 Recombinant human FcRn binding affinities using the Protein L capture method**

| | | Affinity KD (nM) | | | | | | | | |
|---|---|---|---|---|---|---|---|---|---|---|
| | | BPC1492 | | | BPC1494 | | | BPC1496 | | |
| | | Batch | | | Batch | | | Batch | | |
| Expt. | pH | HEK | HEK | CHO | HEK | HEK | GRITS | HEK | HEK | GRITS |
| | | 1406 | 1348 | clinical grade | 1407 | 1350 | 42954 | 1352 | 1408 | 42955 |
| 5 | 6 | 320. 0 | 325. 0 | 315.0 | 6.08* * | 24.9 | 26.2 | 14.3 | 16.9 | 15.4 |
| | 7.4 | NAB | NAB | NAB | 2020 ** | 1260 0 | 11700 | 8980 | 9830 | 9670 |
| 4 | 6 | 50.9 | 54.8 | 55.5 | 1.33 | 4.05 | 4.50 | 2.35 | 3.60 | 2.33 |
| | 7.4 | NAB | NAB | NAB | 303 | 5270 | 4740 | 6820 | 7550 | 7550 |
| 3 | 6 | 16.0 | 16.8 | 17.3 | 0.70 1 | 1.96 0 | 2.430 | 2.20 0 | 4.14 0 | 1.810 |
| | 7.4 | NAB | NAB | NAB | 1760 | 1050 0 | 10900 | 7830 | 8050 | 8460 |
| 2 | 6 | 13.1 | 12.9 | 13.9 | ## | 0.35 9 | 0.979 | 0.97 8 | 2.44 0 | 0.546 |
| | 7.4 | NAB | NAB | NAB | 2010 | 9190 | 9330 | 1090 0 | 9480 | 9550 |
| 1 | 6 | ND | 234 | ND | ND | 66 | ND | ND | 85 | ND |
| | 7.4 | ND | NAB | ND | ND | NAB | ND | ND | 2010 | ND |

| | | | | | | | | | | |
|---|---|---|---|---|---|---|---|---|---|---|
| ** - although data points have been reported, the values should be treated with caution because these data are not consistent with the data obtained for the other batches of the same molecule during this experiment NAB = no analysable binding ND = not tested in this experiment ## = high affinity binding - beyond the sensitivity of the machine | | | | | | | | | | |

**Table 18 Recombinant cynomolgus FcRn binding affinities using the Protein L capture method**

| Batch number | Construct | pH6 KD (nM) | pH7.4 KD (nM) |
|---|---|---|---|
| GRITS44463 | BPC1494 | 46.8 | 14800 |
| MCB16Marc2012 | BPC1494 | 41.8 | 13300 |
| GRITS42954 | BPC1494 | 43.2 | 13700 |
| Clinical grade | BPC1492 | 394 | No binding |
| GRITS44348 | BPC1492 | 398 | No binding |

**Table A**

| **Description** | **Sequence identifier (SEQ ID NO)** | |
|---|---|---|
| | **Polynucleotide** | **Amino acid** |
| Anti-TNF antibody light chain | 1 | 2 |
| Anti-TNF antibody variable domain (VL) | - | 3 |
| anti-TNF antibody heavy chain plus M252Y/S254T/T256E modification | 4 | 5 |
| Anti-TNF antibody heavy variable domain (VH) | - | 6 |
| IgG1 constant domain plus M252Y/S254T/T256E modification | - | 7 |
| Anti-TNF antibody heavy chain plus M428L/N434S modification | 8 | 9 |
| IgG1 constant domain plus M428L/N434S modification | - | 10 |
| Anti-TNF antibody heavy chain (wild-type IgG1) | 11 | 12 |
| IgG1 constant domain (wild-type) | - | 13 |
| Anti-TNF antibody heavy chain plus T250Q/M428L modification | 14 | 15 |
| IgG1 constant domain plus T250Q/M428L modification | - | 16 |
| Anti-TNF antibody heavy chain plus V308F modification | 17 | 18 |
| IgG1 constant domain plus V308F modification | - | 19 |
| Anti-TNF antibody heavy chain plus V259I modification | 20 | 21 |
| IgG1 constant domain plus V259I modification | - | 22 |
| Anti-TNF antibody heavy chain plus P257L and N434Y variant | 23 | 24 |
| IgG1 constant domain plus P257L and N434Y modification | - | 25 |
| Signal peptide sequence | - | 26 |
| Anti-TNF antibody CDRH1 | - | 27 |
| Anti-TNF antibody CDRH2 | - | 28 |
| Anti-TNF antibody CDRH3 | - | 29 |
| Anti-TNF antibody CDRL1 | - | 30 |
| Anti-TNF antibody CDRL2 | - | 31 |
| Anti-TNF antibody CDRL3 | - | 32 |
| Anti-TNF antibody CDRH1 variant | - | 33-38 |
| Cimzia (certolizumab) LC (VL + Ck | | 39 |
| Anti-TNF antibody CDRH3 variant | - | 40-49 |
| Anti-TNF antibody CDRL1 variant | - | 50-61 |
| Anti-TNF antibody CDRL2 variant | - | 62-72 |
| Anti-TNF antibody CDRL3 variant | - | 73-76 |
| cb1-3-VH | 77 | 78 |
| cb2-44-VH | 79 | 80 |
| cb1-39-VH | 81 | 82 |
| cb1-31-VH | 83 | 84 |
| cb2-11-VH | 85 | 86 |
| cb2-40-VH | 87 | 88 |
| cb2-35-VH | 89 | 90 |
| cb2-28-VH | 91 | 92 |
| cb2-38-VH | 93 | 94 |
| cb2-20-VH | 95 | 96 |
| cb1-8-VL | 97 | 98 |
| cb1-43-VL | 99 | 100 |
| cb1-45-VL | 101 | 102 |
| cb1-4-VL | 103 | 104 |
| cb1-41-VL | 105 | 106 |
| cb1-37-VL | 107 | 108 |
| cb1-39-VL | 109 | 110 |
| cb1-33-VL | 111 | 112 |
| cb1-35-VL | 113 | 114 |
| cb1-31-VL | 115 | 116 |
| cb1-29-VL | 117 | 118 |
| cb1-22-VL | 119 | 120 |
| cb1-23-VL | 121 | 122 |
| cb1-12-VL | 123 | 124 |
| cb1-10-VL | 125 | 126 |
| cb2-1-VL | 127 | 128 |
| cb2-11-VL | 129 | 130 |
| cb2-40-VL | 131 | 132 |
| cb2-35-VL | 133 | 134 |
| cb2-28-VL | 135 | 136 |
| cb2-20-VL | 137 | 138 |
| cb1-3-VL | 139 | 140 |
| cb2-6-VL | 141 | 142 |
| cb2-44-VL | 143 | 144 |
| Anti-TNF antibody heavy chain variant cb1-3-VH plus M252Y/S254T/T256E modification | - | 145 |
| Anti-TNF antibody heavy chain variant cb2-44-VH plus M252Y/S254T/T256E modification | - | 146 |
| Anti-TNF antibody light chain variant cb1-3-VL | 147 | 148 |
| Anti-TNF antibody light chain variant cb2-6-VL | 149 | 150 |
| Anti-TNF antibody light chain variant cb2-44-VL | 151 | 152 |
| Anti-TNF antibody heavy chain variant cb1-3-VH | 153 | 154 |
| Anti-TNF antibody heavy chain variant cb2-44-VH | 155 | 156 |
| Pascolizumab heavy chain containing the M252Y/S254T/T256E modifications | 157 | 158 |
| Pascolizumab light chain | 159 | 160 |
| Pascolizumab heavy chain | - | 161 |
| Alternative anti-TNF antibody heavy chain plus M428L/N434S modification | | 162 |
| Alternative IgG1 constant domain plus M428L/N434S modification | | 163 |
| Anti-TNF antibody heavy chain plus H433K/N434F modification | | 164 |
| IgG1 constant domain plus H433K/N434F modification | | 165 |
| Alternative anti-TNF antibody heavy chain plus H433K/N434F modification | | 166 |
| Alternative IgG1 constant domain plus H433K/N434F modification | | 167 |
| Alternative anti-TNF antibody heavy chain plus M428L/N434S modification | | 168 |
| Alternative IgG1/2 constant domain plus M428L/N434S modification | | 169 |
| Golimumab_VH | | 170 |
| Golimumab_VL | | 171 |
| Golimumab HC | | 172 |
| Golimumab LC | | 173 |
| Remicade VH | | 174 |
| Remicade VL | | 175 |
| Remicade HC | | 176 |
| Remicade LC | | 177 |
| Cimzia (certolizumab) VH | | 178 |
| Cimzia (certolizumab) VL | | 179 |
| Cimzia (certolizumab) HC (VH+CH1) | | 180 |

### Sequence listing

SEQ ID NO: 1 Polynucleotide sequence of the anti-TNF antibody light chain
SEQ ID NO: 2 Protein sequence of the anti-TNF antibody light chain
SEQ ID NO: 3 Protein sequence of the anti-TNF antibody variable domain (VL)
SEQ ID NO: 4 Polynucleotide sequence of the anti-TNF antibody heavy chain plus M252Y/S254T/T256E modification
SEQ ID NO: 5 Protein sequence of the anti-TNF antibody heavy chain plus M252Y/S254T/T256E modification
SEQ ID NO: 6 Protein sequence of the anti-TNF antibody heavy variable domain (VH)
SEQ ID NO: 7 Protein sequence of the IgG1 constant domain plus M252Y/S254T/T256E modification
SEQ ID NO: 8 Polynucleotide sequence of the anti-TNF antibody heavy chain plus M428L/N434S modification
SEQ ID NO: 9 Protein sequence of the anti-TNF antibody heavy chain plus M428L/N434S modification
SEQ ID NO: 10 Protein sequence of the IgG1 constant domain plus M428L/N434S modification
SEQ ID NO: 11 Polynucleotide sequence of the anti-TNF antibody heavy chain (wild-type IgG1)
SEQ ID NO: 12 Protein sequence of the anti-TNF antibody heavy chain (wild-type IgG1)
SEQ ID NO: 13 Protein sequence of the IgG1 constant domain (wild-type)
SEQ ID NO: 14 Polynucleotide sequence of the anti-TNF antibody heavy chain plus T250Q/M428L modification
SEQ ID NO: 15 Protein sequence of the anti-TNF antibody heavy chain plus T250Q/M428L modification
SEQ ID NO: 16 Protein sequence of the IgG1 constant domain plus T250Q/M428L modification
SEQ ID NO: 17 Polynucleotide sequence of the anti-TNF antibody heavy chain plus V308F modification
SEQ ID NO: 18 Protein sequence of the anti-TNF antibody heavy chain plus V308F modification
SEQ ID NO: 19 Protein sequence of the IgG1 constant domains plus V308F modification
SEQ ID NO: 20 Polynucleotide sequence of the anti-TNF antibody heavy chain plus V259I modification
SEQ ID NO: 21 Protein sequence of the anti-TNF antibody heavy chain plus V259I modification
SEQ ID NO: 22 Protein sequence of the IgG1 constant domains plus V259I modification
SEQ ID NO: 23 Polynucleotide sequence of the anti-TNF antibody heavy chain plus P257L and N434Y variant
SEQ ID NO: 24 Protein sequence of the anti-TNF antibody heavy chain plus P257L and N434Y modification
SEQ ID NO: 25 Protein sequence of the IgG1 constant domains plus P257L and N434Y modification
SEQ ID NO: 26 Signal peptide sequence MGWSCIILFLVATATGVHS
SEQ ID NO: 27 anti-TNF antibody CDRH1 DYAMH
SEQ ID NO: 28 anti-TNF antibody CDRH2 AITWNSGHIDYADSVEG
SEQ ID NO: 29 anti-TNF antibody CDRH3 VSYLSTASSLDY
SEQ ID NO: 30 anti-TNF antibody CDRL1 RASQGIRNYLA
SEQ ID NO: 31 anti-TNF antibody CDRL2 AASTLQS
SEQ ID NO: 32 anti-TNF antibody CDRL3 QRYNRAPYT
SEQ ID NO: 33 anti-TNF antibody CDRH1 variant QYAMH
SEQ ID NO: 34 anti-TNF antibody CDRH1 variant HYALH
SEQ ID NO: 35 anti-TNF antibody CDRH1 variant HYAMH
SEQ ID NO: 36 anti-TNF antibody CDRH1 variant QHALH
SEQ ID NO: 37 anti-TNF antibody CDRH1 variant QHAMH
SEQ ID NO: 38 anti-TNF antibody CDRH1 variant DHALH
SEQ ID NO: 39 Cimzia (certolizumab) LC (VL + Ck)
SEQ ID NO: 40 anti-TNF antibody CDRH3 variant VHYLSTASQLHH
SEQ ID NO: 41 anti-TNF antibody CDRH3 variant VQYLSTASSLQS
SEQ ID NO: 42 anti-TNF antibody CDRH3 variant VKYLSTASSLHY
SEQ ID NO: 43 anti-TNF antibody CDRH3 variant VKYLSTASNLES
SEQ ID NO: 44 anti-TNF antibody CDRH3 variant VHYLSTASSLDY
SEQ ID NO: 45 anti-TNF antibody CDRH3 variant VSYLSTASSLQS
SEQ ID NO: 46 anti-TNF antibody CDRH3 variant VRYLSTASNLQH
SEQ ID NO: 47 anti-TNF antibody CDRH3 variant VQYLSTASQLHS
SEQ ID NO: 48 anti-TNF antibody CDRH3 variant VRYLSTASQLDY
SEQ ID NO: 49 anti-TNF antibody CDRH3 variant VRYLSTASSLDY
SEQ ID NO: 50 anti-TNF antibody CDRL1 variant HASKKIRNYLA
SEQ ID NO: 51 anti-TNF antibody CDRL1 variant HASRKLRNYLA
SEQ ID NO: 52 anti-TNF antibody CDRL1 variant HASRRLRNYLA
SEQ ID NO: 53 anti-TNF antibody CDRL1 variant HASKRIRNYLA
SEQ ID NO: 54 anti-TNF antibody CDRL1 variant HASRKIRNYLA
SEQ ID NO: 55 anti-TNF antibody CDRL1 variant HASRRIRNYLA
SEQ ID NO: 56 anti-TNF antibody CDRL1 variant HASREIRNYLA
SEQ ID NO: 57 anti-TNF antibody CDRL1 variant HASQGIRNYLA
SEQ ID NO: 58 anti-TNF antibody CDRL1 variant HASQKIRNYLA
SEQ ID NO: 59 anti-TNF antibody CDRL1 variant RASRGLRNYLA
SEQ ID NO: 60 anti-TNF antibody CDRL1 variant HASQRIRNYLA
SEQ ID NO: 61 anti-TNF antibody CDRL1 variant RASRRIRNYLA
SEQ ID NO: 62 anti-TNF antibody CDRL2 variant AASSLLR
SEQ ID NO: 63 anti-TNF antibody CDRL2 variant AASSLLK
SEQ ID NO: 64 anti-TNF antibody CDRL2 variant AASSLLP
SEQ ID NO: 65 anti-TNF antibody CDRL2 variant AASSLQP
SEQ ID NO: 66 anti-TNF antibody CDRL2 variant AASSLLH
SEQ ID NO: 67 anti-TNF antibody CDRL2 variant AASSFLP
SEQ ID NO: 68 anti-TNF antibody CDRL2 variant AASSLLQ
SEQ ID NO: 69 anti-TNF antibody CDRL2 variant AASSLQQ
SEQ ID NO: 70 anti-TNF antibody CDRL2 variant AASTLLK
SEQ ID NO: 71 anti-TNF antibody CDRL2 variant AASSLQN
SEQ ID NO: 72 anti-TNF antibody CDRL2 variant AASSLQK
SEQ ID NO: 73 anti-TNF antibody CDRL3 variant QRYDRPPYT
SEQ ID NO: 74 anti-TNF antibody CDRL3 variant QRYDKPPYT
SEQ ID NO: 75 anti-TNF antibody CDRL3 variant QRYNRPPYT
SEQ ID NO: 76 anti-TNF antibody CDRL3 variant QRYNKPPYT
SEQ ID NO: 77 Polynucleotide sequence of anti-TNF antibody variable heavy domain variant cb1-3-VH (aka cb2-6-VH)
SEQ ID NO: 78 Protein sequence of anti-TNF antibody variable heavy domain variant cb1-3-VH (aka cb2-6-VH)
SEQ ID NO: 79 Polynucleotide sequence of anti-TNF antibody variable heavy domain variant cb2-44-VH
SEQ ID NO: 80 Protein sequence of anti-TNF antibody variable heavy domain variant cb2-44-VH
SEQ ID NO: 81 Polynucleotide sequence of anti-TNF antibody variable heavy domain variant cb1-39-VH
SEQ ID NO: 82 Protein sequence of anti-TNF antibody variable heavy domain variant cb1-39-VH
SEQ ID NO: 83 Polynucleotide sequence of anti-TNF antibody variable heavy domain variant cb1-31-VH
SEQ ID NO: 84 Protein sequence of anti-TNF antibody variable heavy domain variant cb1-31-VH
SEQ ID NO: 85 Polynucleotide sequence of anti-TNF antibody variable heavy domain variant cb2-11-VH
SEQ ID NO: 86 Protein sequence of anti-TNF antibody variable heavy domain variant cb2-11-VH
SEQ ID NO: 87 Polynucleotide sequence of anti-TNF antibody variable heavy domain variant cb2-40-VH
SEQ ID NO: 88 Protein sequence of anti-TNF antibody variable heavy domain variant cb2-40-VH
SEQ ID NO: 89 Polynucleotide sequence of anti-TNF antibody variable heavy domain variant cb2-35-VH
SEQ ID NO: 90 Protein sequence of anti-TNF antibody variable heavy domain variant cb2-35-VH
SEQ ID NO: 91 Polynucleotide sequence of anti-TNF antibody variable heavy domain variant cb2-28-VH
SEQ ID NO: 92 Protein sequence of anti-TNF antibody variable heavy domain variant cb2-28-VH
SEQ ID NO: 93 Polynucleotide sequence of anti-TNF antibody variable heavy domain variant cb2-38-VH
SEQ ID NO: 94 Protein sequence of anti-TNF antibody variable heavy domain variant cb2-38-VH
SEQ ID NO: 95 Polynucleotide sequence of anti-TNF antibody variable heavy domain variant cb2-20-VH
SEQ ID NO: 96 Protein sequence of anti-TNF antibody variable heavy domain variant cb2-20-VH
SEQ ID NO: 97 Polynucleotide sequence of anti-TNF antibody variable light domain variant cb1-8-VL
SEQ ID NO: 98 Protein sequence of anti-TNF antibody variable light domain variant cb1-8-VL
SEQ ID NO: 99 Polynucleotide sequence of anti-TNF antibody variable light domain variant cb1-43-VL
SEQ ID NO: 100 Protein sequence of anti-TNF antibody variable light domain variant cb1-43-VL
SEQ ID NO: 101 Polynucleotide sequence of anti-TNF antibody variable light domain variant cb1-45-VL
SEQ ID NO: 102 Protein sequence of anti-TNF antibody variable light domain variant cb1-45-VL
SEQ ID NO: 103 Polynucleotide sequence of anti-TNF antibody variable light domain variant cb1-4-VL
SEQ ID NO: 104 Protein sequence of anti-TNF antibody variable light domain variant cb1-4-VL
SEQ ID NO: 105 Polynucleotide sequence of anti-TNF antibody variable light domain variant cb1-41-VL
SEQ ID NO: 106 Protein sequence of anti-TNF antibody variable light domain variant cb1-41-VL
SEQ ID NO: 107 Polynucleotide sequence of anti-TNF antibody variable light domain variant cb1-37-VL
SEQ ID NO: 108 Protein sequence of anti-TNF antibody variable light domain variant cb1-37-VL
SEQ ID NO: 109 Polynucleotide sequence of anti-TNF antibody variable light domain variant cb1-39-VL
SEQ ID NO: 110 Protein sequence of anti-TNF antibody variable light domain variant cb1-39-VL
SEQ ID NO: 111 Polynucleotide sequence of anti-TNF antibody variable light domain variant cb1-33-VL
SEQ ID NO: 112 Protein sequence of anti-TNF antibody variable light domain variant cb1-33-VL
SEQ ID NO: 113 Polynucleotide sequence of anti-TNF antibody variable light domain variant cb1-35-VL
SEQ ID NO: 114 Protein sequence of anti-TNF antibody variable light domain variant cb1-35-VL
SEQ ID NO: 115 Polynucleotide sequence of anti-TNF antibody variable light domain variant cb1-31-VL
SEQ ID NO: 116 Protein sequence of anti-TNF antibody variable light domain variant cb1-31-VL
SEQ ID NO: 117 Polynucleotide sequence of anti-TNF antibody variable light domain variant cb1-29-VL
SEQ ID NO: 118 Protein sequence of anti-TNF antibody variable light domain variant cb1-29-VL
SEQ ID NO: 119 Polynucleotide sequence of anti-TNF antibody variable light domain variant cb1-22-VL
SEQ ID NO: 120 Protein sequence of anti-TNF antibody variable light domain variant cb1-22-VL
SEQ ID NO: 121 Polynucleotide sequence of anti-TNF antibody variable light domain variant cb1-23-VL
SEQ ID NO: 122 Protein sequence of anti-TNF antibody variable light domain variant cb1-23-VL
SEQ ID NO: 123 Polynucleotide sequence of anti-TNF antibody variable light domain variant cb1-12-VL
SEQ ID NO: 124 Protein sequence of anti-TNF antibody variable light domain variant cb1-12-VL
SEQ ID NO: 125 Polynucleotide sequence of anti-TNF antibody variable light domain variant cb1-10-VL
SEQ ID NO: 126 Protein sequence of anti-TNF antibody variable light domain variant cb1-10-VL
SEQ ID NO: 127 Polynucleotide sequence of anti-TNF antibody variable light domain variant cb2-1-VL
SEQ ID NO: 128 Protein sequence of anti-TNF antibody variable light domain variant cb2-1-VL
SEQ ID NO: 129 Polynucleotide sequence of anti-TNF antibody variable light domain variant cb2-11-VL
SEQ ID NO: 130 Protein sequence of anti-TNF antibody variable light domain variant cb2-11-VL
SEQ ID NO: 131 Polynucleotide sequence of anti-TNF antibody variable light domain variant cb2-40-VL
SEQ ID NO: 132 Protein sequence of anti-TNF antibody variable light domain variant cb2-40-VL
SEQ ID NO: 133 Polynucleotide sequence of anti-TNF antibody variable light domain variant cb2-35-VL
SEQ ID NO: 134 Protein sequence of anti-TNF antibody variable light domain variant cb2-35-VL
SEQ ID NO: 135 Polynucleotide sequence of anti-TNF antibody variable light domain variant cb2-28-VL
SEQ ID NO: 136 Protein sequence of anti-TNF antibody variable light domain variant cb2-28-VL
SEQ ID NO: 137 Polynucleotide sequence of anti-TNF antibody variable light domain variant cb2-20-VL
SEQ ID NO: 138 Protein sequence of anti-TNF antibody variable light domain variant cb2-20-VL
SEQ ID NO: 139 Polynucleotide sequence of anti-TNF antibody variable light domain variant cb1-3-VL
SEQ ID NO: 140 Protein sequence of anti-TNF antibody variable light domain variant cb1-3-VL
SEQ ID NO: 141 Polynucleotide sequence of anti-TNF antibody variable light domain variant cb2-6-VL
SEQ ID NO: 142 Protein sequence of anti-TNF antibody variable light domain variant cb2-6-VL
SEQ ID NO: 143 Polynucleotide sequence of anti-TNF antibody variable light domain variant cb2-44-VL
SEQ ID NO: 144 Protein sequence of anti-TNF antibody variable light domain variant cb2-44-VL
SEQ ID NO: 145 Protein sequence of anti-TNF antibody heavy chain variant cb1-3-VH plus M252Y/S254T/T256E modification
SEQ ID NO: 146 Protein sequence of anti-TNF antibody heavy chain variant cb2-44-VH plus M252Y/S254T/T256E modification
SEQ ID NO: 147 Polynucleotide sequence of anti-TNF antibody light chain variant cb1-3-VL
SEQ ID NO: 148 Protein sequence of anti-TNF antibody light chain variant cb1-3-VL
SEQ ID NO: 149 Polynucleotide sequence of anti-TNF antibody light chain variant cb2-6-VL
SEQ ID NO: 150 Protein sequence of anti-TNF antibody light chain variant cb2-6-VL
SEQ ID NO: 151 Polynucleotide sequence of anti-TNF antibody light chain variant cb2-44-VL
SEQ ID NO: 152 Protein sequence of anti-TNF antibody light chain variant cb2-44-VL
SEQ ID NO: 153 Polynucleotide sequence of anti-TNF antibody heavy chain variant cb1-3-VH
SEQ ID NO: 154 Protein sequence of anti-TNF antibody heavy chain variant cb1-3-VH
SEQ ID NO: 155 Polynucleotide sequence of anti-TNF antibody heavy chain variant cb2-44-VH
SEQ ID NO: 156 Protein sequence of anti-TNF antibody heavy chain variant cb2-44-VH
SEQ ID NO: 157 Polynucleotide sequence of pascolizumab heavy chain containing the M252Y/S254T/T256E modifications
SEQ ID NO: 158 Protein sequence of pascolizumab heavy chain containing the M252Y/S254T/T256E modifications
SEQ ID NO: 159 Polynucleotide sequence of pascolizumab light chain
SEQ ID NO: 160 Protein sequence of pascolizumab light chain
SEQ ID NO: 161 Protein sequence of pascolizumab heavy chain
SEQ ID NO: 162 Alternative protein sequence of the anti-TNF antibody heavy chain plus M428L/N434S modification
SEQ ID NO: 163 Alternative protein sequence of the IgG1 constant domain plus M428L/N434S modification
SEQ ID NO: 164 Protein sequence of the anti-TNF antibody heavy chain plus H433K/N434F modification
SEQ ID NO: 165 Protein sequence of the IgG1 constant domain plus H433K/N434F modification
SEQ ID NO: 166 Alternative protein sequence of the anti-TNF antibody heavy chain plus H433K/N434F modification
SEQ ID NO: 167 Alternative protein sequence of the IgG1 constant domain plus H433K/N434F modification
SEQ ID NO: 168 Alternative protein sequence of the anti-TNF antibody heavy chain plus M428L/N434S modification
SEQ ID NO: 169 Alternative protein sequence of the IgG1/2 constant domain plus M428L/N434S modification
SEQ ID NO: 170 Golimumab_VH
SEQ ID NO: 171 Golimumab_VL
SEQ ID NO: 172 Golimumab_HC
SEQ ID NO: 173 Golimumab_LC
SEQ ID NO: 174 Remicade_VH
SEQ ID NO: 175 Remicade_VL
SEQ ID NO: 176 Remicade_HC
SEQ ID NO: 177 Remicade_LC
SEQ ID NO: 178 Cimzia (certolizumab) VH
SEQ ID NO: 179 Cimzia (certolizumab) VL
SEQ ID NO: 180 Cimzia (certolizumab) HC (VH+CH1)

## Claims

1. An antigen binding protein which specifically binds to TNF-alpha comprising:
(i) CDRH1 (SEQ ID NO: 27), CDRH2 (SEQ ID NO: 28), CDRH3 (SEQ ID No: 29), CDRL1 (SEQ ID NO: 30), CDRL2 (SEQ ID NO: 31), and CDRL3 (SEQ ID NO: 32); and
(ii) a neonatal Fc receptor (FcRn) binding portion of a human IgG1 constant domain comprising amino acid substitutions relative to the human IgG1 constant domain at amino acid residues 252, 254 and 256 numbered according to EU index of Kabat and wherein the substitution at residue 252 is a substitution of met with tyr; the substitution at residue 254 is a substitution of ser with thr and the substitution at residue 256 is a substitution of thr with glu;
wherein the antigen binding protein has an increased FcRn binding affinity at pH 6 and increased half-life as compared to an IgG comprising the light chain sequence of SEQ ID No: 2 and the heavy chain sequence of SEQ ID No:12. for use in a method of treating a disease wherein the antigen binding protein is administered to patients at a single dose between 35 to 45 mg at a four to eight weekly interval.

2. An antigen binding protein for use as claimed in claim 1 wherein the human IgG1 constant domain has the sequence of SEQ ID No: 13 before amino acid substitutions are introduced.

3. An antigen binding protein for use as claimed in any preceding claim wherein the antigen binding protein is administered to patients subcutaneously as a single 40 mg dose no more than once every four weeks.

4. An antigen binding protein for use as claimed in any preceding claim wherein the antigen binding protein is administered to patients subcutaneously as a single 40 mg dose no more than once every eight weeks.

5. An antigen binding protein for use as claimed in any preceding claim wherein the half-life of the antigen binding protein is increased 2 fold, 3 fold, 4 fold or 5 fold as compared to the native IgG.

6. An antigen binding protein for use as claimed in any preceding claim wherein administration of the antigen binding protein no more than once every four weeks in patients achieves the mean steady-state trough concentration in the patient population of between 4 µg/ml to 7 µg/ml.

7. An antigen binding protein for use as claimed in claim 6 wherein the mean steady-state trough concentration is between 5 µg/ml to 6 µg/ml.

8. An antigen binding protein for use as claimed in any preceding claim wherein the clearance of the antigen binding protein is 2ml/ hr to 4ml/hr

9. An antigen binding protein for use as claimed in any preceding claim comprising a constant domain as shown in SEQ ID No: 7

10. An antigen binding protein for use as claimed in any preceding claim wherein the antigen binding protein is an antibody.

11. An antigen binding protein for use as claimed in any preceding claim wherein the antigen binding protein is to be administered with methotrexate, preferably wherein the antigen binding protein is administered for the treatment of rheumatoid arthritis.

12. An antigen binding protein for use as claimed in any preceding claim comprising the heavy chain sequence as shown in SEQ ID No: 5 optionally with a light chain sequence as shown in SEQ ID No: 2.

13. An antigen binding protein for use as claimed in any preceding claim wherein the disease is rheumatoid arthritis, polyarticular juvenile idiopathic arthritis, psoriatic arthritis, ankylosing spondylitis, Crohn's disease or Psoriasis.

## Patentansprüche

1. Antigen-bindendes Protein, das spezifisch an TNF-alpha bindet, umfassend (i) CDRH1 (SEQ ID NO:27), CDRH2 (SEQ ID NO:28), CDRH3 (SEQ ID NO:29), CDRL1 (SEQ ID NO:30), CDRL2 (SEQ ID NO:31) und CDRL3 (SEQ ID NO:32), und (ii) einen neonatalen Fc-Rezeptor (FcRn)-bindenden Teil einer konstanten Region eines humanen IgG1, der relativ zur konstanten Region des humanen IgG1 Aminosäure-Substitutionen an den Aminosäure-Resten 252, 254 und 256 aufweist, wobei die Nummerierung nach dem EU-Index von Kabat erfolgt, und wobei die Substitution an Rest 252 eine Substitution von Met durch Tyr ist; die Substitution an Rest 254 eine Substitution von Ser durch Thr ist; und die Substitution an Rest 256 eine Substitution von Thr durch Glu ist; wobei das Antigen-bindende Protein eine erhöhte FcRn-Bindungsaffinität bei pH 6 aufweist und eine erhöhte Halbwertszeit im Vergleich zu einem IgG, der die Leichte-Kette-Sequenz von SEQ ID NO:2 umfasst und die Schwere-Kette-Sequenz von SEQ ID NO:12, zur Verwendung in einem Verfahren zur Behandlung einer Erkrankung, wobei das Antigen-bindende Protein an Patienten in einer einzelnen Dosis von 35 bis 45 mg in einem 4 bis 8 wöchigen Intervall verabreicht wird.

2. Antigen-bindendes Protein zur Verwendung nach Anspruch 1, wobei die konstante Domäne des humanen IgG1 die Sequenz von SEQ ID NO:13 aufweist, bevor Aminosäure-Substitutionen eingebracht werden.

3. Antigen-bindendes Protein zur Verwendung nach einem der vorhergehenden Ansprüche, wobei das Antigen-bindende Protein subkutan als einzelne Dosis von 40 mg nicht mehr als einmal alle 4 Wochen an Patienten verabreicht wird.

4. Antigen-bindendes Protein zur Verwendung nach einem der vorhergehenden Ansprüche, wobei das Antigen-bindende Protein subkutan als einzelne Dosis von 40 mg nicht mehr als einmal alle 8 Wochen an Patienten verabreicht wird.

5. Antigen-bindendes Protein zur Verwendung nach einem der vorhergehenden Ansprüche, wobei die Halbwertszeit des Antigen-bindenden Proteins 2-fach, 3-fach, 4-fach oder 5-fach im Vergleich zum nativen IgG erhöht ist.

6. Antigen-bindendes Protein zur Verwendung nach einem der vorhergehenden Ansprüche, wobei die Verabreichung des Antigen-bindenden Proteins, welche nicht mehr als einmal alle 4 Wochen erfolgt, in Patienten die durchschnittliche minimale Steady-State-Konzentration (mean steady state trough concetration) von zwischen 4 µg/ml bis 7 µg/ml in der Patientenpopulation erreicht.

7. Antigen-bindendes Protein zur Verwendung nach Anspruch 6, wobei die durchschnittliche minimale Steady-State-Konzentration (mean steady state trough concetration) zwischen 5 µg/ml bis 6 µg/ml liegt.

8. Antigen-bindendes Protein zur Verwendung nach einem der vorhergehenden Ansprüche, wobei die Beseitigung des Antigen-bindenden Proteins von 2 ml pro Stunde bis 4 ml pro Stunde beträgt.

9. Antigen-bindendes Protein zur Verwendung nach einem der vorhergehenden Ansprüche, das eine konstante Domäne wie sie in SEQ ID NO:7 gezeigt ist, umfasst.

10. Antigen-bindendes Protein zur Verwendung nach einem der vorhergehenden Ansprüche, wobei das Antigen-bindende Protein ein Antikörper ist.

11. Antigen-bindendes Protein zur Verwendung nach einem der vorhergehenden Ansprüche, wobei das Antigen-bindende Protein gemeinsam mit Methotextrat zu verabreichen ist, wobei vorzugsweise das Antigen-bindende Protein für die Behandlung von rheumatoider Arthritis verabreicht wird.

12. Antigen-bindendes Protein zur Verwendung nach einem der vorhergehenden Ansprüche, das eine wie in SEQ ID NO:5 gezeigte Schwere-Kette-Sequenz umfasst, wahlweise mit einer wie in SEQ ID NO:2 gezeigten Leichte-Kette-Sequenz.

13. Antigen-bindendes Protein zur Verwendung nach einem der vorhergehenden Ansprüche, wobei die Krankheit rheumatoide Arthritis, polyartikuläre juvenile idiopathische Arthritis, Psoriasis-Arthritis, Spondylitis ankylosans, Morbus Crohn oder Psoriasis ist.

## Revendications

1. Protéine de liaison à un antigène qui se lie spécifiquement au TNF-alpha, comprenant :
(i) un CDRH1 (SEQ ID NO : 27), un CDRH2 (SEQ ID NO : 28), un CDRH3 (SEQ ID NO : 29), un CDRL1 (SEQ ID NO : 30), un CDRL2 (SEQ ID NO : 31) et un CDRL3 (SEQ ID NO : 32) ; et
(ii) une partie d'un domaine constant d'IgG1 humaine se liant au récepteur Fc néonatal (FcRn), comprenant des substitutions d'acide aminé par rapport au domaine constant d'IgG1 humaine au niveau des résidus d'acide aminé 252, 254 et 256 numérotés selon l'index EU de Kabat et dans laquelle la substitution au niveau du résidu 252 est une substitution de met par tyr ; la substitution au niveau du résidu 254 est une substitution de ser par thr et la substitution au niveau du résidu 256 est une substitution de thr par glu ;
dans laquelle la protéine de liaison à un antigène présente une affinité de liaison accrue pour FcRn à pH 6 et une demi-vie plus longue par rapport à une IgG comprenant la séquence de chaîne légère de SEQ ID No : 2 et la séquence de chaîne lourde de SEQ ID No : 12, pour son utilisation dans un procédé de traitement d'une maladie dans laquelle la protéine de liaison à un antigène est administrée à des patients à une dose unique entre 35 et 45 mg à un intervalle de quatre à huit semaines.

2. Protéine de liaison à un antigène pour son utilisation selon la revendication 1, dans laquelle le domaine constant d'IgG1 humaine possède la séquence de SEQ ID No : 13 avant l'introduction des substitutions d'acide aminé.

3. Protéine de liaison à un antigène pour son utilisation selon l'une quelconque des revendications précédentes, dans laquelle la protéine de liaison à un antigène est administrée aux patients par voie sous-cutanée sous la forme d'une dose unique de 40 mg au plus une fois toutes les quatre semaines.

4. Protéine de liaison à un antigène pour son utilisation selon l'une quelconque des revendications précédentes, dans laquelle la protéine de liaison à un antigène est administrée aux patients par voie sous-cutanée sous la forme d'une dose unique de 40 mg au plus une fois toutes les huit semaines.

5. Protéine de liaison à un antigène pour son utilisation selon l'une quelconque des revendications précédentes, dans laquelle la demi-vie de la protéine de liaison à un antigène est augmentée de 2 fois, de 3 fois, de 4 fois ou de 5 fois par rapport à celle de l'IgG native.

6. Protéine de liaison à un antigène pour son utilisation selon l'une quelconque des revendications précédentes, dans laquelle l'administration de la protéine de liaison à un antigène au plus une fois toutes les quatre semaines chez les patients atteint la concentration minimale moyenne à l'état d'équilibre dans la population de patients entre 4 µg/ml et 7 µg/ml.

7. Protéine de liaison à un antigène pour son utilisation selon la revendication 6, dans laquelle la concentration minimale moyenne à l'état d'équilibre est située entre 5 µg/ml et 6 µg/ml.

8. Protéine de liaison à un antigène pour son utilisation selon l'une quelconque des revendications précédentes, dans laquelle la clairance de la protéine de liaison à un antigène est de 2 ml/heure à 4 ml/heure.

9. Protéine de liaison à un antigène pour son utilisation selon l'une quelconque des revendications précédentes, comprenant un domaine constant tel que représenté par SEQ ID No : 7.

10. Protéine de liaison à un antigène pour son utilisation selon l'une quelconque des revendications précédentes, dans laquelle la protéine de liaison à un antigène est un anticorps.

11. Protéine de liaison à un antigène pour son utilisation selon l'une quelconque des revendications précédentes, dans laquelle la protéine de liaison à un antigène doit être administrée avec du méthotrexate, de préférence dans laquelle la protéine de liaison à un antigène est administrée pour le traitement de la polyarthrite rhumatoïde.

12. Protéine de liaison à un antigène pour son utilisation selon l'une quelconque des revendications précédentes, comprenant la séquence de chaîne lourde représentée par SEQ ID No : 5 facultativement avec une séquence de chaîne légère représentée par SEQ ID No : 2.

13. Protéine de liaison à un antigène pour son utilisation selon l'une quelconque des revendications précédentes, dans laquelle la maladie est la polyarthrite rhumatoïde, la polyarthrite idiopathique juvénile, le rhumatisme psoriasique, la spondylarthrite ankylosante, la maladie de Crohn ou le psoriasis.


### SEQUENCE MISTING

<110> ELLIS, johnathan TOMLINSON, Ian SHAH, Tejash MOLLOY, Micheal YASIN, Ahmed
<120> Antigen-binding proteins with increased
   FcRn binding
<130> PB64705
<150> GB1112429.4
   <151> 2011-07-19
<160> 180
<170> FastSEQ for Windows Version 4.0
<210> 1
   <211> 642
   <212> DNA
   <213> Artificial Sequence
<220>
   <223> Humanised sequence
<400> 1
   gatatccaga tgacccagag ccccagcagc ctgagcgcct ctgtgggcga tagagtgacc 60
   atcacctgcc gggccagcca gggcatcaga aactacctgg cctggtatca gcagaagcct 120
   ggcaaggccc ctaagctgct gatctacgcc gccagcaccc tgcagagcgg cgtgcccagc 180
   agattcagcg gcagcggctc cggcaccgac ttcaccctga ccatcagcag cctgcagccc 240
   gaggacgtgg ccacctacta ctgccagcgg tacaacagag ccccttacac cttcggccag 300
   ggcaccaagg tggagatcaa gcgtacggtg gccgccccca gcgtgttcat cttccccccc 360
   agcgatgagc agctcaagag cggcaccgcc agcgtggtgt gtctgctgaa caacttctac 420
   ccccgggagg ccaaagtgca gtggaaagtg gacaacgccc tgcagagcgg caacagccag 480
   gagagcgtga ccgagcagga cagcaaggac tccacctaca gcctgagcag caccctgacc 540
   ctgagcaagg ccgactacga gaagcacaaa gtgtacgcct gcgaagtgac ccaccagggc 600
   ctgtccagcc ccgtgaccaa gagcttcaac cggggcgagt gc                    642
<210> 2
   <211> 214
   <212> PRT
   <213> Artificial Sequence
<220>
   <223> Humanised sequence
<400> 2
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Arg Ala Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 3
   <211> 109
   <212> PRT
   <213> Artificial Sequence
<220>
   <223> Humanised sequence
<400> 3
   Asp Ile Gln Met Thr Gln Ser Pro Ser Ser Leu Ser Ala Ser Val Gly
    1               5                  10                  15
   Asp Arg Val Thr Ile Thr Cys Arg Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Arg Ala Pro Tyr
                   85                  90                  95
   Thr Phe Gly Gln Gly Thr Lys Val Glu Ile Lys Arg Thr
               100                 105
<210> 4
   <211> 1353
   <212> DNA
   <213> Artificial Sequence
<220>
   <223> Humanised sequence
<400> 4
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgtacat caccagagag 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag                             1353
<210> 5
   <211> 451
   <212> PRT
   <213> Artificial Sequence
<220>
   <223> Humanised sequence
<400> 5
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Tyr
                   245                 250                 255
   Ile Thr Arg Glu Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 6
   <211> 121
   <212> PRT
   <213> Artificial Sequence
<220>
   <223> Humanised sequence
<400> 6
<210> 7
   <211> 330
   <212> PRT
   <213> Artificial Sequence
<220>
   <223> Humanised sequence
<400> 7
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Tyr Ile Thr Arg Glu Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 8
   <211> 1353
   <212> DNA
   <213> Artificial Sequence
<220>
   <223> Humanised sequence
<400> 8
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgctgcacg aggccctgca cagccactac 1320
   acccagaaga gcctgagcct gtcccctggc aag                              1353
<210> 9
   <211> 451
   <212> PRT
   <213> Artificial Sequence
<220>
   <223> Humanised sequence
<400> 9
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Leu
               420                 425                 430
   His Glu Ala Leu His Ser His Tyr Thr Gln Lys Ser Leu Ser Leu Ser
           435                 440                 445
   Pro Gly Lys
       450
<210> 10
   <211> 330
   <212> PRT
   <213> Artificial Sequence
<220>
   <223> Humanised sequence
<400> 10
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Leu His Glu Ala Leu His Ser His Tyr Thr
   305                 310                 315                 320
   Gln Lys Ser Leu Ser Leu Ser Pro Gly Lys
                   325                 330
<210> 11
   <211> 1353
   <212> DNA
   <213> Artificial Sequence
<220>
   <223> Humanised sequence
<400> 11
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag                              1353
<210> 12
   <211> 451
   <212> PRT
   <213> Artificial Sequence
<220>
   <223> Humanised sequence
<400> 12
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 13
   <211> 330
   <212> PRT
   <213> Artificial Sequence
<220>
   <223> Humanised sequence
<400> 13
<210> 14
   <211> 1353
   <212> DNA
   <213> Artificial Sequence
<220>
   <223> Humanised sequence
<400> 14
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggacc aactgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgttgcacg aggccctgca caatcactac 1320
   acccagaaga gcctgagcct gtcccctggc aag                              1353
<210> 15
   <211> 451
   <212> PRT
   <213> Artificial Sequence
<220>
   <223> Humanised sequence
<400> 15
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Gln Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Leu
               420                 425                 430
   His Glu Ala Leu His Asn His Tyr Thr Gln Lys Ser Leu Ser Leu Ser
           435                 440                 445
   Pro Gly Lys
       450
<210> 16
   <211> 330
   <212> PRT
   <213> Artificial Sequence
<220>
   <223> Humanised sequence
<400> 16
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Gln Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Leu His Glu Ala Leu His Asn His Tyr Thr
   305                 310                 315                 320
   Gln Lys Ser Leu Ser Leu Ser Pro Gly Lys
                   325                 330
<210> 17
   <211> 1353
   <212> DNA
   <213> Artificial Sequence
<220>
   <223> Humanised sequence
<400> 17
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accttcctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag                              1353
<210> 18
   <211> 451
   <212> PRT
   <213> Artificial Sequence
<220>
   <223> Humanised sequence
<400> 18
   Glu Val Gln Leu Val Glu Ser Gly Gly Gly Leu Val Gln Pro Gly Arg
   1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Phe Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 19
   <211> 330
   <212> PRT
   <213> Artificial Sequence
<220>
   <223> Humanised sequence
<400> 19
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Phe Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 20
   <211> 1353
   <212> DNA
   <213> Artificial Sequence
<220>
   <223> Humanised sequence
<400> 20
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgagatca cctgtgtggt ggtggatgtg agccacgagg accctgaagt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag                              1353
<210> 21
   <211> 451
   <212> PRT
   <213> Artificial Sequence
<220>
   <223> Humanised sequence
<400> 21
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Ile Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 22
   <211> 330
   <212> PRT
   <213> Artificial Sequence
<220>
   <223> Humanised sequence
<400> 22
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Ile Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Asn His Tyr Thr
   305                 310                 315                 320
   Gln Lys Ser Leu Ser Leu Ser Pro Gly Lys
                   325                 330
<210> 23
   <211> 1353
   <212> DNA
   <213> Artificial Sequence
<220>
   <223> Humanised sequence
<400> 23
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   ctggaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca ctatcactac 1320
   acccagaaga gcctgagcct gtcccctggc aag                                1353
<210> 24
   <211> 451
   <212> PRT
   <213> Artificial Sequence
<220>
   <223> Humanised sequence
<400> 24
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Leu Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Tyr His Tyr Thr Gln Lys Ser Leu Ser Leu Ser
           435                 440                 445
   Pro Gly Lys
       450
<210> 25
   <211> 330
   <212> PRT
   <213> Artificial Sequence
<220>
   <223> Humanised sequence
<400> 25
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Leu Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu His Tyr His Tyr Thr
   305                 310                 315                 320
   Gln Lys Ser Leu Ser Leu Ser Pro Gly Lys
                   325                 330
<210> 26
   <211> 19
   <212> PRT
   <213> Artificial Sequence
<220>
   <223> Humanised sequence
<400> 26
   Met Gly Trp Ser Cys Ile Ile Leu Phe Leu Val Ala Thr Ala Thr Gly
   1 5 10 15
   Val His Ser
<210> 27
   <211> 5
   <212> PRT
   <213> Artificial Sequence
<220>
   <223> Humanised sequence
<400> 27
   Asp Tyr Ala Met His
   1 5
<210> 28
   <211> 17
   <212> PRT
   <213> Artificial Sequence
<220>
   <223> Humanised sequence
<400> 28
   Ala Ile Thr Trp Asn Ser Gly His Ile Asp Tyr Ala Asp Ser Val Glu
   1 5 10 15
   Gly
<210> 29
   <211> 12
   <212> PRT
   <213> Artificial Sequence
<220>
   <223> Humanised sequence
<400> 29
   Val Ser Tyr Leu Ser Thr Ala Ser Ser Leu Asp Tyr
   1 5 10
<210> 30
   <211> 11
   <212> PRT
   <213> Artificial Sequence
<220>
   <223> Humanised sequence
<400> 30
   Arg Ala Ser Gln Gly Ile Arg Asn Tyr Leu Ala
   1 5 10
<210> 31
   <211> 7
   <212> PRT
   <213> Artificial Sequence
<220>
   <223> Humanised sequence
<400> 31
   Ala Ala Ser Thr Leu Gln Ser
   1 5
<210> 32
   <211> 9
   <212> PRT
   <213> Artificial Sequence
<220>
   <223> Humanised sequence
<400> 32
   Gln Arg Tyr Asn Arg Ala Pro Tyr Thr
   1 5
<210> 33
   <211> 5
   <212> PRT
   <213> Artificial Sequence
<220>
   <223> Humanised sequence
<400> 33
   Gln Tyr Ala Met His
   1 5
<210> 34
   <211> 5
   <212> PRT
   <213> Artificial Sequence
<220>
   <223> Humanised sequence
<400> 34
   His Tyr Ala Leu His
   1 5
<210> 35
   <211> 5
   <212> PRT
   <213> Artificial Sequence
<220>
   <223> Humanised sequence
<400> 35
   His Tyr Ala Met His
   1 5
<210> 36
   <211> 5
   <212> PRT
   <213> Artificial Sequence
<220>
   <223> Humanised sequence
<400> 36
   Gln His Ala Leu His
   1 5
<210> 37
   <211> 5
   <212> PRT
   <213> Artificial Sequence
<220>
   <223> Humanised sequence
<400> 37
   Gln His Ala Met His
   1 5
<210> 38
   <211> 5
   <212> PRT
   <213> Artificial Sequence
<220>
   <223> Humanised sequence
<400> 38
   Asp His Ala Leu His
   1 5
<210> 39
   <211> 214
   <212> PRT
   <213> Artificial Sequence
<220>
   <223> Humanised sequence
<400> 39
   Asp Ile Gln Met Thr Gln Ser Pro Ser Ser Leu Ser Ala Ser Val Gly
    1               5                  10                  15
   Asp Arg Val Thr Ile Thr Cys Lys Ala Ser Gln Asn Val Gly Thr Asn
               20                  25                  30
   Val Ala Trp Tyr Gln Gln Lys Pro Gly Lys Ala Pro Lys Ala Leu Ile
           35                  40                  45
   Tyr Ser Ala Ser Phe Leu Tyr Ser Gly Val Pro Tyr Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ile Tyr Pro Leu
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 40
   <211> 12
   <212> PRT
   <213> Artificial Sequence
<220>
   <223> Humanised sequence
<400> 40
   Val His Tyr Leu Ser Thr Ala Ser Gln Leu His His
    1               5                  10
<210> 41
   <211> 12
   <212> PRT
   <213> Artificial Sequence
<220>
   <223> Humanised sequence
<400> 41
   Val Gln Tyr Leu Ser Thr Ala Ser Ser Leu Gln Ser
    1               5                  10
<210> 42
   <211> 12
   <212> PRT
   <213> Artificial Sequence
<220>
   <223> Humanised sequence
<400> 42
   Val Lys Tyr Leu Ser Thr Ala Ser Ser Leu His Tyr
   1 5 10
<210> 43
   <211> 12
   <212> PRT
   <213> Artificial Sequence
<220>
   <223> Humanised sequence
<400> 43
   Val Lys Tyr Leu Ser Thr Ala Ser Asn Leu Glu Ser
   1 5 10
<210> 44
   <211> 12
   <212> PRT
   <213> Artificial Sequence
<220>
   <223> Humanised sequence
<400> 44
   Val His Tyr Leu Ser Thr Ala Ser Ser Leu Asp Tyr
   1 5 10
<210> 45
   <211> 12
   <212> PRT
   <213> Artificial Sequence
<220>
   <223> Humanised sequence
<400> 45
   Val Ser Tyr Leu Ser Thr Ala Ser Ser Leu Gln Ser
   1 5 10
<210> 46
   <211> 12
   <212> PRT
   <213> Artificial Sequence
<220>
   <223> Humanised sequence
<400> 46
   Val Arg Tyr Leu Ser Thr Ala Ser Asn Leu Gln His
   1 5 10
<210> 47
   <211> 12
   <212> PRT
   <213> Artificial Sequence
<220>
   <223> Humanised sequence
<400> 47
   Val Gln Tyr Leu Ser Thr Ala Ser Gln Leu His Ser
   1 5 10
<210> 48
   <211> 12
   <212> PRT
   <213> Artificial Sequence
<220>
   <223> Humanised sequence
<400> 48
   Val Arg Tyr Leu Ser Thr Ala Ser Gln Leu Asp Tyr
   1 5 10
<210> 49
   <211> 12
   <212> PRT
   <213> Artificial Sequence
<220>
   <223> Humanised sequence
<400> 49
   Val Arg Tyr Leu Ser Thr Ala Ser Ser Leu Asp Tyr
   1 5 10
<210> 50
   <211> 11
   <212> PRT
   <213> Artificial Sequence
<220>
   <223> Humanised sequence
<400> 50
   His Ala Ser Lys Lys Ile Arg Asn Tyr Leu Ala
   1 5 10
<210> 51
   <211> 11
   <212> PRT
   <213> Artificial Sequence
<220>
   <223> Humanised sequence
<400> 51
   His Ala Ser Arg Lys Leu Arg Asn Tyr Leu Ala
   1               5                   10
<210> 52
   <211> 11
   <212> PRT
   <213> Artificial Sequence
<220>
   <223> Humanised sequence
<400> 52
   His Ala Ser Arg Arg Leu Arg Asn Tyr Leu Ala
   1               5                   10
<210> 53
   <211> 11
   <212> PRT
   <213> Artificial Sequence
<220>
   <223> Humanised sequence
<400> 53
   His Ala Ser Lys Arg Ile Arg Asn Tyr Leu Ala
   1               5                   10
<210> 54
   <211> 11
   <212> PRT
   <213> Artificial Sequence
<220>
   <223> Humanised sequence
<400> 54
   His Ala Ser Arg Lys Ile Arg Asn Tyr Leu Ala
   1               5                   10
<210> 55
   <211> 11
   <212> PRT
   <213> Artificial Sequence
<220>
   <223> Humanised sequence
<400> 55
   His Ala Ser Arg Arg Ile Arg Asn Tyr Leu Ala
   1 5 10
<210> 56
   <211> 11
   <212> PRT
   <213> Artificial Sequence
<220>
   <223> Humanised sequence
<400> 56
   His Ala Ser Arg Glu Ile Arg Asn Tyr Leu Ala
   1 5 10
<210> 57
   <211> 11
   <212> PRT
   <213> Artificial Sequence
<220>
   <223> Humanised sequence
<400> 57
   His Ala Ser Gln Gly Ile Arg Asn Tyr Leu Ala
   1 5 10
<210> 58
   <211> 11
   <212> PRT
   <213> Artificial Sequence
<220>
   <223> Humanised sequence
<400> 58
   His Ala Ser Gln Lys Ile Arg Asn Tyr Leu Ala
   1 5 10
<210> 59
   <211> 11
   <212> PRT
   <213> Artificial Sequence
<220>
   <223> Humanised sequence
<400> 59
   Arg Ala Ser Arg Gly Leu Arg Asn Tyr Leu Ala
   1 5 10
<210> 60
   <211> 11
   <212> PRT
   <213> Artificial Sequence
<220>
   <223> Humanised sequence
<400> 60
   His Ala Ser Gln Arg Ile Arg Asn Tyr Leu Ala
   1 5 10
<210> 61
   <211> 11
   <212> PRT
   <213> Artificial Sequence
<220>
   <223> Humanised sequence
<400> 61
   Arg Ala Ser Arg Arg Ile Arg Asn Tyr Leu Ala
   1 5 10
<210> 62
   <211> 7
   <212> PRT
   <213> Artificial Sequence
<220>
   <223> Humanised sequence
<400> 62
   Ala Ala Ser Ser Leu Leu Arg
   1 5
<210> 63
   <211> 7
   <212> PRT
   <213> Artificial Sequence
<220>
   <223> Humanised sequence
<400> 63
   Ala Ala Ser Ser Leu Leu Lys
   1 5
<210> 64
   <211> 7
   <212> PRT
   <213> Artificial Sequence
<220>
   <223> Humanised sequence
<400> 64
   Ala Ala Ser Ser Leu Leu Pro
   1 5
<210> 65
   <211> 7
   <212> PRT
   <213> Artificial Sequence
<220>
   <223> Humanised sequence
<400> 65
   Ala Ala Ser Ser Leu Gln Pro
   1 5
<210> 66
   <211> 7
   <212> PRT
   <213> Artificial Sequence
<220>
   <223> Humanised sequence
<400> 66
   Ala Ala Ser Ser Leu Leu His
   1 5
<210> 67
   <211> 7
   <212> PRT
   <213> Artificial Sequence
<220>
   <223> Humanised sequence
<400> 67
   Ala Ala Ser Ser Phe Leu Pro
   1 5
<210> 68
   <211> 7
   <212> PRT
   <213> Artificial Sequence
<220>
   <223> Humanised sequence
<400> 68
   Ala Ala Ser Ser Leu Leu Gln
   1 5
<210> 69
   <211> 7
   <212> PRT
   <213> Artificial Sequence
<220>
   <223> Humanised sequence
<400> 69
   Ala Ala Ser Ser Leu Gln Gln
   1 5
<210> 70
   <211> 7
   <212> PRT
   <213> Artificial Sequence
<220>
   <223> Humanised sequence
<400> 70
   Ala Ala Ser Thr Leu Leu Lys
   1 5
<210> 71
   <211> 7
   <212> PRT
   <213> Artificial Sequence
<220>
   <223> Humanised sequence
<400> 71
   Ala Ala Ser Ser Leu Gln Asn
   1 5
<210> 72
   <211> 7
   <212> PRT
   <213> Artificial Sequence
<220>
   <223> Humanised sequence
<400> 72
   Ala Ala Ser Ser Leu Gln Lys
   1 5
<210> 73
   <211> 9
   <212> PRT
   <213> Artificial Sequence
<220>
   <223> Humanised sequence
<400> 73
   Gln Arg Tyr Asp Arg Pro Pro Tyr Thr
    1               5
<210> 74
   <211> 9
   <212> PRT
   <213> Artificial Sequence
<220>
   <223> Humanised sequence
<400> 74
   Gln Arg Tyr Asp Lys Pro Pro Tyr Thr
    1               5
<210> 75
   <211> 9
   <212> PRT
   <213> Artificial Sequence
<220>
   <223> Humanised sequence
<400> 75
   Gln Arg Tyr Asn Arg Pro Pro Tyr Thr
    1               5
<210> 76
   <211> 9
   <212> PRT
   <213> Artificial Sequence
<220>
   <223> Humanised sequence
<400> 76
   Gln Arg Tyr Asn Lys Pro Pro Tyr Thr
    1               5
<210> 77
   <211> 363
   <212> DNA
   <213> Artificial Sequence
<220>
   <223> Humanised sequence
<400> 77
   gaggtgcagc tggtggagtc tggcggcgga ctggtgcagc ccggcagaag cctgagactg 60
   agctgtgccg ccagcggctt caccttcgac cagtacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agc                                                               363
<210> 78
   <211> 121
   <212> PRT
   <213> Artificial Sequence
<220>
   <223> Humanised sequence
<400> 78
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
                   100                 105                 110
   Gln Gly Thr Leu Val Thr Val Ser Ser
           115                 120
<210> 79
   <211> 363
   <212> DNA
   <213> Artificial Sequence
<220>
   <223> Humanised sequence
<400> 79
   gaggtgcagc tggtggagtc tggcggcgga ctggtgcagc ccggcagaag cctgagactg 60
   agctgtgccg ccagcggctt caccttcgac gaccacgccc tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgagg 300
   tacctgagca ccgccagcag cctggactac tggggccagg gcacactagt gaccgtgtcc 360
   agc                                                               363
<210> 80
   <211> 121
   <212> PRT
   <213> Artificial Sequence
<220>
   <223> Humanised sequence
<400> 80
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp His
               20                  25                  30
   Ala Leu His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Arg Tyr Leu Ser Thr Ala Ser Ser Leu Asp Tyr Trp Gly
               100                 105                 110
   Gln Gly Thr Leu Val Thr Val Ser Ser
           115                 120
<210> 81
   <211> 363
   <212> DNA
   <213> Artificial Sequence
<220>
   <223> Humanised sequence
<400> 81
   gaggtgcagc tggtggagtc tggcggcgga ctggtgcagc ccggcagaag cctgagactg 60
   agctgtgccg ccagcggctt caccttcgac cactacgccc tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agc                                                               363
<210> 82
   <211> 121
   <212> PRT
   <213> Artificial Sequence
<220>
   <223> Humanised sequence
<400> 82
   Glu Val Gln Leu Val Glu Ser Gly Gly Gly Leu Val Gln Pro Gly Arg
    1               5                   10                  15
   Ser Leu Arg Leu Ser Cys Ala Ala Ser Gly Phe Thr Phe Asp His Tyr
               20                  25                  30
   Ala Leu His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser
           115                 120
<210> 83
   <211> 363
   <212> DNA
   <213> Artificial Sequence
<220>
   <223> Humanised sequence
<400> 83
   gaggtgcagc tggtggagtc tggcggcgga ctggtgcagc ccggcagaag cctgagactg 60
   agctgtgccg ccagcggctt caccttcgac gactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgcac 300
   tacctgagca ccgccagcca actgcaccac tggggccagg gcacactagt gaccgtgtcc 360
   agc                                                               363
<210> 84
   <211> 121
   <212> PRT
   <213> Artificial Sequence
<220>
   <223> Humanised sequence
<400> 84
   Glu Val Gln Leu Val Glu Ser Gly Gly Gly Leu Val Gln Pro Gly Arg
    1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                           85                  90                  95
   Ala Lys Val His Tyr Leu Ser Thr Ala Ser Gln Leu His His Trp Gly
               100                 105                 110
   Gln Gly Thr Leu Val Thr Val Ser Ser
           115                 120
<210> 85
   <211> 363
   <212> DNA
   <213> Artificial Sequence
<220>
   <223> Humanised sequence
<400> 85
   gaggtgcagc tggtggagtc tggcggcgga ctggtgcagc ccggcagaag cctgagactg 60
   agctgtgccg ccagcggctt caccttcgac cactacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgcag 300
   tacctgagca ccgccagcag cctgcagagc tggggccagg gcacactagt gaccgtgtcc 360
   agc                                                               363
<210> 86
   <211> 121
   <212> PRT
   <213> Artificial Sequence
<220>
   <223> Humanised sequence
<400> 86
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp His Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Gln Tyr Leu Ser Thr Ala Ser Ser Leu Gln Ser Trp Gly
               100                 105                 110
   Gln Gly Thr Leu Val Thr Val Ser Ser
           115                 120
<210> 87
   <211> 363
   <212> DNA
   <213> Artificial Sequence
<220>
   <223> Humanised sequence
<400> 87
   gaggtgcagc tggtggagtc tggcggcgga ctggtgcagc ccggcagaag cctgagactg 60
   agctgtgccg ccagcggctt caccttcgac cagtacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgaag 300
   tacctgagca ccgccagcag cctgcactac tggggccagg gcacactagt gaccgtgtcc 360
   agc                                                               363
<210> 88
   <211> 121
   <212> PRT
   <213> Artificial Sequence
<220>
   <223> Humanised sequence
<400> 88
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Lys Tyr Leu Ser Thr Ala Ser Ser Leu His Tyr Trp Gly
               100                 105                 110
   Gln Gly Thr Leu Val Thr Val Ser Ser
           115                 120
<210> 89
   <211> 363
   <212> DNA
   <213> Artificial Sequence
<220>
   <223> Humanised sequence
<400> 89
   gaggtgcagc tggtggagtc tggcggcgga ctggtgcagc ccggcagaag cctgagactg 60
   agctgtgccg ccagcggctt caccttcgac cagcacgccc tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgcac 300
   tacctgagca ccgccagcag cctggactac tggggccagg gcacactagt gaccgtgtcc 360
   agc                                                               363
<210> 90
   <211> 121
   <212> PRT
   <213> Artificial Sequence
<220>
   <223> Humanised sequence
<400> 90
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln His
               20                  25                  30
   Ala Leu His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val His Tyr Leu Ser Thr Ala Ser Ser Leu Asp Tyr Trp Gly
               100                 105                 110
   Gln Gly Thr Leu Val Thr Val Ser Ser
           115                 120
<210> 91
   <211> 363
   <212> DNA
   <213> Artificial Sequence
<220>
   <223> Humanised sequence
<400> 91
   gaggtgcagc tggtggagtc tggcggcgga ctggtgcagc ccggcagaag cctgagactg 60
   agctgtgccg ccagcggctt caccttcgac cagtacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgcac 300
   tacctgagca ccgccagcca gctgcaccac tggggccagg gcacactagt gaccgtgtcc 360
   agc                                                               363
<210> 92
   <211> 121
   <212> PRT
   <213> Artificial Sequence
<220>
   <223> Humanised sequence
<400> 92
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val His Tyr Leu Ser Thr Ala Ser Gln Leu His His Trp Gly
               100                 105                 110
   Gln Gly Thr Leu Val Thr Val Ser Ser
           115                 120
<210> 93
   <211> 363
   <212> DNA
   <213> Artificial Sequence
<220>
   <223> Humanised sequence
<400> 93
   gaggtgcagc tggtggagtc tggcggcgga ctggtgcagc ccggcagaag cctgagactg 60
   agctgtgccg ccagcggctt caccttcgac cagcacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agc                                                               363
<210> 94
   <211> 121
   <212> PRT
   <213> Artificial Sequence
<220>
   <223> Humanised sequence
<400> 94
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln His
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser
           115                 120
<210> 95
   <211> 363
   <212> DNA
   <213> Artificial Sequence
<220>
   <223> Humanised sequence
<400> 95
   gaggtgcagc tggtggagtc tggcggcgga ctggtgcagc ccggcagaag cctgagactg 60
   agctgtgccg ccagcggctt caccttcgac cagtacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgaag 300
   tacctgagca ccgccagcaa cctggagagc tggggccagg gcacactagt gaccgtgtcc 360
   agc                                                               363
<210> 96
   <211> 121
   <212> PRT
   <213> Artificial Sequence
<220>
   <223> Humanised sequence
<400> 96
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Lys Tyr Leu Ser Thr Ala Ser Asn Leu Glu Ser Trp Gly
               100                 105                 110
   Gln Gly Thr Leu Val Thr Val Ser Ser
           115                 120
<210> 97
   <211> 327
   <212> DNA
   <213> Artificial Sequence
<220>
   <223> Humanised sequence
<400> 97
   gatatccaga tgacccagag ccccagcagc ctgagcgcct ctgtgggcga tagagtgacc 60
   atcacctgcc acgccagcaa gaagatcaga aactacctgg cctggtatca gcagaagcct 120
   ggcaaggccc ctaagctgct gatctacgcc gccagcagcc tgctgagggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 98
   <211> 109
   <212> PRT
   <213> Artificial Sequence
<220>
   <223> Humanised sequence
<400> 98
   Asp Ile Gln Met Thr Gln Ser Pro Ser Ser Leu Ser Ala Ser Val Gly
    1               5                  10                  15
   Asp Arg Val Thr Ile Thr Cys His Ala Ser Lys Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 99
   <211> 327
   <212> DNA
   <213> Artificial Sequence
<220>
   <223> Humanised sequence
<400> 99
   gatatccaga tgacccagag ccccagcagc ctgagcgcct ctgtgggcga tagagtgacc 60
   atcacctgcc acgccagcag gaagctgaga aactacctgg cctggtatca gcagaagcct 120
   ggcaaggccc ctaagctgct gatctacgcc gccagcagcc tgctgaaggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 100
   <211> 109
   <212> PRT
   <213> Artificial Sequence
<220>
   <223> Humanised sequence
<400> 100
   Asp Ile Gln Met Thr Gln Ser Pro Ser Ser Leu Ser Ala Ser Val Gly
   1               5                   10                  15
   Asp Arg Val Thr Ile Thr Cys His Ala Ser Arg Lys Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Lys Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 101
   <211> 327
   <212> DNA
   <213> Artificial Sequence
<220>
   <223> Humanised sequence
<400> 101
   gatatccaga tgacccagag ccccagcagc ctgagcgcct ctgtgggcga tagagtgacc 60
   atcacctgcc acgccagcag gaggctgaga aactacctgg cctggtatca gcagaagcct 120
   ggcaaggccc ctaagctgct gatctacgcc gccagcagcc tgctgcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 102
   <211> 109
   <212> PRT
   <213> Artificial Sequence
<220>
   <223> Humanised sequence
<400> 102
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Arg Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 103
   <211> 327
   <212> DNA
   <213> Artificial Sequence
<220>
   <223> Humanised sequence
<400> 103
   gatatccaga tgacccagag ccccagcagc ctgagcgcct ctgtgggcga tagagtgacc 60
   atcacctgcc acgccagcag gaagctgaga aactacctgg cctggtatca gcagaagcct 120
   ggcaaggccc ctaagctgct gatctacgcc gccagcagcc tgctgagggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 104
   <211> 109
   <212> PRT
   <213> Artificial Sequence
<220>
   <223> Humanised sequence
<400> 104
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 105
   <211> 327
   <212> DNA
   <213> Artificial Sequence
<220>
   <223> Humanised sequence
<400> 105
<210> 106
   <211> 109
   <212> PRT
   <213> Artificial Sequence
<220>
   <223> Humanised sequence
<400> 106
   Asp Ile Gln Met Thr Gln Ser Pro Ser Ser Leu Ser Ala Ser Val Gly
    1               5                  10                  15
   Asp Arg Val Thr Ile Thr Cys His Ala Ser Lys Arg Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Lys Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Lys Pro Pro Tyr
                   85                  90                  95
   Thr Phe Gly Gln Gly Thr Lys Val Glu Ile Lys Arg Thr
               100                 105
<210> 107
   <211> 327
   <212> DNA
   <213> Artificial Sequence
<220>
   <223> Humanised sequence
<400> 107
   gatatccaga tgacccagag ccccagcagc ctgagcgcct ctgtgggcga tagagtgacc 60
   atcacctgcc acgccagcag gaagctgaga aactacctgg cctggtatca gcagaagcct 120
   ggcaaggccc ctaagctgct gatctacgcc gccagcagcc tgctgagggg cgtgcccagc 180
   agattcagcg gcagcggctc cggcaccgac ttcaccctga ccatcagcag cctgcagccc 240
   gaggacgtgg ccacctacta ctgccagcgg tacaacagac ccccttacac cttcggccag 300
   ggcaccaagg tggagatcaa gcgtacg                                      327
<210> 108
   <211> 109
   <212> PRT
   <213> Artificial Sequence
<220>
   <223> Humanised sequence
<400> 108
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Arg Pro Pro Tyr
                   85                  90                  95
   Thr Phe Gly Gln Gly Thr Lys Val Glu Ile Lys Arg Thr
               100                 105
<210> 109
   <211> 327
   <212> DNA
   <213> Artificial Sequence
<220>
   <223> Humanised sequence
<400> 109
   gatatccaga tgacccagag ccccagcagc ctgagcgcct ctgtgggcga tagagtgacc 60
   atcacctgcc acgccagcag gaagatcaga aactacctgg cctggtatca gcagaagcct 120
   ggcaaggccc ctaagctgct gatctacgcc gccagcagcc tgcagcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                      327
<210> 110
   <211> 109
   <212> PRT
   <213> Artificial Sequence
<220>
   <223> Humanised sequence
<400> 110
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 111
   <211> 327
   <212> DNA
   <213> Artificial Sequence
<220>
   <223> Humanised sequence
<400> 111
   gatatccaga tgacccagag ccccagcagc ctgagcgcct ctgtgggcga tagagtgacc 60
   atcacctgcc acgccagcag gaggatcaga aactacctgg cctggtatca gcagaagcct 120
   ggcaaggccc ctaagctgct gatctacgcc gccagcagcc tgctgcacgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 112
   <211> 109
   <212> PRT
   <213> Artificial Sequence
<220>
   <223> Humanised sequence
<400> 112
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Arg Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu His Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 113
   <211> 327
   <212> DNA
   <213> Artificial Sequence
<220>
   <223> Humanised sequence
<400> 113
   gatatccaga tgacccagag ccccagcagc ctgagcgcct ctgtgggcga tagagtgacc 60
   atcacctgcc acgccagcag gaggctgaga aactacctgg cctggtatca gcagaagcct 120
   ggcaaggccc ctaagctgct gatctacgcc gccagcagcc tgcagcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 114
   <211> 109
   <212> PRT
   <213> Artificial Sequence
<220>
   <223> Humanised sequence
<400> 114
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Arg Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 115
   <211> 327
   <212> DNA
   <213> Artificial Sequence
<220>
   <223> Humanised sequence
<400> 115
   gatatccaga tgacccagag ccccagcagc ctgagcgcct ctgtgggcga tagagtgacc 60
   atcacctgcc acgccagcag gaggctgaga aactacctgg cctggtatca gcagaagcct 120
   ggcaaggccc ctaagctgct gatctacgcc gccagcagcc tgctgaaggg cgtgcccagc 180
   agattcagcg gcagcggctc cggcaccgac ttcaccctga ccatcagcag cctgcagccc 240
   gaggacgtgg ccacctacta ctgccagcgg tacaacaagc ccccttacac cttcggccag 300
   ggcaccaagg tggagatcaa gcgtacg                                     327
<210> 116
   <211> 109
   <212> PRT
   <213> Artificial Sequence
<220>
   <223> Humanised sequence
<400> 116
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Arg Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Lys Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Lys Pro Pro Tyr
                   85                  90                  95
   Thr Phe Gly Gln Gly Thr Lys Val Glu Ile Lys Arg Thr
               100                 105
<210> 117
   <211> 327
   <212> DNA
   <213> Artificial Sequence
<220>
   <223> Humanised sequence
<400> 117
   gatatccaga tgacccagag ccccagcagc ctgagcgcct ctgtgggcga tagagtgacc 60
   atcacctgcc acgccagcag gaagatcaga aactacctgg cctggtatca gcagaagcct 120
   ggcaaggccc ctaagctgct gatctacgcc gccagcagct tcctgcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 118
   <211> 109
   <212> PRT
   <213> Artificial Sequence
<220>
   <223> Humanised sequence
<400> 118
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Phe Leu Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 119
   <211> 327
   <212> DNA
   <213> Artificial Sequence
<220>
   <223> Humanised sequence
<400> 119
   gatatccaga tgacccagag ccccagcagc ctgagcgcct ctgtgggcga tagagtgacc 60
   atcacctgcc acgccagcaa gaagatcaga aactacctgg cctggtatca gcagaagcct 120
   ggcaaggccc ctaagctgct gatctacgcc gccagcagcc tgcagcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 120
   <211> 109
   <212> PRT
   <213> Artificial Sequence
<220>
   <223> Humanised sequence
<400> 120
   Asp Ile Gln Met Thr Gln Ser Pro Ser Ser Leu Ser Ala Ser Val Gly
    1               5                  10                  15
   Asp Arg Val Thr Ile Thr Cys His Ala Ser Lys Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 121
   <211> 327
   <212> DNA
   <213> Artificial Sequence
<220>
   <223> Humanised sequence
<400> 121
   gatatccaga tgacccagag ccccagcagc ctgagcgcct ctgtgggcga tagagtgacc 60
   atcacctgcc acgccagcag gaggatcaga aactacctgg cctggtatca gcagaagcct 120
   ggcaaggccc ctaagctgct gatctacgcc gccagcagcc tgctgcaggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                    327
<210> 122
   <211> 109
   <212> PRT
   <213> Artificial Sequence
<220>
   <223> Humanised sequence
<400> 122
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Arg Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Gln Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 123
   <211> 327
   <212> DNA
   <213> Artificial Sequence
<220>
   <223> Humanised sequence
<400> 123
   gatatccaga tgacccagag ccccagcagc ctgagcgcct ctgtgggcga tagagtgacc 60
   atcacctgcc acgccagcag gaagctgaga aactacctgg cctggtatca gcagaagcct 120
   ggcaaggccc ctaagctgct gatctacgcc gccagcagcc tgcagcaggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                    327
<210> 124
   <211> 109
   <212> PRT
   <213> Artificial Sequence
<220>
   <223> Humanised sequence
<400> 124
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Gln Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 125
   <211> 327
   <212> DNA
   <213> Artificial Sequence
<220>
   <223> Humanised sequence
<400> 125
   gatatccaga tgacccagag ccccagcagc ctgagcgcct ctgtgggcga tagagtgacc 60
   atcacctgcc acgccagcag gaagctgaga aactacctgg cctggtatca gcagaagcct 120
   ggcaaggccc ctaagctgct gatctacgcc gccagcagcc tgctgcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                    327
<210> 126
   <211> 109
   <212> PRT
   <213> Artificial Sequence
<220>
   <223> Humanised sequence
<400> 126
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 127
   <211> 327
   <212> DNA
   <213> Artificial Sequence
<220>
   <223> Humanised sequence
<400> 127
   gatatccaga tgacccagag ccccagcagc ctgagcgcct ctgtgggcga tagagtgacc 60
   atcacctgcc acgccagcag ggagatcaga aactacctgg cctggtatca gcagaagcct 120
   ggcaaggccc ctaagctgct gatctacgcc gccagcagcc tgctgcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 128
   <211> 109
   <212> PRT
   <213> Artificial Sequence
<220>
   <223> Humanised sequence
<400> 128
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Glu Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 129
   <211> 327
   <212> DNA
   <213> Artificial Sequence
<220>
   <223> Humanised sequence
<400> 129
   gatatccaga tgacccagag ccccagcagc ctgagcgcct ctgtgggcga tagagtgacc 60
   atcacctgcc acgccagcca gggcatcaga aactacctgg cctggtatca gcagaagcct 120
   ggcaaggccc ctaagctgct gatctacgcc gccagcaccc tgctgaaggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                    327
<210> 130
   <211> 109
   <212> PRT
   <213> Artificial Sequence
<220>
   <223> Humanised sequence
<400> 130
   Asp Ile Gln Met Thr Gln Ser Pro Ser Ser Leu Ser Ala Ser Val Gly
    1               5                  10                  15
   Asp Arg Val Thr Ile Thr Cys His Ala Ser Gln Gly Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Thr Leu Leu Lys Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 131
   <211> 327
   <212> DNA
   <213> Artificial Sequence
<220>
   <223> Humanised sequence
<400> 131
   gatatccaga tgacccagag ccccagcagc ctgagcgcct ctgtgggcga tagagtgacc 60
   atcacctgcc acgccagcca gaagatcaga aactacctgg cctggtatca gcagaagcct 120
   ggcaaggccc ctaagctgct gatctacgcc gccagcagcc tgcagcaggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                    327
<210> 132
   <211> 109
   <212> PRT
   <213> Artificial Sequence
<220>
   <223> Humanised sequence
<400> 132
   Asp Ile Gln Met Thr Gln Ser Pro Ser Ser Leu Ser Ala Ser Val Gly
   1               5                   10                  15
   Asp Arg Val Thr Ile Thr Cys His Ala Ser Gln Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Gln Gln Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 133
   <211> 327
   <212> DNA
   <213> Artificial Sequence
<220>
   <223> Humanised sequence
<400> 133
   gatatccaga tgacccagag ccccagcagc ctgagcgcct ctgtgggcga tagagtgacc 60
   atcacctgcc acgccagcag gaggctgaga aactacctgg cctggtatca gcagaagcct 120
   ggcaaggccc ctaagctgct gatctacgcc gccagcagcc tgctgcacgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                    327
<210> 134
   <211> 109
   <212> PRT
   <213> Artificial Sequence
<220>
   <223> Humanised sequence
<400> 134
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Arg Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu His Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 135
   <211> 327
   <212> DNA
   <213> Artificial Sequence
<220>
   <223> Humanised sequence
<400> 135
   gatatccaga tgacccagag ccccagcagc ctgagcgcct ctgtgggcga tagagtgacc 60
   atcacctgcc acgccagcag gaggctgaga aactacctgg cctggtatca gcagaagcct 120
   ggcaaggccc ctaagctgct gatctacgcc gccagcagcc tgctgaaggg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 136
   <211> 109
   <212> PRT
   <213> Artificial Sequence
<220>
   <223> Humanised sequence
<400> 136
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Arg Leu Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Lys Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 137
   <211> 327
   <212> DNA
   <213> Artificial Sequence
<220>
   <223> Humanised sequence
<400> 137
   gatatccaga tgacccagag ccccagcagc ctgagcgcct ctgtgggcga tagagtgacc 60
   atcacctgcc acgccagcaa gaggatcaga aactacctgg cctggtatca gcagaagcct 120
   ggcaaggccc ctaagctgct gatctacgcc gccagcagcc tgctgagggg cgtgcccagc 180
   agattcagcg gcagcggctc cggcaccgac ttcaccctga ccatcagcag cctgcagccc 240
   gaggacgtgg ccacctacta ctgccagcgg tacaacaagc ccccttacac cttcggccag 300
   ggcaccaagg tggagatcaa gcgtacg                                    327
<210> 138
   <211> 109
   <212> PRT
   <213> Artificial Sequence
<220>
   <223> Humanised sequence
<400> 138
   Asp Ile Gln Met Thr Gln Ser Pro Ser Ser Leu Ser Ala Ser Val Gly
    1               5                  10                  15
   Asp Arg Val Thr Ile Thr Cys His Ala Ser Lys Arg Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Lys Pro Pro Tyr
                   85                  90                  95
   Thr Phe Gly Gln Gly Thr Lys Val Glu Ile Lys Arg Thr
               100                 105
<210> 139
   <211> 327
   <212> DNA
   <213> Artificial Sequence
<220>
   <223> Humanised sequence
<400> 139
   gatatccaga tgacccagag ccccagcagc ctgagcgcct ctgtgggcga tagagtgacc 60
   atcacctgcc acgccagcag gaagatcaga aactacctgg cctggtatca gcagaagcct 120
   ggcaaggccc ctaagctgct gatctacgcc gccagcagcc tgctgagggg cgtgcccagc 180
   agattcagcg gcagcggctc cggcaccgac ttcaccctga ccatcagcag cctgcagccc 240
   gaggacgtgg ccacctacta ctgccagcgg tacgacaagc ccccttacac cttcggccag 300
   ggcaccaagg tggagatcaa gcgtacg                                    327
<210> 140
   <211> 109
   <212> PRT
   <213> Artificial Sequence
<220>
   <223> Humanised sequence
<400> 140
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Lys Pro Pro Tyr
                   85                  90                  95
   Thr Phe Gly Gln Gly Thr Lys Val Glu Ile Lys Arg Thr
                       100                 105
<210> 141
   <211> 327
   <212> DNA
   <213> Artificial Sequence
<220>
   <223> Humanised sequence
<400> 141
   gatatccaga tgacccagag ccccagcagc ctgagcgcct ctgtgggcga tagagtgacc 60
   atcacctgcc acgccagcaa gaggatcaga aactacctgg cctggtatca gcagaagcct 120
   ggcaaggccc ctaagctgct gatctacgcc gccagcagcc tgctgaaggg cgtgcccagc 180
   agattcagcg gcagcggctc cggcaccgac ttcaccctga ccatcagcag cctgcagccc 240
   gaggacgtgg ccacctacta ctgccagcgg tacaacaagc ccccttacac cttcggccag 300
   ggcaccaagg tggagatcaa gcgtacg                                     327
<210> 142
   <211> 109
   <212> PRT
   <213> Artificial Sequence
<220>
   <223> Humanised sequence
<400> 142
   Asp Ile Gln Met Thr Gln Ser Pro Ser Ser Leu Ser Ala Ser Val Gly
   1               5                   10                  15
   Asp Arg Val Thr Ile Thr Cys His Ala Ser Lys Arg Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Lys Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Lys Pro Pro Tyr
                   85                  90                  95
   Thr Phe Gly Gln Gly Thr Lys Val Glu Ile Lys Arg Thr
               100                 105
<210> 143
   <211> 327
   <212> DNA
   <213> Artificial Sequence
<220>
   <223> Humanised sequence
<400> 143
   gatatccaga tgacccagag ccccagcagc ctgagcgcct ctgtgggcga tagagtgacc 60
   atcacctgcc acgccagcag gaagatcaga aactacctgg cctggtatca gcagaagcct 120
   ggcaaggccc ctaagctgct gatctacgcc gccagcagcc tgctgcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacg                                     327
<210> 144
   <211> 109
   <212> PRT
   <213> Artificial Sequence
<220>
   <223> Humanised sequence
<400> 144
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr
               100                 105
<210> 145
   <211> 451
   <212> PRT
   <213> Artificial Sequence
<220>
   <223> Humanised sequence
<400> 145
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Tyr
                   245                 250                 255
   Ile Thr Arg Glu Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 146
   <211> 451
   <212> PRT
   <213> Artificial Sequence
<220>
   <223> Humanised sequence
<400> 146
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp His
               20                  25                  30
   Ala Leu His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Arg Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Tyr
                   245                 250                 255
   Ile Thr Arg Glu Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 147
   <211> 642
   <212> DNA
   <213> Artificial Sequence
<220>
   <223> Humanised sequence
<400> 147
   gatatccaga tgacccagag ccccagcagc ctgagcgcct ctgtgggcga tagagtgacc 60
   atcacctgcc acgccagcag gaagatcaga aactacctgg cctggtatca gcagaagcct 120
   ggcaaggccc ctaagctgct gatctacgcc gccagcagcc tgctgagggg cgtgcccagc 180
   agattcagcg gcagcggctc cggcaccgac ttcaccctga ccatcagcag cctgcagccc 240
   gaggacgtgg ccacctacta ctgccagcgg tacgacaagc ccccttacac cttcggccag 300
   ggcaccaagg tggagatcaa gcgtacggtg gccgccccca gcgtgttcat cttccccccc 360
   agcgatgagc agctgaagag cggcaccgcc agcgtggtgt gtctgctgaa caacttctac 420
   ccccgggagg ccaaggtgca gtggaaggtg gacaatgccc tgcagagcgg caacagccag 480
   gagagcgtga ccgagcagga cagcaaggac tccacctaca gcctgagcag caccctgacc 540
   ctgagcaagg ccgactacga gaagcacaag gtgtacgcct gtgaggtgac ccaccagggc 600
   ctgtccagcc ccgtgaccaa gagcttcaac cggggcgagt gc                    642
<210> 148
   <211> 214
   <212> PRT
   <213> Artificial Sequence
<220>
   <223> Humanised sequence
<400> 148
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Arg Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Lys Pro Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 149
   <211> 642
   <212> DNA
   <213> Artificial Sequence
<220>
   <223> Humanised sequence
<400> 149
   gatatccaga tgacccagag ccccagcagc ctgagcgcct ctgtgggcga tagagtgacc 60
   atcacctgcc acgccagcaa gaggatcaga aactacctgg cctggtatca gcagaagcct 120
   ggcaaggccc ctaagctgct gatctacgcc gccagcagcc tgctgaaggg cgtgcccagc 180
   agattcagcg gcagcggctc cggcaccgac ttcaccctga ccatcagcag cctgcagccc 240
   gaggacgtgg ccacctacta ctgccagcgg tacaacaagc ccccttacac cttcggccag 300
   ggcaccaagg tggagatcaa gcgtacggtg gccgccccca gcgtgttcat cttccccccc 360
   agcgatgagc agctgaagag cggcaccgcc agcgtggtgt gtctgctgaa caacttctac 420
   ccccgggagg ccaaggtgca gtggaaggtg gacaatgccc tgcagagcgg caacagccag 480
   gagagcgtga ccgagcagga cagcaaggac tccacctaca gcctgagcag caccctgacc 540
   ctgagcaagg ccgactacga gaagcacaag gtgtacgcct gtgaggtgac ccaccagggc 600
   ctgtccagcc ccgtgaccaa gagcttcaac cggggcgagt gc                   642
<210> 150
   <211> 214
   <212> PRT
   <213> Artificial Sequence
<220>
   <223> Humanised sequence
<400> 150
   Asp Ile Gln Met Thr Gln Ser Pro Ser Ser Leu Ser Ala Ser Val Gly
    1               5                  10                  15
   Asp Arg Val Thr Ile Thr Cys His Ala Ser Lys Arg Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Lys Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asn Lys Pro Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 151
   <211> 642
   <212> DNA
   <213> Artificial Sequence
<220>
   <223> Humanised sequence
<400> 151
   gatatccaga tgacccagag ccccagcagc ctgagcgcct ctgtgggcga tagagtgacc 60
   atcacctgcc acgccagcag gaagatcaga aactacctgg cctggtatca gcagaagcct 120
   ggcaaggccc ctaagctgct gatctacgcc gccagcagcc tgctgcccgg cgtgcccagc 180
   agattcagcg gcagcggctc cggcaccgac ttcaccctga ccatcagcag cctgcagccc 240
   gaggacgtgg ccacctacta ctgccagcgg tacgacagac ccccttacac cttcggccag 300
   ggcaccaagg tggagatcaa gcgtacggtg gccgccccca gcgtgttcat cttccccccc 360
   agcgatgagc agctgaagag cggcaccgcc agcgtggtgt gtctgctgaa caacttctac 420
   ccccgggagg ccaaggtgca gtggaaggtg gacaatgccc tgcagagcgg caacagccag 480
   gagagcgtga ccgagcagga cagcaaggac tccacctaca gcctgagcag caccctgacc 540
   ctgagcaagg ccgactacga gaagcacaag gtgtacgcct gtgaggtgac ccaccagggc 600
   ctgtccagcc ccgtgaccaa gagcttcaac cggggcgagt gc                   642
<210> 152
   <211> 214
   <212> PRT
   <213> Artificial Sequence
<220>
   <223> Humanised sequence
<400> 152
   Asp Ile Gln Met Thr Gln Ser Pro Ser Ser Leu Ser Ala Ser Val Gly
    1               5                  10                  15
   Asp Arg Val Thr Ile Thr Cys His Ala Ser Arg Lys Ile Arg Asn Tyr
               20                  25                  30
   Leu Ala Trp Tyr Gln Gln Lys Pro Gly Lys Ala Pro Lys Leu Leu Ile
           35                  40                  45
   Tyr Ala Ala Ser Ser Leu Leu Pro Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Val Ala Thr Tyr Tyr Cys Gln Arg Tyr Asp Arg Pro Pro Tyr
                   85                  90                  95
   Thr Phe Gly Gln Gly Thr Lys Val Glu Ile Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 153
   <211> 1353
   <212> DNA
   <213> Artificial Sequence
<220>
   <223> Humanised sequence
<400> 153
   gaggtgcagc tggtggagtc tggcggcgga ctggtgcagc ccggcagaag cctgagactg 60
   agctgtgccg ccagcggctt caccttcgac cagtacgcca tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgtcc 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag                              1353
<210> 154
   <211> 451
   <212> PRT
   <213> Artificial Sequence
<220>
   <223> Humanised sequence
<400> 154
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Gln Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 155
   <211> 1353
   <212> DNA
   <213> Artificial Sequence
<220>
   <223> Humanised sequence
<400> 155
   gaggtgcagc tggtggagtc tggcggcgga ctggtgcagc ccggcagaag cctgagactg 60
   agctgtgccg ccagcggctt caccttcgac gaccacgccc tgcactgggt gaggcaggcc 120
   cctggcaagg gcctggagtg ggtgtccgcc atcacctgga atagcggcca catcgactac 180
   gccgacagcg tggagggcag attcaccatc agccgggaca acgccaagaa cagcctgtac 240
   ctgcagatga acagcctgag agccgaggac accgccgtgt actactgtgc caaggtgagg 300
   tacctgagca ccgccagcag cctggactac tggggccagg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgatgat cagcagaacc 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag                              1353
<210> 156
   <211> 451
   <212> PRT
   <213> Artificial Sequence
<220>
   <223> Humanised sequence
<400> 156
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp His
               20                  25                  30
   Ala Leu His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Arg Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 157
   <211> 1353
   <212> DNA
   <213> Artificial Sequence
<220>
   <223> Humanised sequence
<400> 157
   caggtgaccc tgagggagag cggccccgcc ctggtgaagc ccacccagac cctgaccctg 60
   acctgcacct tcagcggctt tagcctcagc acctccggca tgggcgtgag ctggatcagg 120
   cagccacccg gcaaaggcct ggagtggctg gcccacatct actgggacga cgacaagagg 180
   tacaacccca gcctgaagag ccggctgacc atcagcaagg ataccagcag gaaccaggtg 240
   gtgctgacca tgaccaacat ggaccccgtg gacaccgcta cctactactg cgccaggagg 300
   gagaccgtct tctactggta cttcgacgtg tggggaaggg gcacactagt gaccgtgtcc 360
   agcgccagca ccaagggccc cagcgtgttc cccctggccc ccagcagcaa gagcaccagc 420
   ggcggcacag ccgccctggg ctgcctggtg aaggactact tccccgaacc ggtgaccgtg 480
   tcctggaaca gcggagccct gaccagcggc gtgcacacct tccccgccgt gctgcagagc 540
   agcggcctgt acagcctgag cagcgtggtg accgtgccca gcagcagcct gggcacccag 600
   acctacatct gtaacgtgaa ccacaagccc agcaacacca aggtggacaa gaaggtggag 660
   cccaagagct gtgacaagac ccacacctgc cccccctgcc ctgcccccga gctgctggga 720
   ggccccagcg tgttcctgtt cccccccaag cctaaggaca ccctgtacat caccagagag 780
   cccgaggtga cctgtgtggt ggtggatgtg agccacgagg accctgaggt gaagttcaac 840
   tggtacgtgg acggcgtgga ggtgcacaat gccaagacca agcccaggga ggagcagtac 900
   aacagcacct accgggtggt gtccgtgctg accgtgctgc accaggattg gctgaacggc 960
   aaggagtaca agtgtaaggt gtccaacaag gccctgcctg cccctatcga gaaaaccatc 1020
   agcaaggcca agggccagcc cagagagccc caggtgtaca ccctgccccc tagcagagat 1080
   gagctgacca agaaccaggt gtccctgacc tgcctggtga agggcttcta ccccagcgac 1140
   atcgccgtgg agtgggagag caacggccag cccgagaaca actacaagac caccccccct 1200
   gtgctggaca gcgatggcag cttcttcctg tacagcaagc tgaccgtgga caagagcaga 1260
   tggcagcagg gcaacgtgtt cagctgctcc gtgatgcacg aggccctgca caatcactac 1320
   acccagaaga gcctgagcct gtcccctggc aag                               1353
<210> 158
   <211> 451
   <212> PRT
   <213> Artificial Sequence
<220>
   <223> Humanised sequence
<400> 158
   Gln Val Thr Leu Arg Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Met Gly Val Ser Trp Ile Arg Gln Pro Pro Gly Lys Gly Leu Glu
           35                  40                  45
   Trp Leu Ala His Ile Tyr Trp Asp Asp Asp Lys Arg Tyr Asn Pro Ser
       50                  55                  60
   Leu Lys Ser Arg Leu Thr Ile Ser Lys Asp Thr Ser Arg Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Arg Glu Thr Val Phe Tyr Trp Tyr Phe Asp Val Trp Gly
               100                 105                 110
   Arg Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Tyr
                   245                 250                 255
   Ile Thr Arg Glu Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 159
   <211> 654
   <212> DNA
   <213> Artificial Sequence
<220>
   <223> Humanised sequence
<400> 159
   gacatcgtgc tgacccagag cccctcttcc ctgagcgcaa gcgtgggcga tagggtgacc 60
   atcacctgca aggccagcca gagcgtggac tacgacggcg acagctacat gaactggtac 120
   cagcagaagc ccggcaaggc ccccaaactg ctgatctacg ccgccagcaa cctcgagtca 180
   ggcattccca gcaggtttag cggcagcggc agcggcaccg acttcacctt cacaatcagc 240
   agcctgcagc ccgaggacat cgccacctac tactgccagc agagcaacga ggaccctccc 300
   accttcggac agggcaccaa ggtcgagatc aagcgtacgg tggccgcccc cagcgtgttc 360
   atcttccccc ccagcgatga gcagctgaag agcggcaccg ccagcgtggt gtgtctgctg 420
   aacaacttct acccccggga ggccaaggtg cagtggaagg tggacaatgc cctgcagagc 480
   ggcaacagcc aggagagcgt gaccgagcag gacagcaagg actccaccta cagcctgagc 540
   agcaccctga ccctgagcaa ggccgactac gagaagcaca aggtgtacgc ctgtgaggtg 600
   acccaccagg gcctgtccag ccccgtgacc aagagcttca accggggcga gtgc       654
<210> 160
   <211> 218
   <212> PRT
   <213> Artificial Sequence
<220>
   <223> Humanised sequence
<400> 160
   Asp Ile Val Leu Thr Gln Ser Pro Ser Ser Leu Ser Ala Ser Val Gly
   1               5                   10                  15
   Asp Arg Val Thr Ile Thr Cys Lys Ala Ser Gln Ser Val Asp Tyr Asp
               20                  25                  30
   Gly Asp Ser Tyr Met Asn Trp Tyr Gln Gln Lys Pro Gly Lys Ala Pro
           35                  40                  45
   Lys Leu Leu Ile Tyr Ala Ala Ser Asn Leu Glu Ser Gly Ile Pro Ser
       50                  55                  60
   Arg Phe Ser Gly Ser Gly Ser Gly Thr Asp Phe Thr Phe Thr Ile Ser
   65                  70                  75                  80
   Ser Leu Gln Pro Glu Asp Ile Ala Thr Tyr Tyr Cys Gln Gln Ser Asn
                   85                  90                  95
   Glu Asp Pro Pro Thr Phe Gly Gln Gly Thr Lys Val Glu Ile Lys Arg
               100                 105                 110
   Thr Val Ala Ala Pro Ser Val Phe Ile Phe Pro Pro Ser Asp Glu Gln
           115                 120                 125
   Leu Lys Ser Gly Thr Ala Ser Val Val Cys Leu Leu Asn Asn Phe Tyr
       130                 135                 140
   Pro Arg Glu Ala Lys Val Gln Trp Lys Val Asp Asn Ala Leu Gln Ser
   145                 150                 155                 160
   Gly Asn Ser Gln Glu Ser Val Thr Glu Gln Asp Ser Lys Asp Ser Thr
                   165                 170                 175
   Tyr Ser Leu Ser Ser Thr Leu Thr Leu Ser Lys Ala Asp Tyr Glu Lys
               180                 185                 190
   His Lys Val Tyr Ala Cys Glu Val Thr His Gln Gly Leu Ser Ser Pro
           195                 200                 205
   Val Thr Lys Ser Phe Asn Arg Gly Glu Cys
       210                 215
<210> 161
   <211> 451
   <212> PRT
   <213> Artificial Sequence
<220>
   <223> Humanised sequence
<400> 161
   Gln Val Thr Leu Arg Glu Ser Gly Pro Ala Leu Val Lys Pro Thr Gln
   1               5                   10                  15
   Thr Leu Thr Leu Thr Cys Thr Phe Ser Gly Phe Ser Leu Ser Thr Ser
               20                  25                  30
   Gly Met Gly Val Ser Trp Ile Arg Gln Pro Pro Gly Lys Gly Leu Glu
           35                  40                  45
   Trp Leu Ala His Ile Tyr Trp Asp Asp Asp Lys Arg Tyr Asn Pro Ser
       50                  55                  60
   Leu Lys Ser Arg Leu Thr Ile Ser Lys Asp Thr Ser Arg Asn Gln Val
   65                  70                  75                  80
   Val Leu Thr Met Thr Asn Met Asp Pro Val Asp Thr Ala Thr Tyr Tyr
                   85                  90                  95
   Cys Ala Arg Arg Glu Thr Val Phe Tyr Trp Tyr Phe Asp Val Trp Gly
               100                 105                 110
   Arg Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu His Asn His Tyr Thr Gln Lys Ser Leu Ser Leu Ser
           435                 440                 445
   Pro Gly Lys
       450
<210> 162
   <211> 451
   <212> PRT
   <213> Artificial Sequence
<220>
   <223> Humanised sequence
<400> 162
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Leu
               420                 425                 430
   His Glu Ala Leu His Ser His Tyr Thr Gln Lys Ser Leu Ser Leu Ser
           435                 440                 445
   Pro Gly Lys
       450
<210> 163
   <211> 330
   <212> PRT
   <213> Artificial Sequence
<220>
   <223> Humanised sequence
<400> 163
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Leu His Glu Ala Leu His Ser His Tyr Thr
   305                 310                 315                 320
   Gln Lys Ser Leu Ser Leu Ser Pro Gly Lys
                   325                 330
<210> 164
   <211> 451
   <212> PRT
   <213> Artificial Sequence
<220>
   <223> Humanised sequence
<400> 164
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Asp Glu Leu Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu Lys Phe His Tyr Thr Gln Lys Ser Leu Ser Leu Ser
           435                 440                 445
   Pro Gly Lys
       450
<210> 165
   <211> 330
   <212> PRT
   <213> Artificial Sequence
<220>
   <223> Humanised sequence
<400> 165
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Asp Glu
   225                 230                 235                 240
   Leu Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu Lys Phe His Tyr Thr
   305                 310                 315                 320
   Gln Lys Ser Leu Ser Leu Ser Pro Gly Lys
                   325                 330
<210> 166
   <211> 451
   <212> PRT
   <213> Artificial Sequence
<220>
   <223> Humanised sequence
<400> 166
   Glu Val Gln Leu Val Glu Ser Gly Gly Gly Leu Val Gln Pro Gly Arg
    1               5                  10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Glu Leu Leu Gly
   225                 230                 235                 240
   Gly Pro Ser Val Phe Leu Phe Pro Pro Lys Pro Lys Asp Thr Leu Met
                   245                 250                 255
   Ile Ser Arg Thr Pro Glu Val Thr Cys Val Val Val Asp Val Ser His
               260                 265                 270
   Glu Asp Pro Glu Val Lys Phe Asn Trp Tyr Val Asp Gly Val Glu Val
           275                 280                 285
   His Asn Ala Lys Thr Lys Pro Arg Glu Glu Gln Tyr Asn Ser Thr Tyr
       290                 295                 300
   Arg Val Val Ser Val Leu Thr Val Leu His Gln Asp Trp Leu Asn Gly
   305                 310                 315                 320
   Lys Glu Tyr Lys Cys Lys Val Ser Asn Lys Ala Leu Pro Ala Pro Ile
                   325                 330                 335
   Glu Lys Thr Ile Ser Lys Ala Lys Gly Gln Pro Arg Glu Pro Gln Val
               340                 345                 350
   Tyr Thr Leu Pro Pro Ser Arg Glu Glu Met Thr Lys Asn Gln Val Ser
           355                 360                 365
   Leu Thr Cys Leu Val Lys Gly Phe Tyr Pro Ser Asp Ile Ala Val Glu
       370                 375                 380
   Trp Glu Ser Asn Gly Gln Pro Glu Asn Asn Tyr Lys Thr Thr Pro Pro
   385                 390                 395                 400
   Val Leu Asp Ser Asp Gly Ser Phe Phe Leu Tyr Ser Lys Leu Thr Val
                   405                 410                 415
   Asp Lys Ser Arg Trp Gln Gln Gly Asn Val Phe Ser Cys Ser Val Met
               420                 425                 430
   His Glu Ala Leu Lys Phe His Tyr Thr Gln Lys Ser Leu Ser Leu Ser
           435                 440                 445
   Pro Gly Lys
       450
<210> 167
   <211> 330
   <212> PRT
   <213> Artificial Sequence
<220>
   <223> Humanised sequence
<400> 167
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Glu Leu Leu Gly Gly Pro Ser Val Phe Leu Phe Pro Pro
           115                 120                 125
   Lys Pro Lys Asp Thr Leu Met Ile Ser Arg Thr Pro Glu Val Thr Cys
       130                 135                 140
   Val Val Val Asp Val Ser His Glu Asp Pro Glu Val Lys Phe Asn Trp
   145                 150                 155                 160
   Tyr Val Asp Gly Val Glu Val His Asn Ala Lys Thr Lys Pro Arg Glu
                   165                 170                 175
   Glu Gln Tyr Asn Ser Thr Tyr Arg Val Val Ser Val Leu Thr Val Leu
               180                 185                 190
   His Gln Asp Trp Leu Asn Gly Lys Glu Tyr Lys Cys Lys Val Ser Asn
           195                 200                 205
   Lys Ala Leu Pro Ala Pro Ile Glu Lys Thr Ile Ser Lys Ala Lys Gly
       210                 215                 220
   Gln Pro Arg Glu Pro Gln Val Tyr Thr Leu Pro Pro Ser Arg Glu Glu
   225                 230                 235                 240
   Met Thr Lys Asn Gln Val Ser Leu Thr Cys Leu Val Lys Gly Phe Tyr
                   245                 250                 255
   Pro Ser Asp Ile Ala Val Glu Trp Glu Ser Asn Gly Gln Pro Glu Asn
               260                 265                 270
   Asn Tyr Lys Thr Thr Pro Pro Val Leu Asp Ser Asp Gly Ser Phe Phe
           275                 280                 285
   Leu Tyr Ser Lys Leu Thr Val Asp Lys Ser Arg Trp Gln Gln Gly Asn
       290                 295                 300
   Val Phe Ser Cys Ser Val Met His Glu Ala Leu Lys Phe His Tyr Thr
   305                 310                 315                 320
   Gln Lys Ser Leu Ser Leu Ser Pro Gly Lys
                   325                 330
<210> 168
   <211> 450
   <212> PRT
   <213> Artificial Sequence
<220>
   <223> Humanised sequence
<400> 168
   Glu Val Gln Leu Val Glu Ser Gly Gly Gly Leu Val Gln Pro Gly Arg
    1               5                   10                  15
   Ser Leu Arg Leu Ser Cys Ala Ala Ser Gly Phe Thr Phe Asp Asp Tyr
               20                  25                  30
   Ala Met His Trp Val Arg Gln Ala Pro Gly Lys Gly Leu Glu Trp Val
           35                  40                  45
   Ser Ala Ile Thr Trp Asn Ser Gly His Ile Asp Tyr Ala Asp Ser Val
       50                  55                  60
   Glu Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Lys Val Ser Tyr Leu Ser Thr Ala Ser Ser Leu Asp Tyr Trp Gly
               100                 105                 110
   Gln Gly Thr Leu Val Thr Val Ser Ser Ala Ser Thr Lys Gly Pro Ser
           115                 120                 125
   Val Phe Pro Leu Ala Pro Ser Ser Lys Ser Thr Ser Gly Gly Thr Ala
       130                 135                 140
   Ala Leu Gly Cys Leu Val Lys Asp Tyr Phe Pro Glu Pro Val Thr Val
   145                 150                 155                 160
   Ser Trp Asn Ser Gly Ala Leu Thr Ser Gly Val His Thr Phe Pro Ala
                   165                 170                 175
   Val Leu Gln Ser Ser Gly Leu Tyr Ser Leu Ser Ser Val Val Thr Val
               180                 185                 190
   Pro Ser Ser Ser Leu Gly Thr Gln Thr Tyr Ile Cys Asn Val Asn His
           195                 200                 205
   Lys Pro Ser Asn Thr Lys Val Asp Lys Lys Val Glu Pro Lys Ser Cys
       210                 215                 220
   Asp Lys Thr His Thr Cys Pro Pro Cys Pro Ala Pro Pro Val Ala Gly
   225                 230                 235                 240
   Pro Ser Val Phe Leu Phe Pro Pro Lys Pro Lys Asp Thr Leu Met Ile
                   245                 250                 255
   Ser Arg Thr Pro Glu Val Thr Cys Val Val Val Asp Val Ser His Glu
               260                 265                 270
   Asp Pro Glu Val Gln Phe Asn Trp Tyr Val Asp Gly Val Glu Val His
           275                 280                 285
   Asn Ala Lys Thr Lys Pro Arg Glu Glu Gln Phe Asn Ser Thr Phe Arg
       290                 295                 300
   Val Val Ser Val Leu Thr Val Val His Gln Asp Trp Leu Asn Gly Lys
   305                 310                 315                 320
   Glu Tyr Lys Cys Lys Val Ser Asn Lys Gly Leu Pro Ala Pro Ile Glu
                   325                 330                 335
   Lys Thr Ile Ser Lys Thr Lys Gly Gln Pro Arg Glu Pro Gln Val Tyr
               340                 345                 350
   Thr Leu Pro Pro Ser Arg Glu Glu Met Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Met
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Leu His
               420                 425                 430
   Glu Ala Leu His Ser His Tyr Thr Gln Lys Ser Leu Ser Leu Ser Pro
           435                 440                 445
   Gly Lys
       450
<210> 169
   <211> 329
   <212> PRT
   <213> Artificial Sequence
<220>
   <223> Humanised sequence
<400> 169
   Ala Ser Thr Lys Gly Pro Ser Val Phe Pro Leu Ala Pro Ser Ser Lys
    1               5                  10                  15
   Ser Thr Ser Gly Gly Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Gln Thr
   65                  70                  75                  80
   Tyr Ile Cys Asn Val Asn His Lys Pro Ser Asn Thr Lys Val Asp Lys
                   85                  90                  95
   Lys Val Glu Pro Lys Ser Cys Asp Lys Thr His Thr Cys Pro Pro Cys
               100                 105                 110
   Pro Ala Pro Pro Val Ala Gly Pro Ser Val Phe Leu Phe Pro Pro Lys
           115                 120                 125
   Pro Lys Asp Thr Leu Met Ile Ser Arg Thr Pro Glu Val Thr Cys Val
       130                 135                 140
   Val Val Asp Val Ser His Glu Asp Pro Glu Val Gln Phe Asn Trp Tyr
   145                 150                 155                 160
   Val Asp Gly Val Glu Val His Asn Ala Lys Thr Lys Pro Arg Glu Glu
                   165                 170                 175
   Gln Phe Asn Ser Thr Phe Arg Val Val Ser Val Leu Thr Val Val His
               180                 185                 190
   Gln Asp Trp Leu Asn Gly Lys Glu Tyr Lys Cys Lys Val Ser Asn Lys
           195                 200                 205
   Gly Leu Pro Ala Pro Ile Glu Lys Thr Ile Ser Lys Thr Lys Gly Gln
       210                 215                 220
   Pro Arg Glu Pro Gln Val Tyr Thr Leu Pro Pro Ser Arg Glu Glu Met
   225                 230                 235                 240
   Thr Lys Asn Gln Val Ser Leu Thr Cys Leu Val Lys Gly Phe Tyr Pro
                   245                 250                 255
   Ser Asp Ile Ala Val Glu Trp Glu Ser Asn Gly Gln Pro Glu Asn Asn
               260                 265                 270
   Tyr Lys Thr Thr Pro Pro Met Leu Asp Ser Asp Gly Ser Phe Phe Leu
           275                 280                 285
   Tyr Ser Lys Leu Thr Val Asp Lys Ser Arg Trp Gln Gln Gly Asn Val
       290                 295                 300
   Phe Ser Cys Ser Val Leu His Glu Ala Leu His Ser His Tyr Thr Gln
   305                 310                 315                 320
   Lys Ser Leu Ser Leu Ser Pro Gly Lys
                   325
<210> 170
   <211> 126
   <212> PRT
   <213> Artificial Sequence
<220>
   <223> Humanised sequence
<400> 170
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Ile Phe Ser Ser Tyr
               20                  25                  30
   Ala Met His Trp Val Arg Gln Ala Pro Gly Asn Gly Leu Glu Trp Val
           35                  40                  45
   Ala Phe Met Ser Tyr Asp Gly Ser Asn Lys Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Gly Ile Ala Ala Gly Gly Asn Tyr Tyr Tyr Tyr Gly
               100                 105                 110
   Met Asp Val Trp Gly Gln Gly Thr Thr Val Thr Val Ser Ser
           115                 120                 125
<210> 171
   <211> 110
   <212> PRT
   <213> Artificial Sequence
<220>
   <223> Humanised sequence
<400> 171
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Tyr Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Pro
                   85                  90                  95
   Phe Thr Phe Gly Pro Gly Thr Lys Val Asp Ile Lys Arg Thr
               100                 105                 110
<210> 172
   <211> 456
   <212> PRT
   <213> Artificial Sequence
<220>
   <223> Humanised sequence
<400> 172
   Gln Val Gln Leu Val Glu Ser Gly Gly Gly Val Val Gln Pro Gly Arg
    1               5                  10                  15
   Ser Leu Arg Leu Ser Cys Ala Ala Ser Gly Phe Ile Phe Ser Ser Tyr
               20                  25                  30
   Ala Met His Trp Val Arg Gln Ala Pro Gly Asn Gly Leu Glu Trp Val
           35                  40                  45
   Ala Phe Met Ser Tyr Asp Gly Ser Asn Lys Lys Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Asp Arg Gly Ile Ala Ala Gly Gly Asn Tyr Tyr Tyr Tyr Gly
               100                 105                 110
   Met Asp Val Trp Gly Gln Gly Thr Thr Val Thr Val Ser Ser Ala Ser
           115                 120                 125
   Thr Lys Gly Pro Ser Val Phe Pro Leu Ala Pro Ser Ser Lys Ser Thr
       130                 135                 140
   Ser Gly Gly Thr Ala Ala Leu Gly Cys Leu Val Lys Asp Tyr Phe Pro
   145                 150                 155                 160
   Glu Pro Val Thr Val Ser Trp Asn Ser Gly Ala Leu Thr Ser Gly Val
                   165                 170                 175
   His Thr Phe Pro Ala Val Leu Gln Ser Ser Gly Leu Tyr Ser Leu Ser
               180                 185                 190
   Ser Val Val Thr Val Pro Ser Ser Ser Leu Gly Thr Gln Thr Tyr Ile
           195                 200                 205
   Cys Asn Val Asn His Lys Pro Ser Asn Thr Lys Val Asp Lys Lys Val
       210                 215                 220
   Glu Pro Lys Ser Cys Asp Lys Thr His Thr Cys Pro Pro Cys Pro Ala
   225                 230                 235                 240
   Pro Glu Leu Leu Gly Gly Pro Ser Val Phe Leu Phe Pro Pro Lys Pro
                   245                 250                 255
   Lys Asp Thr Leu Tyr Ile Thr Arg Glu Pro Glu Val Thr Cys Val Val
               260                 265                 270
   Val Asp Val Ser His Glu Asp Pro Glu Val Lys Phe Asn Trp Tyr Val
           275                 280                 285
   Asp Gly Val Glu Val His Asn Ala Lys Thr Lys Pro Arg Glu Glu Gln
       290                 295                 300
   Tyr Asn Ser Thr Tyr Arg Val Val Ser Val Leu Thr Val Leu His Gln
   305                 310                 315                 320
   Asp Trp Leu Asn Gly Lys Glu Tyr Lys Cys Lys Val Ser Asn Lys Ala
                   325                 330                 335
   Leu Pro Ala Pro Ile Glu Lys Thr Ile Ser Lys Ala Lys Gly Gln Pro
               340                 345                 350
   Arg Glu Pro Gln Val Tyr Thr Leu Pro Pro Ser Arg Asp Glu Leu Thr
           355                 360                 365
   Lys Asn Gln Val Ser Leu Thr Cys Leu Val Lys Gly Phe Tyr Pro Ser
       370                 375                 380
   Asp Ile Ala Val Glu Trp Glu Ser Asn Gly Gln Pro Glu Asn Asn Tyr
   385                 390                 395                 400
   Lys Thr Thr Pro Pro Val Leu Asp Ser Asp Gly Ser Phe Phe Leu Tyr
                   405                 410                 415
   Ser Lys Leu Thr Val Asp Lys Ser Arg Trp Gln Gln Gly Asn Val Phe
               420                 425                 430
   Ser Cys Ser Val Met His Glu Ala Leu His Asn His Tyr Thr Gln Lys
           435                 440                 445
   Ser Leu Ser Leu Ser Pro Gly Lys
       450                 455
<210> 173
   <211> 215
   <212> PRT
   <213> Artificial Sequence
<220>
   <223> Humanised sequence
<400> 173
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Tyr Ser Tyr
               20                  25                  30
   Leu Ala Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Gln Arg Ser Asn Trp Pro Pro
                   85                  90                  95
   Phe Thr Phe Gly Pro Gly Thr Lys Val Asp Ile Lys Arg Thr Val Ala
               100                 105                 110
   Ala Pro Ser Val Phe Ile Phe Pro Pro Ser Asp Glu Gln Leu Lys Ser
           115                 120                 125
   Gly Thr Ala Ser Val Val Cys Leu Leu Asn Asn Phe Tyr Pro Arg Glu
       130                 135                 140
   Ala Lys Val Gln Trp Lys Val Asp Asn Ala Leu Gln Ser Gly Asn Ser
   145                 150                 155                 160
   Gln Glu Ser Val Thr Glu Gln Asp Ser Lys Asp Ser Thr Tyr Ser Leu
                   165                 170                 175
   Ser Ser Thr Leu Thr Leu Ser Lys Ala Asp Tyr Glu Lys His Lys Val
               180                 185                 190
   Tyr Ala Cys Glu Val Thr His Gln Gly Leu Ser Ser Pro Val Thr Lys
           195                 200                 205
   Ser Phe Asn Arg Gly Glu Cys
       210                 215
<210> 174
   <211> 120
   <212> PRT
   <213> Artificial Sequence
<220>
   <223> Humanised sequence
<400> 174
   Glu Val Lys Leu Glu Glu Ser Gly Gly Gly Leu Val Gln Pro Gly Gly
    1               5                  10                  15
   Ser Met Lys Leu Ser Cys Val Ala Ser Gly Phe Ile Phe Ser Asn His
               20                  25                  30
   Trp Met Asn Trp Val Arg Gln Ser Pro Glu Lys Gly Leu Glu Trp Val
           35                  40                  45
   Ala Glu Ile Arg Ser Lys Ser Ile Asn Ser Ala Thr His Tyr Ala Glu
       50                  55                  60
   Ser Val Lys Gly Arg Phe Thr Ile Ser Arg Asp Asp Ser Lys Ser Ala
   65                  70                  75                  80
   Val Tyr Leu Gln Met Thr Asp Leu Arg Thr Glu Asp Thr Gly Val Tyr
                   85                  90                  95
   Tyr Cys Ser Arg Asn Tyr Tyr Gly Ser Thr Tyr Asp Tyr Trp Gly Gln
               100                 105                 110
   Gly Thr Thr Leu Thr Val Ser Ser
           115                 120
<210> 175
   <211> 109
   <212> PRT
   <213> Artificial Sequence
<220>
   <223> Humanised sequence
<400> 175
   Asp Ile Leu Leu Thr Gln Ser Pro Ala Ile Leu Ser Val Ser Pro Gly
    1               5                  10                  15
   Glu Arg Val Ser Phe Ser Cys Arg Ala Ser Gln Phe Val Gly Ser Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Met Ser Gly Ile Pro Ser Arg Phe Ser Gly
       50                  55                  60
   Ser Gly Ser Gly Thr Asp Phe Thr Leu Ser Ile Asn Thr Val Glu Ser
   65                  70                  75                  80
   Glu Asp Ile Ala Asp Tyr Tyr Cys Gln Gln Ser His Ser Trp Pro Phe
                   85                  90                  95
   Thr Phe Gly Ser Gly Thr Asn Leu Glu Val Lys Arg Thr
               100                 105
<210> 176
   <211> 450
   <212> PRT
   <213> Artificial Sequence
<220>
   <223> Humanised sequence
<400> 176
   Glu Val Lys Leu Glu Glu Ser Gly Gly Gly Leu Val Gln Pro Gly Gly
    1               5                  10                  15
   Ser Met Lys Leu Ser Cys Val Ala Ser Gly Phe Ile Phe Ser Asn His
               20                  25                  30
   Trp Met Asn Trp Val Arg Gln Ser Pro Glu Lys Gly Leu Glu Trp Val
           35                  40                  45
   Ala Glu Ile Arg Ser Lys Ser Ile Asn Ser Ala Thr His Tyr Ala Glu
       50                  55                  60
   Ser Val Lys Gly Arg Phe Thr Ile Ser Arg Asp Asp Ser Lys Ser Ala
   65                  70                  75                  80
   Val Tyr Leu Gln Met Thr Asp Leu Arg Thr Glu Asp Thr Gly Val Tyr
                   85                  90                  95
   Tyr Cys Ser Arg Asn Tyr Tyr Gly Ser Thr Tyr Asp Tyr Trp Gly Gln
               100                 105                 110
   Gly Thr Thr Leu Thr Val Ser Ser Ala Ser Thr Lys Gly Pro Ser Val
           115                 120                 125
   Phe Pro Leu Ala Pro Ser Ser Lys Ser Thr Ser Gly Gly Thr Ala Ala
       130                 135                 140
   Leu Gly Cys Leu Val Lys Asp Tyr Phe Pro Glu Pro Val Thr Val Ser
   145                 150                 155                 160
   Trp Asn Ser Gly Ala Leu Thr Ser Gly Val His Thr Phe Pro Ala Val
                   165                 170                 175
   Leu Gln Ser Ser Gly Leu Tyr Ser Leu Ser Ser Val Val Thr Val Pro
               180                 185                 190
   Ser Ser Ser Leu Gly Thr Gln Thr Tyr Ile Cys Asn Val Asn His Lys
           195                 200                 205
   Pro Ser Asn Thr Lys Val Asp Lys Lys Val Glu Pro Lys Ser Cys Asp
       210                 215                 220
   Lys Thr His Thr Cys Pro Pro Cys Pro Ala Pro Glu Leu Leu Gly Gly
   225                 230                 235                 240
   Pro Ser Val Phe Leu Phe Pro Pro Lys Pro Lys Asp Thr Leu Tyr Ile
                   245                 250                 255
   Thr Arg Glu Pro Glu Val Thr Cys Val Val Val Asp Val Ser His Glu
               260                 265                 270
   Asp Pro Glu Val Lys Phe Asn Trp Tyr Val Asp Gly Val Glu Val His
           275                 280                 285
   Asn Ala Lys Thr Lys Pro Arg Glu Glu Gln Tyr Asn Ser Thr Tyr Arg
       290                 295                 300
   Val Val Ser Val Leu Thr Val Leu His Gln Asp Trp Leu Asn Gly Lys
   305                 310                 315                 320
   Glu Tyr Lys Cys Lys Val Ser Asn Lys Ala Leu Pro Ala Pro Ile Glu
                   325                 330                 335
   Lys Thr Ile Ser Lys Ala Lys Gly Gln Pro Arg Glu Pro Gln Val Tyr
               340                 345                 350
   Thr Leu Pro Pro Ser Arg Asp Glu Leu Thr Lys Asn Gln Val Ser Leu
           355                 360                 365
   Thr Cys Leu Val Lys Gly Phe Tyr Pro Ser Asp Ile Ala Val Glu Trp
       370                 375                 380
   Glu Ser Asn Gly Gln Pro Glu Asn Asn Tyr Lys Thr Thr Pro Pro Val
   385                 390                 395                 400
   Leu Asp Ser Asp Gly Ser Phe Phe Leu Tyr Ser Lys Leu Thr Val Asp
                   405                 410                 415
   Lys Ser Arg Trp Gln Gln Gly Asn Val Phe Ser Cys Ser Val Met His
               420                 425                 430
   Glu Ala Leu His Asn His Tyr Thr Gln Lys Ser Leu Ser Leu Ser Pro
           435                 440                 445
   Gly Lys
       450
<210> 177
   <211> 214
   <212> PRT
   <213> Artificial Sequence
<220>
   <223> Humanised sequence
<400> 177
   Asp Ile Leu Leu Thr Gln Ser Pro Ala Ile Leu Ser Val Ser Pro Gly
    1               5                  10                  15
   Glu Arg Val Ser Phe Ser Cys Arg Ala Ser Gln Phe Val Gly Ser Ser
               20                  25                  30
   Ile His Trp Tyr Gln Gln Arg Thr Asn Gly Ser Pro Arg Leu Leu Ile
           35                  40                  45
   Lys Tyr Ala Ser Glu Ser Met Ser Gly Ile Pro Ser Arg Phe Ser Gly
       50                  55                  60
   Ser Gly Ser Gly Thr Asp Phe Thr Leu Ser Ile Asn Thr Val Glu Ser
   65                  70                  75                  80
   Glu Asp Ile Ala Asp Tyr Tyr Cys Gln Gln Ser His Ser Trp Pro Phe
                   85                  90                  95
   Thr Phe Gly Ser Gly Thr Asn Leu Glu Val Lys Arg Thr Val Ala Ala
               100                 105                 110
   Pro Ser Val Phe Ile Phe Pro Pro Ser Asp Glu Gln Leu Lys Ser Gly
           115                 120                 125
   Thr Ala Ser Val Val Cys Leu Leu Asn Asn Phe Tyr Pro Arg Glu Ala
       130                 135                 140
   Lys Val Gln Trp Lys Val Asp Asn Ala Leu Gln Ser Gly Asn Ser Gln
   145                 150                 155                 160
   Glu Ser Val Thr Glu Gln Asp Ser Lys Asp Ser Thr Tyr Ser Leu Ser
                   165                 170                 175
   Ser Thr Leu Thr Leu Ser Lys Ala Asp Tyr Glu Lys His Lys Val Tyr
               180                 185                 190
   Ala Cys Glu Val Thr His Gln Gly Leu Ser Ser Pro Val Thr Lys Ser
           195                 200                 205
   Phe Asn Arg Gly Glu Cys
       210
<210> 178
   <211> 118
   <212> PRT
   <213> Artificial Sequence
<220>
   <223> Humanised sequence
<400> 178
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Tyr Val Phe Thr Asp Tyr
               20                  25                  30
   Gly Met Asn Trp Val Arg Gln Ala Pro Gly Lys Gly Leu Glu Trp Met
           35                  40                  45
   Gly Trp Ile Asn Thr Tyr Ile Gly Glu Pro Ile Tyr Ala Asp Ser Val
       50                  55                  60
   Lys Gly Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Gly Tyr Arg Ser Tyr Ala Met Asp Tyr Trp Gly Gln Gly Thr
               100                 105                 110
   Leu Val Thr Val Ser Ser
           115
<210> 179
   <211> 109
   <212> PRT
   <213> Artificial Sequence
<220>
   <223> Humanised sequence
<400> 179
   Asp Ile Gln Met Thr Gln Ser Pro Ser Ser Leu Ser Ala Ser Val Gly
    1               5                  10                  15
   Asp Arg Val Thr Ile Thr Cys Lys Ala Ser Gln Asn Val Gly Thr Asn
               20                  25                  30
   Val Ala Trp Tyr Gln Gln Lys Pro Gly Lys Ala Pro Lys Ala Leu Ile
           35                  40                  45
   Tyr Ser Ala Ser Phe Leu Tyr Ser Gly Val Pro Tyr Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Tyr Asn Ile Tyr Pro Leu
                   85                  90                  95
   Thr Phe Gly Gln Gly Thr Lys Val Glu Ile Lys Arg Thr
               100                 105
<210> 180
   <211> 229
   <212> PRT
   <213> Artificial Sequence
<220>
   <223> Humanised sequence
<400> 180
   Glu Val Gln Leu Val Glu Ser Gly Gly Gly Leu Val Gln Pro Gly Gly
    1               5                  10                  15
   Ser Leu Arg Leu Ser Cys Ala Ala Ser Gly Tyr Val Phe Thr Asp Tyr
               20                  25                  30
   Gly Met Asn Trp Val Arg Gln Ala Pro Gly Lys Gly Leu Glu Trp Met
           35                  40                  45
   Gly Trp Ile Asn Thr Tyr Ile Gly Glu Pro Ile Tyr Ala Asp Ser Val
       50                  55                  60
   Lys Gly Arg Phe Thr Phe Ser Leu Asp Thr Ser Lys Ser Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Gly Tyr Arg Ser Tyr Ala Met Asp Tyr Trp Gly Gln Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Ala Ala
   225